(19) Europäisches Patentamt
European Patent Office
Office européen des brevets

(11) EP 1 073 643 B1

(12) EUROPEAN PATENT SPECIFICATION

(45) Date of publication and mention of the grant of the patent:
**29.12.2004 Bulletin 2004/53**

(21) Application number: **98953969.7**

(22) Date of filing: **26.10.1998**

(51) Int Cl.$^7$: **C07D 239/90**

(86) International application number:
**PCT/US1998/022568**

(87) International publication number:
**WO 1999/008501 (25.02.1999 Gazette 1999/08)**

(54) **NEW HETEROCYCLIC COMPOUNDS AND THEIR USE IN MEDICINE, PROCESS FOR THEIR PREPARATION AND PHARMACEUTICAL COMPOSITIONS CONTAINING THEM**

HETEROZYKLISCHE VERBINDUNGEN,UND DEREN VERWENDUNG IN ARZNEIMITTEL,VERFAHREN ZU DEREN HERSTELLUNG UND DIESE ENTHALTENDE PHARMAZEUTISCHE ZUSAMMENSTELLUNGEN

NOUVEAUX COMPOSES HETEROCYCLIQUES, LEUR UTILISATION EN MEDECINE, LEUR PROCEDE DE PREPARATION ET COMPOSITIONS PHARMACEUTIQUES LES CONTENANT

(84) Designated Contracting States:
**AT BE CH CY DE DK ES FI FR GB GR IE IT LI LU MC NL PT SE**
Designated Extension States:
**AL LT LV MK RO SI**

(30) Priority: **23.04.1998 US 82825 P**

(43) Date of publication of application:
**07.02.2001 Bulletin 2001/06**

(73) Proprietor: **Dr. Reddy's Laboratories Ltd.**
**Hyderabad 500 016 (IN)**

(72) Inventors:
- **LOHRAY, Vidya, Bhushan**
  **Hyderabad, Andhra Pradesh 500 016 (IN)**
- **LOHRAY, Braj, Bhushan**
  **Hyderabad, Andhra Pradesh 500 016 (IN)**
- **PARASELLI, Rao, Bheema**
  **Hyderabad, Andhra Pradesh 500 016 (IN)**
- **RAMANUJAM, Rajagopalan**
  **Hyderabad, Andhra Pradesh 500 016 (IN)**
- **CHAKRABARTI, Ranjan**
  **Hyderabad, Andhra Pradesh 500 016 (IN)**

(74) Representative:
**Ebner von Eschenbach, Jennifer et al**
**Ladas & Parry,**
**Dachauerstrasse 37**
**80335 München (DE)**

(56) References cited:
**WO-A-94/01420** **WO-A-97/41097**

Remarks:
The file contains technical information submitted after the application was filed and not included in this specification

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

### Field of Invention

The present invention relates to novel antiobesity and hypocholesterolemic compounds, their tautomeric forms, their stereoisomers, their polymorphs, their pharmaceutically acceptable salts, their pharmaceutically acceptable solvates and pharmaceutically acceptable compositions containing them. More particularly, the present invention relates to novel β-aryl-α-oxysubstituted alkylcarboxylic acids of the general formula (I), their tautomeric forms, their stereoisomers, their polymorphs, their pharmaceutically acceptable salts, their pharmaceutically acceptable solvates and pharmaceutically acceptable compositions containing them.

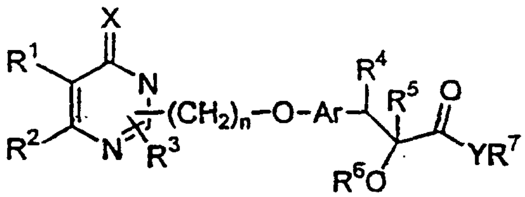

(I)

The present invention also relates to a process for the preparation of the above said novel compounds, their tautomeric forms, their stereoisomers, their polymorphs, their pharmaceutically acceptable salts, their pharmaceutically acceptable solvates, novel intermediates and pharmaceutical compositions containing them.

The compounds of the present invention lower total cholesterol (TC); increase high density lipoprotein (HDL) and decrease low density lipoprotein (LDL), which have beneficial effects on coronary heart disease and atherosclerosis.

The compounds of general formula (I) are useful in reducing body weight and for the treatment and/or prophylaxis of diseases such as hypertension, coronary heart disease, atherosclerosis, stroke, peripheral vascular diseases and related disorders. These compounds are useful for the treatment of familial hypercholesterolemia, hypertriglyceridemia, lowering of atherogenic lipoproteins, very low density lipoprotein (VLDL) and LDL. The compounds of the present invention can be used for the treatment of certain renal diseases including glomerulonephritis, glomerulosclerosis, nephrotic syndrome, hypertensive nephrosclerosis, retinopathy, and nephropathy. The compounds of general formula (I) are also useful for the treatment/prophylaxis of insulin resistance (type II diabetes), leptin resistance, impaired glucose tolerance, dyslipidemia, disorders related to syndrome X such as hypertension, obesity, insulin resistance, coronary heart disease, and other cardiovascular disorders. These compounds may also be useful as aldose reductase inhibitors, for improving cognitive functions in dementia, treating diabetic complications, disorders related to endothelial cell activation, psoriasis, polycystic ovarian syndrome (PCOS), inflammatory bowel diseases, osteoporosis, myotonic dystrophy, pancreatitis, arteriosclerosis, xanthoma and for the treatment of cancer. The compounds of the present inventions are useful in the treatment and/or prophylaxis of the above said diseases in combination/concomittant with one or more HMG CoA reductase inhibitors, and/or hypolipidemic/hypolipoproteinemic agents such as fibric acid derivatives, nicotinic acid, cholestyramine, colestipol, or probucol.

### Background of Invention

Atherosclerosis and other peripheral vascular diseases effect the quality of life of millions of people. Therefore, considerable attention has been directed towards understanding the etiology of hypercholesterolemia and hyperlipidemia and the development of effective therapeutic strategies.

Hypercholesterolemia has been defined as plasma cholesterol level that exceeds arbitrarily defined value called "normal" level. Recently, it has been accepted that "ideal" plasma levels of cholesterol are much below the "normal" level of cholesterol in the general population and the risk of coronary artery disease (CAD) increases as cholesterol level rises above the "optimum" (or "ideal") value. There is clearly a definite cause and effect-relationship between hypercholesterolemia and CAD, particularly for individuals with multiple risk factors. Most of the cholesterol is present in the esterified forms with various lipoproteins such as low density lipoprotein (LDL), intermediate density lipoprotein (IDL), high density lipoprotein (HDL) and partially as very low density lipoprotein (VLDL). Studies clearly indicate that there is an inverse correlationship between CAD and atherosclerosis with serum HDL-cholesterol concentrations. (Stampfer *et al.*, *N. Engl. J. Med,* **325** (1991), 373-381) and the risk of CAD increases with increasing levels of LDL and VLDL.

In CAD, generally "fatty streaks" in carotid, coronary and cerebral arteries, are found which are primarily free and esterified cholesterol. Miller *et al.*, *(Br. Med J.*, **282** (1981), 1741-1744) have shown that increase in HDL-particles

may decrease the number of sites of stenosis in coronary arteries of humans, and high level of HDL-cholesterol may protect against the progression of atherosclerosis. Picardo *et al*., (*Arteriosclerosis* **6** (1986) 434 - 441) have shown by *in vitro* experiments that HDL is capable of removing cholesterol from cells. They suggest that HDL may deplete tissues of excess free cholesterol and transfer them to the liver (Macikinnon *et al*., *J. Biol. Chem*. **261.** (1986), 2548-2552). Therefore, agents that increase HDL cholesterol would have therapeutic significance for the treatment of hypercholesterolemia and coronary heart diseases (CHD).

Obesity is a disease highly prevalent in affluent societies and in the developing world and is a major cause of morbidity and mortality. It is a state of excess body fat accumulation. The causes of obesity are unclear. It is believed to be of genetic origin or promoted by an interaction between the genotype and environment. Irrespective of the cause, the result is fat deposition due to imbalance between the energy intake versus energy expenditure. Dieting, exercise and appetite suppression have been a part of obesity treatment. There is a need for efficient therapy to fight this disease since it may lead to coronary heart disease, diabetes, stroke, hyperlipidemia, gout, osteoarthritis, reduced fertility and many other psychological and social problems.

Diabetes and insulin resistance is yet another disease which severely effects the quality of life of a large population in the world. Insulin resistance is the diminished ability of insulin to exert its biological action across a broad range of concentrations. In insulin resistance, the body secretes abnormally high amounts of insulin to compensate for this defect; failing which, the plasma glucose concentration inevitably rises and develops into diabetes. Among the developed countries, diabetes mellitus is a common problem and is associated with a variety of abnormalities including obesity, hypertension, hyperlipidemia (J. Clin. Invest., (1985) 75:809-817; N. Engl. J. Med. (1987) 317:350-357; J. Clin. Endocrinol. Metab., (1988) 66 : 580 - 583; J. Clin. Invest., (1975) 68:957-969) and other renal complications (See Patent Application No. WO 95/21608). It is now increasingly being recognized that insulin resistance and relative hyperinsulinemia have a contributory role in obesity, hypertension, atherosclerosis and type 2 diabetes mellitus. The association of insulin resistance with obesity, hypertension and angina has been described as a syndrome having insulin resistance as the central pathogenic link-Syndrome-X.

Hyperlipidemia is the primary cause of cardiovascular (CVD) and other peripheral vascular diseases. High risk of CVD is related to the higher LDL (Low Density Lipoprotein) and VLDL (Very Low Density Lipoprotein) seen in hyperlipidemia. Patients having glucose intolerance/insulin resistance in addition to hyperlipidemia have higher risk of CVD. Numerous studies in the past have shown that lowering of plasma triglycerides and total cholesterol, in particular LDL and VLDL and increasing HDL cholesterol help in preventing cardiovascular diseases.

Peroxisome proliferator activated receptors (PPAR) are members of the nuclear receptor super family. The gamma ($\gamma$) isoform of PPAR (PPAR$\gamma$) has been implicated in regulating differentiation of adipocytes (Endocrinology, (1994) 135: 798-800) and energy homeostasis (Cell, (1995) 83: 803-812), whereas the alpha ($\alpha$) isoform of PPAR (PPAR$\alpha$) mediates fatty acid oxidation (Trend. Endocrin. Metab., (1993) 4: 291-296) thereby resulting in reduction of circulating free fatty acid in plasma (Current Biol. (1995) 5: 618 -621). PPAR$\alpha$ agonists have been found useful for the treatment of obesity (WO 97/36579). It has been recently disclosed that the hypolipidaemic effect is enhanced when the molecule has both PPAR$\alpha$ and PPAR$\gamma$ agonist activity and suggested to be useful for the treatment of syndrome X (WO 97/25042). Synergism between the insulin sensitizer (PPAR$\gamma$ agonist) and HMG CoA reductase inhibitor has been observed which may be useful for the treatment of atherosclerosis and xanthoma. (EP 0 753 298).

It is known that PPAR$\gamma$ plays an important role in adipocyte differentiation (Cell, (1996) 87, 377-389). Ligand activation of PPAR is sufficient to cause complete terminal differentiation (Cell, (1994) 79, 1147-1156) including cell cycle withdrawal. PPAR$\gamma$ is consistently expressed in certain cells and activation of this nuclear receptor with PPAR$\gamma$ agonists would stimulate the terminal differentiation of adipocyte precursors and cause morphological and molecular changes characteristics of a more differentiated, less malignant state (Molecular Cell, (1998), 465-470; Carcinogenesis, (1998), 1949-53; Proc. Natl. Acad. Sci., (1997) 94,237-241) and inhibition of expression of prostate cancer tissue (Cancer Research (1998), 58:3344-3352). This would be useful in the treatment of certain types of cancer, which express PPAR$\gamma$ and could lead to a quite nontoxic chemotherapy.

Leptin resistance is a condition wherein the target cells are unable to respond to leptin signals. This may give rise to obesity due to excess food intake and reduced energy expenditure and cause impaired glucose tolerance, type 2 diabetes, cardiovascular diseases and such other interrelated complications. Kallen *et al* (Proc. Natl. Acad. Sci., (1996) 93, 5793-5796) have reported that insulin sensitizers which perhaps due to their PPAR agonist expression and lower plasma leptin concentrations. However, it has been recently disclosed that compounds having insulin sensitizing property also possess leptin sensitization activity. They lower the circulating plasma leptin concentrations by improving the target cell response to leptin (WO 98/02159).

A few $\beta$-aryl-$\alpha$-hydroxy propionic acids, their derivatives, and their analogs have been reported to be useful in the treatment of hyperglycemia and hypercholesterolemia. Some of such compounds described in the prior art are outlined below:

i) U.S. Pat. 5,306,726 and WO 91/19702 disclose several 3-aryl-2-hydroxypropionic acid derivatives of general

formula (IIa) and (IIb) as hypolipidemic and hypoglycemic agents.

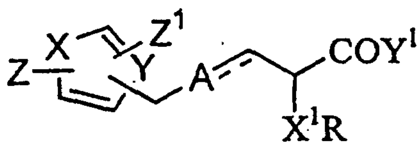

**(II a)**

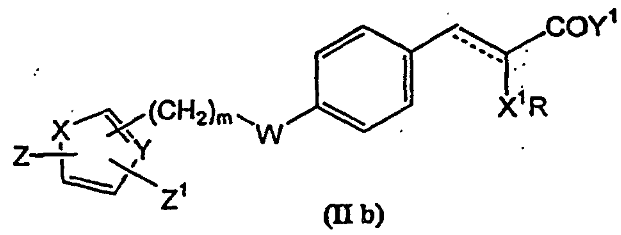

**(II b)**

Examples of these compounds are shown in formula (II c) and (II d)

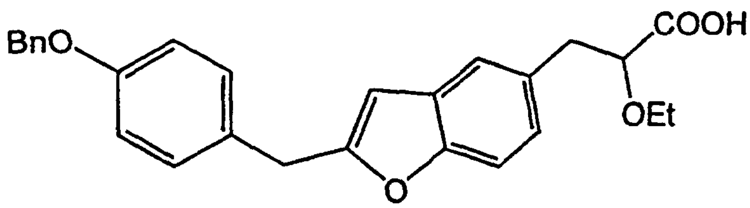

**(II c)**

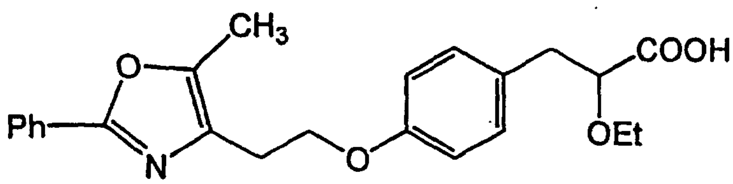

**(II d)**

ii) International Patent Applications, WO 95/03038 and WO 96/04260 disclose compounds of formula (II e)

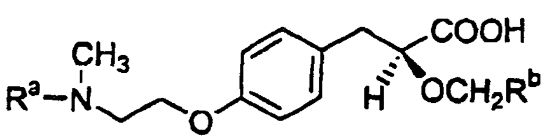

**(II e)**

wherein $R^a$ represents 2-benzoxazolyl or 2-pyridyl and $R^b$ represent $CF_3$, $CH_2OCH_3$ or $CH_3$. A typical example is (*S*)-3-[4-[2-[N-(2-benzoxazolyl)N-methylamino] ethoxy]phenyl]-2-(2,2,2-trifluoroethoxy)propanoic acid (II f).

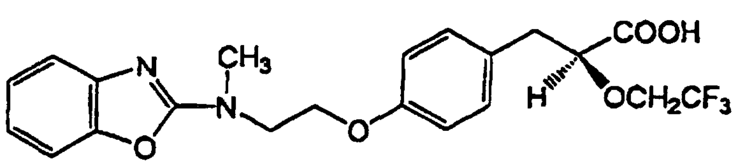

**(II f)**

iii) International Patent Application Nos. WO 94/13650, WO 94/01420 and WO 95/17394 disclose the compounds of general formula (II g)

$$A^1—X—(CH_2)_n—O—A^2—A^3—Y \cdot R^2 \qquad \text{(II g)}$$

wherein $A^1$ represent aromatic heterocycle, $A^2$ represents substituted benzene ring and $A^3$ represents moiety of formula $(CH_2)_m$-CH-($OR^1$), wherein $R^1$ represents alkyl groups, m is an integer of 1-5; X represents substituted or unsubstitured N; Y represents C=O or C=S, $R^2$ represents $OR^3$ where $R^3$ may be hydrogen, alkyl, aralkyl, or aryl group and n is an integer of 2-6. An example of these compounds is shown in formula (II h)

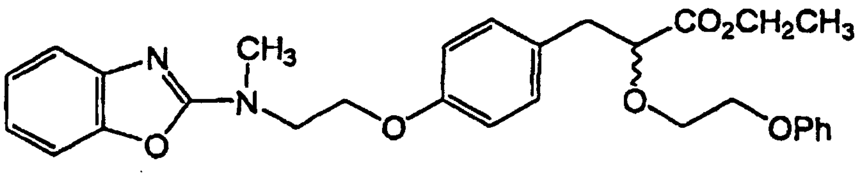

(II h)

iv) International Patent Application WO 97/41097 discloses compounds of general formula (I)

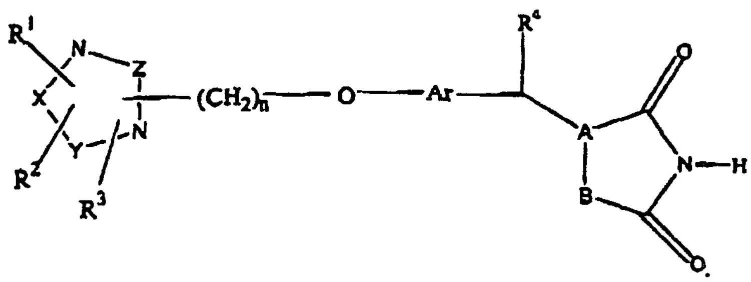

(I)

where at least one of X, Y or Z is C=O or C=S, and any two of $R^1$, $R^2$ and $R^3$ along with the adjacent atoms to which they are attached may form a substituted or unsubstituted cyclic ring

## Summary of the Invention

With an objective to develop novel compounds for lowering cholesterol and reducing body weight with beneficial effects in the treatment and/or prophylaxis of diseases related to increased levels of lipids, atherosclerosis, coronary artery diseases, Syndrome-X, impaired glucose tolerance, insulin resistance, insulin resistance leading to type 2 diabetes and diabetes complications thereof, for the treatment of diseases wherein insulin resistance is the pathophysiological mechanism, for the treatment and/ or prophylaxis of leptin resistance and complications thereof, hypertension, atherosclerosis and coronary artery diseases with better efficacy, potency and lower toxicity, we focussed our research to develop new compounds effective in the treatment of above mentioned diseases. Effort in this direction has led to compounds having general formula (I).

The main objective of the present invention is therefore, to provide novel β-aryl-α-oxysubstituted alkylcarboxylic acids, their tautomeric forms, their stereoisomers, their polymorphs, their pharmaceutically acceptable salts, and their pharmaceutically acceptable solvates and pharmaceutical compositions containing them, or their mixtures.

Another objective of the present invention is to provide novel β-aryl-α-oxy-substituted alkylcarboxylic acids, their tautomeric forms, their stereoisomers, their polymorphs, their pharmaceutically acceptable salts, and their pharmaceutically acceptable solvates and pharmaceutical compositions containing them or their mixtures which may have agonist activity against PPARα and/or PPARγ, and may inhibit HMG CoA reductase, in addition to agonist activity against PPARα and/or PPARγ.

Another objective of the present invention is to provide novel β-aryl-α-oxy-substituted alkylcarboxylic acids, their tautomeric forms, their stereoisomers, their polymorphs, their pharmaceutically acceptable salts, and their pharmaceutically acceptable solvates and pharmaceutical compositions containing them or their mixtures having enhanced activities, without toxic effect or with reduced toxic effect

Yet another objective of the present invention is to produce a process for the preparation of novel β-aryl-α-oxysubstituted alkylcarboxylic acids of the formula (I) as defined above, their tautomeric forms, their stereoisomers, their polymorphs, their pharmaceutically acceptable salts and their pharmaceutically acceptable solvates.

Still another objective of the present invention is to provide pharmaceutical compositions containing compounds of the general formula (I), their tautomers, their stereoisomers, their polymorphs, their salts, solvates or their mixtures in combination with suitable carriers, solvents, diluents and other media normally employed in preparing such

compositions.

A further objective of the present invention is to provide novel intermediates, a process for preparation of the intermediates and a process for the preparation of novel β-aryl-α-oxysubstituted alkylcarboxylic acids of formula (I), their tautomers their stereoisomers, their polymorphs, their pharmaceutically acceptable salts and their pharmaceutically acceptable solvates using the intermediates.

## Detailed Description of the Invention

β-aryl α-oxysubstituted propionic acids of the present invention have the general formula (I)

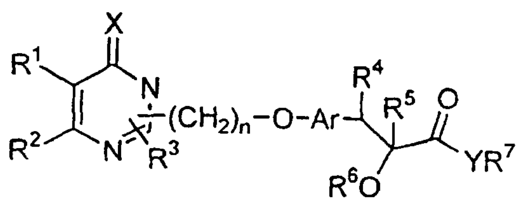

(I)

where X represents O or S; the groups $R^1$, $R^2$ and group $R^3$ when present on carbon atom, are the same or different and represent hydrogen, halogen, hydroxy, or unsubstituted or substituted groups selected from $(C_1$-$C_6)$ alkoxy, $(C_1$-$C_6)$ alkyl, or phenyl, wherein when said groups are substituted the substituent(s) is selected from halogen or hydroxy; or $R^1$, $R^2$ along with the adjacent atoms to which they are attached form a substituted or unsubstituted phenyl group, wherein when the phenyl group formed by $R^1$ and $R^2$ is substituted, the substituent(s) is selected from halogen, hydroxy, $(C_1$-$C_3)$ alkyl or $(C_1$-$C_3)$alkoxy; $R^3$ when attached to nitrogen atom represents hydrogen, or unsubstituted or substituted groups selected from $(C_1$-$C_6)$alkyl, phenyl, naphthyl, benzyl or phenethyl, wherein when the group $R^3$ attached to nitrogen is substituted, the substituent is selected from halogen, hydroxy, methyl, ethyl, isopropyl, n-propyl, n-butyl or $(C_1$-$C_3)$alkoxy; the linking group represented by $-(CH_2)_n-O-$ may be attached either through nitrogen atom or through carbon atom where n is an integer ranging from 1 - 4; Ar represents phenylene ; $R^4$ represents hydrogen atom, methyl, ethyl, hydroxy, $(C_1$-$C_3)$ alkoxy or forms a bond with $R^5$; $R^5$ represents hydrogen methyl, ethyl, hydroxy, $(C_1$-$C_3)$ alkoxy or forms a bond with $R^4$; $R^6$ represents hydrogen, $(C_1$-$C_{12})$alkyl, $(C_1$-$C_6)$alkoxy$(C_1$-$C_6)$alkyl, phenyl or benzyl, with a provision that $R^6$ does not represent hydrogen when $R^7$ represents hydrogen or $(C_1$-$C_{12})$ alkyl group; $R^7$ represents hydrogen or unsubstituted or substituted group selected from $(C_1$-$C_{12})$ alkyl or phenyl, wherein when $R^7$ is substituted the substituent(s) on $R^7$ is selected from halogen ; Y represents oxygen or $NR^8$, where $R^8$ represents hydrogen or $(C_1$-$C_{12})$alkyl ; or $R^7$ and $R^8$ together with the nitrogen atom to which they are attached form a 5 or 6 membered cyclic structure containing carbon atoms, a nitrogen atom and optionally an oxygen atom.

Suitable groups represented by $R^1$, $R^2$ and the group $R^3$ when attached to carbon atom may be selected from hydrogen, halogen atom such as fluorine, chlorine, bromine, or iodine; hydroxy; substituted or unsubstituted linear or branched $(C_1$-$C_6)$alkyl group, such as methyl, ethyl, n-propyl, iso-propyl, n-butyl, iso-butyl, t-butyl, n-pentyl, iso-pentyl, hexyl; phenyl, which may be substituted; $(C_1$-$C_6)$alkoxy such as methoxy, ethoxy, propyloxy, butyloxy, iso-propyloxy, which may be substituted;

When the groups represented by $R^1$, $R^2$ and the group $R^3$ when attached to carbon atom are substituted, the substituents may be selected from halogen or hydroxy.

The cyclic structure formed by $R^1$ and $R^2$ together with the carbon atoms to which they are attached may be a substituted or unsubstituted phenyl group. Suitable substituents on the cyclic structure formed by $R^1$ & $R^2$ together with the adjacent carbon atoms to which they are attached include hydroxy, halogen atom such as chlorine, bromine and iodine; $(C_1$-$C_3)$alkyl or $(C_1$-$C_3)$ alkoxy.

Suitable $R^3$ when attached to nitrogen atom is selected from hydrogen, substituted or unsubstituted, linear or branched $(C_1$-$C_6)$alkyl group, such as methyl, ethyl, n-propyl, isopropyl, n-butyl, iso-butyl, t-butyl, n-pentyl, isopentyl, hexyl; substituted or unsubstituted phenyl or naphthyl; and substituted or unsubstituted benzyl or phenethyl.

When the groups represented by $R^3$ attached to nitrogen are substituted, preferred substituents may be selected from halogen such as fluorine, chlorine; and hydroxy.

n is an integer ranging from 1-4. It is preferred that n be 1 or 2.

Ar represents divalent phenylene.

Suitable $R^4$ includes hydrogen, methyl, ethyl, hydroxy, $(C_1$-$C_3)$alkoxy, or $R^4$ together with $R^5$ represent a bond.

Suitable $R^5$ may be hydrogen, methyl, ethyl, hydroxy, $(C_1$-$C_3)$alkoxy, or together with $R^4$ forms a bond.

It is preferred that $R^4$ and $R^5$ represent hydrogen atom or $R^4$ and $R^5$ together represent a bond.

Suitable groups represented by $R^6$ may be selected from hydrogen; $(C_1$-$C_{12})$alkyl group such as methyl, ethyl,

n-propyl, iso-propyl, n-butyl, iso-butyl, pentyl, hexyl, octyl; phenyl; benzyl; $(C_1-C_6)$alkoxy$(C_1-C_6)$alkyl group such as methoxymethyl, ethoxymethyl, methoxyethyl, ethoxypropyl;

Suitable groups represented by $R^7$ may be selected from hydrogen; substituted or unsubstituted, linear or branched $(C_1-C_{12})$alkyl group such as methyl, ethyl, n-propyl, iso-propyl, n-butyl, iso-butyl, pentyl, hexyl, octyl; and phenyl, which may be substituted The substituents on $R^7$ may be selected from halogen.

Suitable groups represented by $R^8$ may be selected from hydrogen, and linear or branched $(C_1-C_{12})$alkyl.

The cyclic structure formed by $R^7$ and $R^8$ together with the nitrogen atom to which they are attached may be a 5 or 6 membered cyclic structure containing carbon atoms, a nitrogen atom and optionally an oxygen atom. The cyclic structure may contain one or more double bonds.

Suitable ring structures formed by $R^7$ and $R^8$ together may be selected from pyrrolidinyl, piperidinyl, morpholinyl, and oxazolinyl.

For any $R^1$, $R^2$, $R^3$, $R^4$, $R^5$, $R^6$, $R^7$, $R^8$ and Ar that may be substituted, the substituents are as defined above.

Suitable n is an integer ranging from 1 to 4, preferably n represents an integer 1 or 2.

The compounds of formula (I) where $R^6$ represents hydrogen atom and $R^7$ represents hydrogen or lower alkyl group have been claimed in our U.S. Patent 5,885,997 and U.S. Patent 5,985,884.

Pharmaceutically acceptable salts forming part of this invention include salts of the carboxylic acid moiety such as alkali metal salts like Li, Na, and K salts; alkaline earth metal salts like Ca and Mg salts; salts of organic bases such as lysine, arginine, guanidine, diethanolamine, choline, tromethamine; ammonium or substituted ammonium salts and aluminum salts. Salts may include acid addition salts where appropriate which are, sulphates, nitrates, phosphates, perchlorates, borates, hydrohalides, acetates, tartrates, maleates, citrates, succinates, palmoates, methane-sulphonates, benzoates, salicylates, hydroxynaphthoates, benzenesulfonates, ascorbates, glycerophosphates and ketoglutarates. Pharmaceutically acceptable solvates may be hydrates or comprising other solvents of crystallization such as alcohols.

Particularly useful compounds according to the present invention includes:

(±)-Ethyl 2-ethoxy-3-[4-[[3-methyl-4-oxo-3,4-dihydro-2-quinazolinyl] methoxy]phenyl]propanoate;
(±)-2-Ethoxy-3-[4-[[3-methyl-4-oxo-3,4-dihydro-2-quinazolinyl] methoxy] phenyl]propanoic acid;
(±)-Sodium 2-ethoxy-3-[4-[[3-methyl-4-oxo-3,4-dihydro-2-quinazolinyl] methoxy]phenyl]propanoate;
(+)-2-Ethoxy-3-[4-[[3-methyl-4-oxo-3,4-dihydro-2-quinazolinyl] methoxy] phenyl]propanoic acid;
(-)-2-Ethoxy-3-[4-[[3-methyl-4-oxo-3,4-dihydro-2-quinazolinyl]methoxy] phenyl]propanoic acid;
(-)-Sodium 2-ethoxy-3-[4-[[3-methyl-4-oxo-3,4-dihydro-2-quinazolinyl] methoxy]phenyl]propanoate;
(±)-(Morpholine-4-yl) 2-ethoxy-3-[4-[[3-methyl-4-oxo-3,4-dihydro-2-quinazolinyl]methoxy]phenyl]propanamide;
(±)-2-Ethoxy-3-[4-[[3-methyl-4-oxo-3,4-dihydro-2-quinazolinyl] methoxy] phenyl]-N-(2-fluorophenyl)propanamide;
(±)-Ethyl 2-methoxy-3-[4-[[3-methyl-4-oxo-3,4-dihydro-2-quinazolinyl] methoxy]phenyl]propanoate;
(±)-2-Methoxy-3-[4-[[3-methyl-4-oxo-3,4-dihydro-2-quinazolinyl]methoxy] phenyl]propanoic acid;
(±)-Ethyl 2-propoxy-3-[4-[[3-methyl-4-oxo-3,4-dihydro-2-quinazolinyl] methoxy]phenyl]propanoate;
(±)-2-Propoxy-3-[4-[[3-methyl-4-oxo-3,4-dihydro-2-quinazolinyl] methoxy] phenyl]propanoic acid;
(±)-Ethyl 2-(n-butoxy)-3-[4-[[3-methyl-4-oxo-3,4-dihydro-2-quinazolinyl] methoxy]phenyl]propanoate;
(±)-2-(n-Butoxy)-3-[4-[[3-methyl-4-oxo-3,4-dihydro-2-quinazolinyl] methoxy] phenyl]propanoic acid;
(±)-Ethyl 2-(n-octyloxy)-3-[4-[[3-methyl-4-oxo-3,4-dihydro-2-quinazolinyl] methoxy]phenyl]propanoate;
(±)-2-(n-Octyloxy)-3-[4-[[3-methyl-4-oxo-3,4-dihydro-2-quinazolinyl] methoxy] phenyl]propanoic acid>
(±)-Ethyl 2-benzyloxy-3-[4-[[3-methyl-4-oxo-3,4-dihydro-2-quinazolinyl] methoxy]phenyl] propanoate;
(±)-2-Benzyloxy-3-[4-[[3-methyl-4-oxo-3,4-dihydro-2-quinazolinyl] methoxy] phenyl]propanoic acid;
(±)-Ethyl 2-phenoxy 3-[4-[[3-methyl-4-oxo-3,4-dihydro-2-quinazolinyl] methoxy]phenyl]propanoate;
(±)-2-Phenoxy-3-[4-[[3-methyl-4-oxo-3,4-dihydro-2-quinazolinyl] methoxy] phenyl] propanoic acid;
(±)-Ethyl 2-(2-methoxyethoxy)-3-[4-[[3-methyl-4-oxo-3,4-dihydro-2-quinazolinyl]methoxy]phenyl]propanoate;
(±)-2-(2-Methoxyethoxy)-3-[4-[[3-methyl-4-oxo-3,4-dihydro-2-quinazolinyl] methoxy]phenyl]propanoic acid;
(±)-Ethyl 2-ethoxy-3-[4-[2-[2-ethyl-4-oxo-3,4-dihydro-3-quinazolinyl] ethoxy] phenyl]propanoate;
(±)-2-Ethoxy-3-[4-[2-[2-ethyl-4-oxo-3,4-dihydro-3-quinazolinyl] ethoxy] phenyl]propanoic acid;
(+) -2-Ethoxy-3-[4-[2-[2-ethyl-4-oxo-3,4-dihydro-3-quinazolinyl] ethoxy] phenyl]propanoic acid;
(-)-2-Ethoxy-3-[4-[2-[2-ethyl-4-oxo-3,4-dihydro-3-quinazolinyl] ethoxy] phenyl]propanoic acid;
(+)-Ethyl 2-ethoxy-3-[4-[2-[2-ethyl-4-oxo-3,4-dihydro-3-quinazolinyl] ethoxy] phenyl]propanoate;
(-)-Ethyl 2-ethoxy-3-[4-[2-[2-ethyl-4-oxo-3,4-dihydro-3-quinazolinyl] ethoxy] phenyl]propanoate;
(±)-Ethyl 2-ethoxy-3-[4-[2-[2-methyl-4-oxo-3,4-dihydro-3-quinazolinyl] ethoxy]phenyl]propanoate;
(±)-2-Ethoxy-3-[4-[2-[2-methyl-4-oxo-3,4-dihydro-3-quinazolinyl] ethoxy] phenyl]propanoic acid;
(+)-2-Ethoxy-3-[4-[2-[2-methyl-4-oxo-3,4-dihydro-3-quinazolinyl] ethoxy] phenyl]propanoic acid;
(-)-2-Ethoxy-3-[4-[2-[2-methyl-4-oxo-3,4-dihydro-3-quinazolinyl] ethoxy] phenyl]propanoic acid;
(+)-Ethyl 2-ethoxy-3-[4-[2-[2-methyl-4-oxo-3,4-dihydro-3-quinazolinyl] ethoxy]phenyl]propanoate;

(-)-Ethyl-2-ethoxy-3-[4-[2-[2-methyl-4-oxo-3,4-dihydro-3-quinazolinyl] ethoxy]phenyl]propanoate;
(±)-Ethyl 2-ethoxy-3-[4-[2-[4-oxo-3,4-dihydro-3-quinazolinyl) ethoxy] phenyl]propanoate;
(±)-2-Ethoxy-3-[4-[2-[4-oxo-3,4-dihydro-3-quinazolinyl]ethoxy]p phenyl] propanoic acid;
(±)-Ethyl 2-phenoxy-3-[4-[2-[2-ethyl-4-oxo-3,4-dihydro-3-quinazolinyl] ethoxy]phenyl]propanoate;
(±)-2-Phenoxy-3-[4-[2-[2-ethyl-4-oxo-3,4-dihydro-3-quinazolinyl] ethoxy] phenyl]propanoic acid;
(±)-Ethyl 2-phenoxy-3-[4-[2-[2-methyl-4-oxo-3,4-dihydro-3-quinazolinyl] ethoxy]phenyl]propanoate;
(±)-2-Phenoxy-3-[4-[2-[2-methyl-4-oxo-3,4-dihydro-3-quinazolinyl] ethoxy] phenyl]propanoic acid;
(±)-Ethyl 2-ethoxy-3-[4-[2-[2-ethyl-4-methyl-6-oxo-1-pyrimidinyl]ethoxy] phenyl]propanoate;
(±)-2-Ethoxy-3-[4-[2-[2-ethyl-4-methyl-6-oxo-1-pyrimidinyl] ethoxy] phenyl] propanoic acid;
(±)-Ethyl 2-ethoxy-3-[4-[[3-phenyl-4-oxo-3,4-dihydro-2-quinazolinyl] methoxy]phenyl]propanoate;
(±)-2-Ethoxy-3-[4-[2-[2-ethyl-4-methyl-6-oxo-1-pyrimidinyl] ethoxy] phenyl] propanoic acid;
(±)-Ethyl 2-ethoxy-3-[4-[[3-phenyl-4-oxo-3,4-dihydro-2-quinazolinyl] methoxy]phenyl]propanoate;
(±)-2-Ethoxy-3-[4-[[3-phenyl-4-oxo-3,4-dihydro-2-quinazolinyl] methoxy] phenyl]propanoic acid;
(±)-Ethyl 2-ethoxy-3-[4-[[3-methyl-4-oxo-3,4-dihydro-6,7-dimethoxy-2-quinazolinyl]methoxy]phenyl]propanoate;
(±)- 2-Ethoxy-3-[4-[[3-methyl-4-oxo-3,4-dihydro-6,7-dimethoxy-2-quinazolinyl]methoxy] phenyl]propanoic acid;
(±)-Ethyl 2-ethoxy-3-[4-[[3-(4-methylphenyl)-4-oxo-3,4-dihydro-2-quinazolinyl]methoxy] phenyl]propanoate;
(±)-2-Ethoxy-3-[4-[[3-(4-methylphenyl)-4-oxo-3,4-dihydro-2-quinazolinyl] ethoxy] phenyl]propanoic acid;
(±)-Ethyl 2-ethoxy-3-[4-[[3-(4-methoxyphenyl)-4-oxo-3,4-dihydro-2-quinazolinyl]methoxy] phenyl]propanoate;
(±)-2-Ethoxy-3-[4-[[3-(4-methoxyphenyl)-4-oxo-3,4-dihydro-2-quinazolinyl] methoxy]phenyl]propanoic acid;
(±)-Ethyl 2-ethoxy-3-[4-[[3-benzyl-4-oxo-3,4-dihydro-2-quinazolinyl] methoxy]phenyl]propanoate;
(±)-2-Ethoxy-3-[4-[[3-benzyl-4-oxo-3,4-dihydro-2-quinazolinyl] methoxy] phenyl]propanoic acid;
(±)-Ethyl 2-ethoxy-3-[4-[[3-(3-chlorophenyl)-4-oxo-3,4-dihydro-2-quinazolinyl]methoxy]phenyl]propanoate;
(±)-2-Ethoxy-3-[4-[[3-(3-chlorophenyl)-4-oxo-3,4-dihydro-2-quinazolinyl] methoxy]phenyl]propanoic acid;
(±)-Ethyl 2-ethoxy-3-[4-[[3-(3-chloro-4-fluorophenyl)-4-oxo-3,4-dihydro-2-quinazolinyl]methoxy]phenyl]propanoate; and
(±)-2-Ethoxy-3-[4-[[3-(3-chloro-4-fluorophenyl)-4-oxo-3,4-dihydro-2-quinazolinyl]methoxy]phenyl]propanoic acid.

The compound of general formula (I) where $R^4$ and $R^5$ together represent a bond, Y represents an oxygen atom, $R^1$, $R^2$, $R^3$, $R^6$, $R^7$, X, n and Ar are as defined earlier, can be prepared by any of the following routes shown in Scheme-I below.

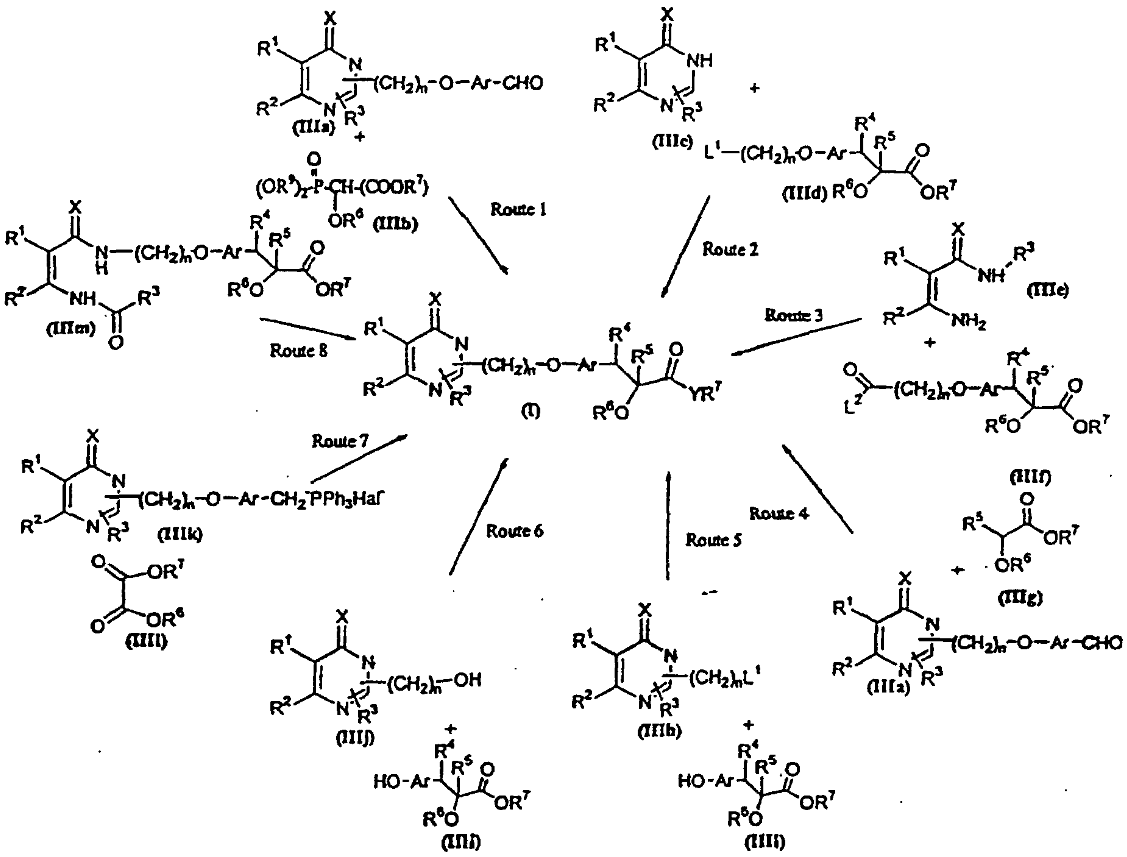

**Scheme - I**

Route (1): The reaction of a compound of the general formula (IIIa), where all symbols are as defined earlier, with a compound of formula (IIIb), where $R^6$ and $R^7$ are as defined earlier excluding hydrogen, and $R^9$ represents $(C_1-C_6)$alkyl, to yield compound of general formula (I) where $R^6$ and $R^7$ are as defined above excluding hydrogen, and all other symbols are as defined above, may be carried out in the presence of a base such as alkali metal hydrides like NaH, or KH or organolithiums like $CH_3Li$, BuLi, and the like, or alkoxides such as NaOMe, NaOEt, $K^+BuO^-$ or mixtures thereof. The reaction may be carried out in the presence of solvents such as THF, dioxane, DMF, DMSO, DME, and the like, or mixtures thereof. HMPA may be used as a cosolvent. The reaction temperature may range from -78°C to 50°C, preferably at a temperature in the range of -10°C to 30°C. The reaction is more effective under anhydrous conditions. The compound of general formula (IIIb) may be prepared by Arbuzov reaction.

Alternatively, the compound of formula (I) may be prepared by reacting the compound of formula (IIIa), where all symbols are as defined earlier, with Wittig reagents such as $Hal^-Ph_3P^+CH-(OR^6)CO_2R^7$ under similar reaction conditions as described above.

Route (2): The reaction of a compound of general formula (IIIc), where all symbols are as defined earlier, with a compound of general formula (IIId), where $R^4$ and $R^5$ together represent a bond and all symbols are as defined earlier, and $L^1$ is a leaving group such as halogen atom, p-toluenesulfonate, methanesulfonate, trifluoromethanesulfonate, preferably a halogen atom, to produce a compound of general formula (I) where $-(CH_2)_n-$ linker group is attached through the nitrogen atom and all other symbols are as defined above, may be carried out in the presence of solvents such as DMSO, DMF, DME, THF, dioxane, ether, and the like, or a combination thereof. The reaction may be carried out in an inert atmosphere which may be maintained by using inert gases such as $N_2$, Ar, or He. The reaction may be effected in the presence of a base such as alkalis like sodium hydroxide, or potassium hydroxide, alkali metal carbonates like sodium carbonate, or potassium carbonate; alkali metal hydrides such as sodium hydride or potassium hydride; organometallic bases like n-butyl lithium, alkali metal amides like sodamide or mixtures thereof. The amount of base may range from 1 to 5 equivalents, based on the amount of the compound of formula (IIIc), preferably the amount of base ranges from 1 to 3 equivalents. Phase transfer catalysts such as tetraalkylammonium halide or hydroxide may be added. Additives such as alkali metal halides such as LiBr may be added. The reaction may be carried out at a temperature in the range of 0°C to 150°C, preferably at a temperature in the range of 15°C to 100°C. The duration of

the reaction may range from 0.25 to 48 hours, preferably from 0.25 to 12 hours.

Route (3): The reaction of compound of general formula (IIIe) with a compound of general formula (IIIf) where $R^4$, $R^5$ together represent a bond, $L^2$ is halogen, -OH, $-OR^{10}$, $-O-C(=O)-OR^{10}$, where $R^{10}$ is $(C_1-C_5)$alkyl, and other symbols are as defined earlier, to produce a compound of general formula (I) where $-(CH_2)_n$-linker group is attached through the carbon atom and all other symbols are as defined above, may be carried out in the presence of solvents such as xylene, toluene, THF, dioxane, acetic acid, DMF, DMSO, and the like, or mixtures thereof. The reaction may be carried out in an inert atmosphere which may be maintained by using inert gases such as $N_2$, Ar or He. The reaction may be carried out at a temperature in the range of 50°C to 200°C, preferably at a temperature in the range of 60° C to 180°C. The reaction may be effected in the presence or in absence of a base or an acid. The nature of the base or the acid is not critical. Examples of such bases include organic bases such as pyridine, lutidine, triethyl amine, diisopropylethyl amine, and the like, and metal carbonates such as $K_2CO_3$, or $Na_2CO_3$. Examples of acids include organic acids such as AcOH, $C_2H_5COOH$, butyric acid, trifluoroacetic acid, p-toluenesulfonic acid, benzenesulfonic acid, and the like, and mineral acids such as HCl, HBr etc. The duration of the reaction may range from 0.25 to 48 hours, preferably from 0.50 to 18 hours. This process is preferably used for the preparation of a compound of formula (I) wherein $R^1$ and $R^2$ together represent a cyclic structure defined earlier.

Route (4): The reaction of a compound of the general formula (IIIa) where all symbols are as defined earlier, with a compound of formula (IIIg), where $R^5$ is hydrogen and all other symbols are as defined earlier, may be carried out in the presence of a base. The nature of the base is not critical. Any base normally employed for aldol condensation reaction may be employed; bases like metal hydride such as NaH, or KH; metal alkoxides such as NaOMe, $t\text{-}BuO^-K^+$, or NaOEt; or metal amides such as $LiNH_2$, $LiN(ipr)_2$ may be used. Aprotic solvents such as THF, ether, dioxane may be used. The reaction may be carried out in an inert atmosphere which may be maintained by using inert gases such as $N_2$, Ar, or He and the reaction is more effective under anhydrous conditions. Temperature in the range of -80°C to 35°C may be used. The β-hydroxy product initially produced may be dehydrated under conventional dehydration conditions such as treating with PTSA in solvents such as benzene or toluene. The nature of solvent and dehydrating agent is not critical. Temperatures in the range of 20°C to reflux temperature of the solvent used may be employed, preferably at reflux temperature of the solvent by continuous removal of water using a Dean Stark water separator.

Route (5): The reaction of compound of formula (IIIh), where all symbols are as defined earlier and $L^1$ represents a halogen atom, p-toluene-sulfonate, methanesulfonate or trifluoromethanesulfonate, preferably a halogen atom, with compound of formula (IIIi), where $R^4$ and $R^5$ together represent a bond and all other symbols are as defined earlier, to produce a compound of the formula (I) defined above may be carried out in the presence of aprotic solvents such as THF, DMF, DMSO, DME, and the like, or mixtures thereof. The reaction may be carried out in an inert atmosphere which may be maintained by using inert gases such as $N_2$, Ar, or He. The reaction may be effected in the presence of a base such as $K_2CO_3$, $Na_2CO_3$ or NaH or mixtures thereof. Acetone may be used as solvent when $Na_2CO_3$ or $K_2CO_3$ is used as a base. The reaction temperature may range from 0°C-120°C, preferably at a temperature in the range of 30°C-100°C. The duration of the reaction may range from 1 to 24 hours, preferably from 2 to 12 hours. The compound of formula (IIIi) can be prepared according to known procedure by a Wittig Horner reaction between the hydroxy protected aryl aldehyde such as benzyloxyaryl aldehyde and compound of formula (IIIb), followed by deprotection.

Route (6): The reaction of compound of general formula (IIIj), where all symbols are as defined earlier, with a compound of general formula (IIIi), where $R^4$ and $R^5$ together represent a bond and all symbols are as defined earlier, may be carried out using suitable coupling agents such as dicyclohexyl urea, or triarylphosphine/dialkylazadicarboxylate such as $PPh_3$/DEAD, and the like. The reaction may be carried out in the presence of solvents such as THF, DME, $CH_2Cl_2$, $CHCl_3$, toluene, acetonitrile, carbon tetrachloride, and the like. The inert atmosphere may be maintained by using inert gases such as $N_2$, Ar, or He. The reaction may be effected in the presence of DMAP, HOBT and they may be used in the range of 0.05 to 2 equivalents, preferably 0.25 to 1 equivalents. The reaction temperature may be in the range of 0°C to 100°C, preferably at a temperature in the range of 20°C to 80°C. The duration of the reaction may range from 0.5 to 24 hours, preferably from 6 to 12 hours.

Route 7: The reaction of a compound of formula (IIIk), where all symbols are as defined earlier, with a compound of formula (IIIl), where $R^6 = R^7$ and are as defined earlier excluding hydrogen, to produce a compound of the formula (I), where $R^4$ and $R^5$ together represent a bond, may be carried out neat in the presence of a base such as alkali metal hydrides like NaH, KH or organolithiums like $CH_3Li$, BuLi, and the like, or alkoxides such as NaOMe, NaOEt, $t\text{-}BuO^-K^+$, and the like, or mixtures thereof The reaction may be carried out in the presence of aprotic solvents such as THF, dioxane, DMF, DMSO, DME, and the like, or mixtures thereof. HMPA may be used as cosolvent. The reaction temperature may range from -78°C to 100°C, preferably at a temperature in the range of -10°C to 50°C.

Route 8: The cyclization of compound of general formula (IIIm), where $R^4$ and $R^5$ together represent a bond, $R^7$ is as defined earlier excluding hydrogen atom, and all other symbols are as defined earlier, to produce a compound of general formula (I), where $-(CH_2)n-$ linker group is attached through nitrogen atom and all other symbols are as defined earlier, may be carried out neat or in the presence of solvents such as xylene, toluene, THF, dioxane, acetic acid, DMF, DMSO, and the like, or mixtures thereof. The reaction may be carried out in an inert atmosphere which may

be maintained by using inert gases such as $N_2$, Ar or He. The reaction may be carried out at a temperature in the range of 50°C to 200°C, preferably at a temperature in the range of 60°C to 180°C. The reaction may be effected in the presence or in the absence of a base or an acid. The nature of the base or the acid is not critical. Examples of such bases include organic bases such as pyridine, lutidine, triethyl amine, diisopropylethyl amine, and the like; metal carbonates such as $K_2CO_3$, or $Na_2CO_3$. Examples of acids include organic acids such as AcOH, $C_2H_5COOH$, butyric acid, trifluoroacetic acid, p-toluenesulfonic acid, benzenesulfonic acid, and the like, or mineral acids such as HCl, HBr, etc. The duration of the reaction may range from 0.25 to 48 hours, preferably from 0.50 to 18 hours. This process is preferably used for the preparation of a compound of formula (I) wherein $R^1$ and $R^2$ together represent a cyclic structure as defined earlier.

In accordance with the present invention, the compound of the general formula (I) where $R^1$, $R^2$, $R^3$, $R^4$, $R^5$, $R^6$, $R^7$, X, n and Ar are as defined earlier and Y represents oxygen atom can be prepared by one or more of the processes shown in Scheme-II below.

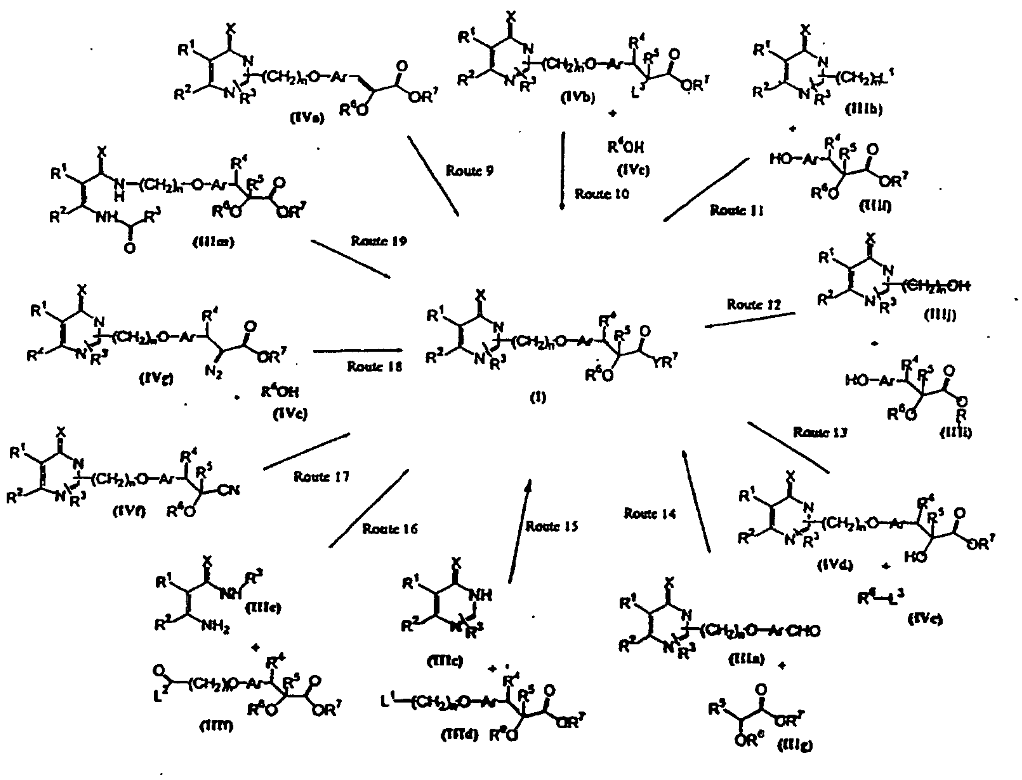


**Scheme - II**

Route 9: The reduction of compound of the formula (IVa), which represents a compound of formula (I) where $R^4$ and $R^5$ together represent a bond and Y represents oxygen atom and all other symbols are as defined earlier, obtained as described earlier(Scheme-I), to yield a compound of the general formula (I) where $R^4$ and $R^5$ each represent hydrogen atom and all symbols are as defined earlier, may be carried out in the presence of gaseous hydrogen and a catalyst such as Pd/C, Rh/C, Pt/C, and the like. Mixtures of catalysts may be used. The reaction may also be conducted in the presence of solvents such as dioxane, acetic acid, and ethyl acetate, or alcohol such as methanol, ethanol, and the like. A pressure between atmospheric pressure and 0.55 MPa (80 psi) may be employed. The catalyst may be preferably 5-10 % Pd/C and the amount of catalyst used may range from 5-100% w/w. The reaction may also be carried out by employing metal solvent reduction such as magnesium in alcohol or sodium amalgam in alcohol, preferably methanol. The hydrogenation may be carried out in the presence of metal catalysts containing chiral ligands to obtain a compound of formula (I) in optically active form. The metal catalyst may contain Rhodium, Ruthenium, Indium, and the like. The chiral ligands may preferably be chiral phosphines such as optically pure enantiomers of 2,3-bis(diphenylphosphino)butane, 1,2-bis(diphenylphosphino)ethane, 1,2-bis(2-methoxyphenyl phenylphosphino)ethane, 2,3-isopropylidene-2,3-dihydroxy-1,4-bis(diphenylphosphino) butane, and the like. Any suitable chiral catalyst may be employed which would give required optical purity of the product (I) (Ref: Principles of Asymmetric Synthesis, Tetrahedron Series Vol 14, pp311-316, Ed. Baldwin J. E.).

Route 10: The reaction of compound of formula (IVb), where $R^7$ is as defined earlier excluding hydrogen, all other symbols are as defined earlier, and $L^3$ is a halogen atom, with an alcohol of general formula (IVc), where $R^6$ is as defined earlier excluding hydrogen, to produce a compound of the formula (I) defined earlier, may be carried out in the presence of solvents such as THF, DMF, DMSO, DME, and the like, or mixtures thereof. The reaction may be carried out in an inert atmosphere which may be maintained by using inert gases such as $N_2$, Ar, or He. The reaction may be effected in the presence of a base such as KOH, NaOH, NaOMe, NaOEt, t-$BuO^-K^+$ or NaH or mixtures thereof. Phase transfer catalysts such as tetraalkylammonium halides or hydroxides may be employed. The reaction temperature may range from 20°C-120°C, preferably at a temperature in the range of 30°C-100°C. The duration of the reaction may range from 1 to 12 hours, preferably from 2 to 6 hours. The compound of general formula (IVb), where $R^7$ represents hydrogen or lower alkyl group, and its preparation has been disclosed in U.S. Patents 5,885,997 and 5,985,884.

Route 11: The reaction of compound of formula (IIIh) defined earlier with compound of formula (IIIi), where all symbols are as defined earlier, to produce a compound of the formula (I) defined above, may be carried out in the presence of solvents such as THF, DMF, DMSO, DME, and the like, or mixtures thereof. The reaction may be carried out in an inert atmosphere which is maintained by using inert gases such as $N_2$, Ar or He. The reaction may be effected in the presence of a base such as $K_2CO_3$, $Na_2CO_3$ or NaH or mixtures thereof. Acetone may be used as a solvent when $K_2CO_3$ or $Na_2CO_3$ is used as a base. The reaction temperature may range from 20°C-120°C, preferably at a temperature in the range of 30°C-80°C. The duration of the reaction may range from 1 to 24 hours, preferably from 2 to 12 hours. The compound of formula (IIIi) may be prepared by Wittig Homer reaction between the protected hydroxyaryl aldehyde and compound of formula (IIIb) followed by reduction of the double bond and deprotection. Alternatively, the compound of formula (IIIi) may be prepared by following a procedure disclosed in WO 94/01420.

Route 12: The reaction of compound of general formula (IIIj) defined earlier with a compound of general formula (IIIi), where all symbols are as defined above, may be carried out using suitable coupling agents such as dicyclohexyl urea, triaryl-phosphine/dialkylazadicarboxylate such as $PPh_3$/DEAD, and the like. The reaction may be carried out in the presence of solvents such as THF, DME, $CH_2Cl_2$, $CHCl_3$, toluene, acetonitrile, carbon tetrachloride, and the like. The inert atmosphere may be maintained by using inert gases such as $N_2$, Ar, or He. The reaction may be effected in the presence of DMAP, HOBT and they may be used in the range of 0.05 to 2 equivalents, preferably 0.25 to 1 equivalents. The reaction temperature may be in the range of 0°C to 100°C, preferably at a temperature in the range of 20°C to 80°C. The duration of the reaction may range from 0.5 to 24 hours, preferably from 6 to 12 hours.

Route 13: The reaction of compound of formula (IVd), which represents a compound of formula (I) where $R^6$ represents hydrogen atom and all other symbols are as defined above, with a compound of formula (IVe) where $R^6$ is as defined earlier excluding hydrogen, and $L^3$ is a halogen atom, may be carried out in the presence of solvents such as THF, DMF, DMSO, DME, and the like. The inert atmosphere may be maintained by using inert gases such as $N_2$, Ar or He. The reaction may be effected in the presence of a base such as KOH, NaOH, NaOMe, t-$BuO^-K^+$, NaH, and the like. Phase transfer catalyst such as tetraalkylammonium halides or hydroxides may be employed. The reaction temperature may range from 20°C to 150°C, preferably at a temperature in the range of 30°C to 100°C. The duration of the reaction may range from 1 to 24 hours, preferably from 2 to 6 hours.

The compound of formula (IVd), where $R^7$ represents hydrogen or lower alkyl group,and its preparation has been disclosed in U.S. Patents 5,885,997 and 5,985,884. The compound of formula (IVd) represents a compound of formula (I) where $R^6$ represents hydrogen atom and all other symbols are as defined earlier.

Route 14: The reaction of a compound of the general formula (IIIa) as defined above with a compound of formula (IIIg) , where $R^5$ is hydrogen and all other symbols are as defined earlier, may be carried out under conventional conditions. The base is not critical. Any base normally employed for aldol condensation reaction may be employed, metal hydride such as NaH, or KH; metal alkoxides such as NaOMe, t-$BuO^-K^t$, or NaOEt; or metal amides such as $LiNH_2$, or $LiN(iPr)_2$. Aprotic solvent such as THF may be used. Inert atmosphere may be employed such as argon and the reaction is more effective under anhydrous conditions. Temperature in the range of -80°C to 25°C may be used. The β-hydroxy aldol product may be dehydroxylated using conventional methods, conveniently by ionic hydrogenation technique such as by treating with a trialkyl silane in the presence of an acid such as trifluoroacetic acid. Solvent such as $CH_2Cl_2$ may be used. Favorably, reaction proceeds at 25°C. Higher temperature may be employed if the reaction is slow.

Route 15: The reaction of a compound of general formula (IIIc), where all symbols are as defined earlier, with a compound of general formula (IIId) where $L^1$ is a halogen atom, p-toluenesulfonate, methanesulfonate or trifluoromethanesulfonate, preferably a halogen atom, and all other symbols are as defined earlier, to produce a compound of general formula (I), where $-(CH_2)_n-O-$ is attached through nitrogen atom and all other symbols are as defined above, may be carried out in the presence of solvents such as DMSO, DMF, DME, THF, dioxane, ether, and the like, or a combination thereof. The reaction may be carried out in an inert atmosphere which may be maintained by using inert

gases such as $N_2$, Ar, or He. The reaction may be effected in the presence of a base such as alkalis like sodium hydroxide, potassium hydroxide, alkali metal carbonates like sodium carbonate, or potassium carbonate; alkali metal hydrides such as sodium hydride or potassium hydride; organometallic bases like n-butyl lithium, alkali metal amides like sodamide or mixtures thereof. The amount of base may range from 1 to 5 equivalents, based on the amount of the compound of formula (IIIc), preferably the amount of base ranges from 1 to 3 equivalents. Additives such as alkali metal halides such as LiBr may be added. The reaction may be carried out at a temperature in the range of 0°C to 150°C, preferably at a temperature in the range of 15°C to 100°C. The duration of the reaction may range from 0.25 to 24 hours, preferably from 0.25 to 12 hours.

Route 16: The reaction of compound of general formula (IIIe) as defined earlier with a compound of general formula (IIIf), where $L^2$ is halogen, and all other symbols are as defined earlier, to produce a compound of general formula (I) where $-(CH_2)_n-O-$ is attached through carbon atom and all other symbols are as defined above, may be carried out in neat or in the presence of solvents such as xylene, toluene, THF, dioxane, acetic acid, DMF, DMSO, and the like, or mixtures thereof. The reaction may be carried out in an inert atmosphere which may be maintained by using inert gases such as $N_2$, Ar or He. The reaction may be carried out at a temperature in the range of 50°C to 200°C, preferably at a temperature in the range of 60°C to 180°C. The reaction may be effected in the presence or in absence of a base or an acid. The nature of the base or the acid is not critical. Examples of such bases include organic bases such as pyridine, lutidine, triethyl amine, diisopropylethyl amine, and the like; metal carbonates such as $K_2CO_3$, or $Na_2CO_3$. Examples of acids include organic acids such as AcOH, $C_2H_5COOH$, butyric acid, trifluoroacetic acid, p-toluenesulfonic acid, benzenesulfonic acid,and the like, mineral acids such as HCl, or HBr, etc. The duration of the reaction may range from 0.25 to 48 hours, preferably from 0.50 to 18 hours. This process is preferably used for the preparation of a compound of formula (I) wherein $R^1$ and $R^2$ together represent a cyclic structure defined earlier.

Route 17: The conversion of compound of formula (IVf), where all symbols are as defined earlier, to a compound of formula (I) may be carried out either in the presence of base or acid and the selection of base or acid is not critical. Any base normally used for hydrolysis of nitrile to acid may be employed, metal hydroxides such as NaOH, KOH in an aqueous solvent or any acid normally used for hydrolysis of nitrile to ester may be employed such as dry HCl in an excess of alcohol such as methanol, ethanol, propanol, etc. The reaction may be carried out at a temperature in the range of 0°C to reflux temperature of the solvent used, preferably at a temperature in the range of 25°C to reflux temperature of the solvent used. The duration of the reaction may range from 0.25 to 48 hrs.

Route 18: The reaction of a compound of formula (IVg), where $R^7$ is as defined earlier excluding hydrogen and all symbols are as defined earlier, with a compound of formula (IVc), where $R^6$ is as defined earlier excluding hydrogen, to produce a compound of formula (I) (by a rhodium carbenoid mediated insertion reaction),may be carried out in the presence of rhodium (II) salts such as rhodium (II) acetate. The reaction may be carried out in the presence of solvents such as benzene, toluene, dioxane, ether, THF, and the like, or a combination thereof or, when practicable, in the presence of $R^6OH$ as solvent at any temperature providing a convenient rate of formation of the required product, generally at an elevated temperature, such as reflux temperature of the solvent. The inert atmosphere may be maintained by using inert gases such as $N_2$, Ar, or He. The duration of the reaction may be range from 0.5 to 24 h, preferably from 0.5 to 6 h.

Route 19: The cyclization of compound of general formula (IIIm), where $R^7$ is as defined earlier excluding hydrogen atom and all other symbols are as defined above, to produce a compound of general formula (I), where $-(CH_2)_n-O-$ linker group is attached through nitrogen atom and all other symbols are as defined earlier, may be carried out neat or in the presence of solvents such as xylene, toluene, THF, dioxane, acetic acid, DMF, DMSO, and the like, or mixtures thereof. The reaction may be carried out in an inert atmosphere which may be maintained by using inert gases such as $N_2$, Ar or He. The reaction may be carried out at a temperature in the range of 50°C to 200°C, preferably at a temperature in the range of 60°C to 180°C. The reaction may be effected in the presence or in absence of a base or an acid. The nature of the base or the acid is not critical. Examples of such bases include organic bases such as pyridine, lutidine, triethyl amine, diisopropylethyl amine, and the like; or metal carbonates such as $K_2CO_3$, or $Na_2CO_3$. Examples of acids include organic acids such as AcOH, $C_2H_5COOH$, butyric acid, trifluoroacetic acid, p-toluenesulfonic acid, benzenesulfonic acid, and the like, or mineral acids such as HCl, HBr, etc. The duration of the reaction may range from 0.25 to 48 hours, preferably from 0.50 to 18 hours. This process is preferably used for the preparation of a compound of formula (I) wherein $R^1$ and $R^2$ together represent a cyclic structure as defined earlier.

The compound of general formula (I), where $R^7$ represents hydrogen atom and Y represents oxygen atom, may be prepared by hydrolysing using conventional methods, a compound of formula (I) where $R^7$ represents all groups defined earlier except hydrogen. The hydrolysis may be carried out in the presence of a base such as $Na_2CO_3$ and a suitable solvent such as methanol, ethanol, and the like, or mixtures thereof. The reaction may be carried out at a temperature in the range of 20-40°C, preferably at 25-30°C. The reaction time may range from 2 to 12 h, preferably from 4 to 8 h.

The compound of general formula (I), where Y represents oxygen and $R^7$ represents hydrogen or a $(C_1-C_{12})$ alkyl group, may be converted to compound of formula (I), where Y represents $NR^8$, by reaction with appropriate amines

of formula $NHR^7R^8$ where $R^7$ and $R^8$ are as defined earlier. Alternatively, the compound of formula (I) where $YR^7$ represents OH may be converted to acid halide, preferably $YR^7$ = Cl, by reacting with appropriate reagents such as oxalyl chloride, thionyl chloride and the like, followed by treatment with amines of formula $NHR^7R^8$ where $R^7$ and $R^8$ are as defined earlier. Alternatively, mixed anhydrides may be prepared from compound of formula (I), where $YR^7$ represents OH and all other symbols are as defined earlier, by treating with acid halides such acetyl chloride, acetyl bromide, pivaloyl chloride, dichlorobenzoyl chloride, and the like. The reaction may be carried out in the presence of suitable base such as pyridine, triethylamine, diisopropyl ethyl amine, and the like. Solvents such as halogenated hydrocarbons like $CHCl_3$, or $CH_2Cl_2$; hydrocarbons such as benzene, toluene, xylene, and the like, may be used. The reaction may be carried out at a temperature in the range of -40°C to 40°C, preferably 0°C to 20°C. The acid halide or mixed anhydride thus prepared may further be treated with appropriate amines of formula $NHR^7R^8$ where $R^7$ and $R^8$ are as defined earlier.

In another embodiment of the present invention there is provided the novel intermediates of formula (IVf)

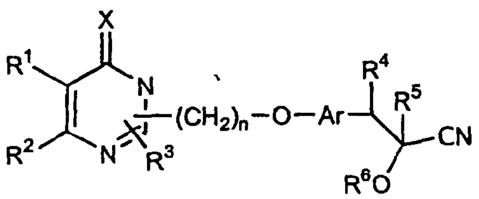

(IVf)

wherein X, $R^1$, $R^2$ ,$R^3$ , n, Ar , $R^4$, $R^5$ and $R^6$ are as defined above for formula (I), and a process for its preparation, and its use in the preparation of β-aryl-α-oxysubstituted alkylcarboxylic acids is provided (Scheme-III).

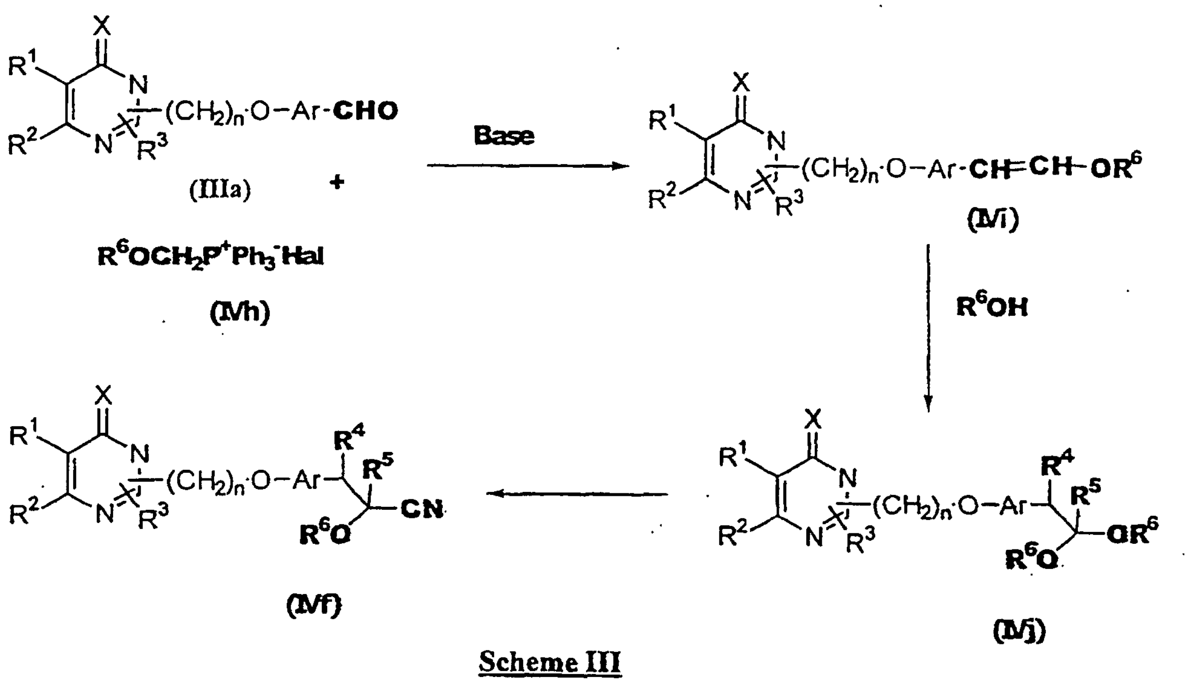


**Scheme III**

The reaction of a compound of formula. (IIIa) where all symbols are as defined earlier with a compound of formula (IVh), where $R^6$ is as defined earlier excluding hydrogen and Hal represent a halogen atom such as Cl, Br, or I, to produce a compound of formula (IVi), where all symbols are defined earlier and $R^6$ is as defined earlier excluding hydrogen, may be carried out under conventional conditions in the presence of a base. The base is not critical. Any base normally employed for Wittig reaction may be employed, metal hydride such as NaH, or KH; metal alkoxides such as NaOMe, $K^tBuO^-$, or NaOEt; or metal amides such as $LiNH_2$, or $LiN(iPr)_2$. Aprotic solvent such as THF, DMSO, dioxane, DME, and the like, may be used. Mixture of solvents may be used. HMPA may be used as cosolvent. Inert atmosphere may be employed such as argon and the reaction is more effective under anhydrous conditions. Temperature in the range of -80°C to 100°C may be used.

The compound of formula (IVi), where all symbols are as defined earlier and $R^6$ is as defined earlier excluding hydrogen, may be converted to a compound of formula (IVj) where $R^4$ and $R^5$ represent H atoms, $R^6$ is as defined earlier excluding hydrogen and all other symbols are as defined earlier, by treating with an alcohol under anhydrous conditions in the presence of a strong anhydrous acid such as p-toluenesulfonic acid.

The compound of formula (IVj) defined above upon treatment with trialkylsilyl cyanide such as trimethylsilyl cyanide produces a compound of formula (IVf) where $R^4$ and $R^5$ represent H atoms, $R^6$ is as defined earlier excluding hydrogen and all other symbols are as defined earlier.

In still another embodiment of the present invention there is provided the novel intermediates of formula (IVg)

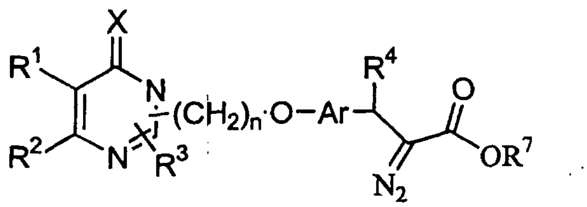

(IVg)

wherein X, $R^1$, $R^2$, $R^3$, n, Ar, $R^4$, and $R^7$ are as defined above for formula (I), and a process for its preparation and its use in the preparation of β-aryl-α-oxysubstituted alkylcarboxylic acids is provided.

The compound of formula (IVg) where all other symbols are as defined earlier may be prepared by reacting a compound of formula (IVk)

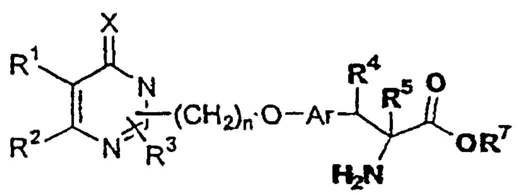

(IVk)

where $R^5$ is hydrogen atom and all other symbols are as defined earlier, with an appropriate diazotizing agent.

The diazotization reaction may be under conventional conditions. A suitable diazotizing agent is an alkyl nitrile, such as iso-amyl nitrile. The reaction may be carried out in presence of solvents such as THF, dioxane, ether, benzene, and the like, or a combination thereof. Temperatures in the range of -50°C to 80 may be used. The reaction may be carried out in an inert atmosphere which may be maintained by using inert gases such as $N_2$, Ar or He. The duration of the reaction may range from 1 to 24 h, preferably, 1 to 12 h.

The compound of formula (IVk) may also be prepared by a reaction between (IIIh), where all symbols are as defined earlier, and a compound of formula (IV1)

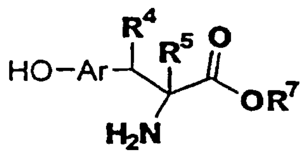

(IVl)

where $R^5$ is hydrogen atom and all other symbols are as defined earlier.

The reaction of compound of formula (IIIh), where all symbols are as defined earlier, and a compound of formula (IVl) where all symbols are as defined earlier, may be carried out in the presence of solvents such as THF, DMF, DMSO, DME, and the like, or mixtures thereof. The reaction may be carried out in an inert atmosphere which is maintained by using inert gases such as $N_2$, Ar or He. The reaction may be effected in the presence of a base such as $K_2CO_3$, $Na_2CO_3$ or NaH or mixtures thereof. Acetone may be used as a solvent when $K_2CO_3$ or $Na_2CO_3$ is used as a base. The reaction temperature may range from 20°C-120°C, preferably at a temperature in the range of 30°C-80°C. The duration of the reaction may range from 1 to 24 hours, preferably from 2 to 12 hours.

In another embodiment of the present invention there is provided the novel intermediate of formula (IIIm)

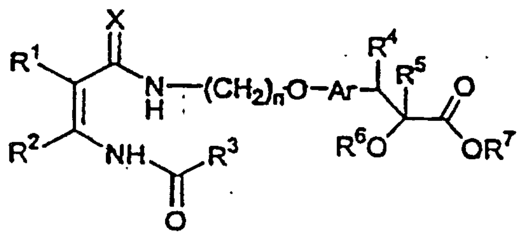

(IIIm)

wherein X, $R^1$, $R^2$, $R^3$, n, Ar, $R^4$ , $R^5$, $R^6$ and $R^7$ are as defined above for formula (I), and a process for its preparation and its use in the preparation of β-aryl-α-oxysubstituted alkylcarboxylic acids is provided.

The compound of formula (IIIm), where all symbols are as defined earlier, may be prepared by reacting a compound of formula (IIIn)

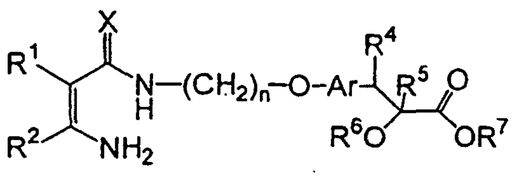

(IIIn)

where all symbols are as defined earlier, with a compound of formula (IIIo)

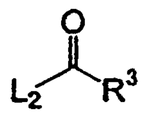

(IIIo)

where $L^2$ is halogen, and $R^3$ is as defined earlier.

The reaction of compound of formula (IIIn), where $R^7$ is as defined earlier excluding hydrogen and all other symbols are as defined above, to produce a compound of general formula (IIIo), where all symbols are as defined above, to produce a compound of general formula (IIIm), all symbols are as defined above, may be carried out in neat or in the presence of solvents such as xylene, toluene, THF, dioxane, acetic acid, DMF, DMSO and the like of mixtures thereof. The reaction may be carried out in an inert atmosphere which may be maintained by using inert gases such as $N_2$, Ar or He. The reaction may be carried out at a temperature in the range of -10°C to 80°C, preferably at a temperature in the range of O°C to 60°C. The reaction may be effected in the presence or in absence of a base or an acid. The nature of the base or the acid is not critical. Bases such as pyridine, lutidine, triethyl amine, diisopropylethyl amine, and the like, and acids such as AcOH, $C_2H_5COOH$, butyric acid, trifluoroacetic acid, p-toluenesulfonic acid, benzenesulfonic acid and the like, may be used. The duration of the reaction may range from 0.25 to 24 hours, preferably from 0.50 to 6 hours.

In yet another embodiment of the present invention there is provided the novel intermediates of formula (IIIn)

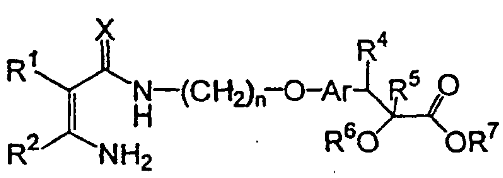

(IIIn)

wherein X, $R^1$, $R^2$, n, Ar, $R^4$, $R^5$, $R^6$, and $R^7$ are as defined above for formula (I), and a process for its preparation and its use in the preparation of β-aryl-α-oxysubstituted alkylcarboxylic acids is provided.

The compound of formula (IIIn), where all symbols are as defined above, may be prepared by reacting a compound of formula (IVm)

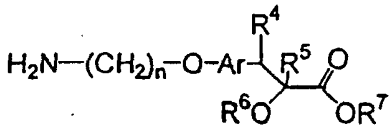

**(IVm)**

where all symbols are as defined earlier, with a compound of formula (IVo)

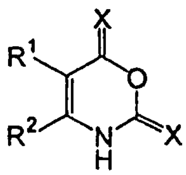

**(IVo)**

The reaction of compound of formula (IVm), where all symbols are as defined earlier, with a compound of formula (IVo), where $R^1$, $R^2$ and X are as defined earlier, to produce a compound of formula (IIIn) defined earlier, may be carried out neat or in the presence of solvents such as xylene, toluene, dioxane, THF, DMF, DMSO, DME, and the like, or their mixtures. The reaction may be carried out in an inert atmosphere which is maintained by using inert gases such as $N_2$, Ar or He. The reaction temperature may range from 0°C-150°C, preferably at a temperature in the range of 0°C-120°C. The duration of the reaction may range from 0.5 to 12 hours, preferably from 0.5 to 6 hours.

The pharmaceutically acceptable salts are prepared by reacting the compound of formula (I) or a compound of formula (IIIm) whereever applicable with 1 to 4 equivalents of a base such as sodium hydroxide, sodium methoxide, sodium hydride, potassium t-butoxide, calcium hydroxide, magnesium hydroxide and the like, in solvents like ether, THF, methanol, t-butanol, dioxane, isopropanol, ethanol etc. Mixture of solvents may be used. Organic bases like lysine, arginine, diethanolamine, choline, tromethamine, guanidine and their derivatives etc. may also be used. Alternatively, acid addition salts wherever applicable are prepared by treatment with acids such as hydrochloric acid, hydrobromic acid, nitric acid, sulfuric acid, phosphoric acid, p-toluenesulphonic acid, methanesulfonic acid, acetic acid, citric acid, maleic acid salicylic acid, hydroxynaphthoic acid, ascorbic acid, palmitic acid, succinic acid, benzoic acid, benzenesulfonic acid, tartaric acid and the like in solvents like ethyl acetate, ether, alcohols, acetone, THF, dioxane etc. Mixture of solvents may also be used.

The stereoisomers of the compounds forming part of this invention may be prepared by using reactants in their single enantiomeric form in the process wherever possible or by conducting the reaction in the presence of reagents or catalysts in their single enantiomer form or by resolving the mixture of stereoisomers by conventional methods. Some of the preferred methods include use of microbial resolution, resolving the diastereomeric salts formed with chiral acids such as mandelic acid, camphorsulfonic acid, tartaric acid, lactic acid, and the like wherever applicable or chiral bases such as brucine, cinchona alkaloids and their derivatives and the like. Commonly used methods are compiled by Jaques et al in "Enantiomers, Racemates and Resolution" (Wiley Interscience, 1981). More specifically the compound of formula (I) where $YR^7$ represents OH or a compound of formula (IIIm) may be converted to a 1:1 mixture of diastereomeric amides by treating with chiral amines, aminoacids, aminoalcohols derived from aminoacids; conventional reaction conditions may be employed to convert acid into an amide; the diastereomers may be separated either by fractional crystallization or chromatography and the stereoisomers of compound of formula (I) or a compound of formula (IIIm) may be prepared by hydrolyzing the pure diastereomeric amide.

Various polymorphs of compound of general formula (I) and compounds of formula (IIIm) forming part of this invention may be prepared by crystallization of compound of formula (I) or compound of formula (IIIm) under different conditions. For example, using different solvents commonly used or their mixtures for crystallization; crystallizations at different temperatures; various modes of cooling, ranging from very fast to very slow cooling during crystallizations. Polymorphs may also be obtained by heating or melting the compound followed by gradual or fast cooling. The presence of poly-morphs may be determined by solid probe NMR spectroscopy, IR spectroscopy, differential scanning calorimetry, powder X-ray diffraction or such other techniques.

The compounds of general formula (I) or the compounds of formula (IIIm) are useful for the treatment and/or

prophylaxis diseases such as hypertension, coronary heart disease, atherosclerosis, stroke, peripheral vascular diseases and related disorders. These compounds are useful for the treatment of familial hypercholesterolemia, hypertriglyceridemia, lowering of atherogenic lipoproteins, VLDL and LDL. The compounds of the present invention can be used for the treatment of certain renal diseases including glomerulonephritis, glomerulosclerosis, nephrotic syndrome, hypertensive nephrosclerosis, retinopathy, and nephropathy. The compounds of general formula (I) or the compositions of formula (IIIm) are also useful for the treatment/prophylaxis of insulin resistance (type II diabetes), leptin resistance, impaired glucose tolerance, dyslipidemia, disorders related to syndrome X such as hypertension, obesity, insulin resistance, coronary heart disease, and other cardiovascular disorders. These compounds may also be useful as aldose reductase inhibitors, for improving cognitive functions in dementia, treating diabetic complications, disorders related to endothelial cell activation, psoriasis, polycystic ovarian syndrome (PCOS), inflammatory bowel diseases, osteoporosis, myotonic dystrophy, pancreatitis, arteriosclerosis, xanthoma and for the treatment of cancer. The compounds of the present inventions are useful in the treatment and/or prophylaxis of the above said diseases in combination/concomittant with one or more HMG CoA reductase inhibitors, hypolipidemic/hypolipoproteinemic agents such as fibric acid derivatives, nicotinic acid, cholestyramine, colestipol, or probucol. The compounds of the present invention in combination with HMG CoA reductase inhibitors, and/or hypolipidemic/hypolipoproteinemic agents can be administered together or within such a period to act synergistically. The HMG CoA reductase inhibitors may be selected from those used for the treatment or prevention of hyperlipidemia such as lovastatin, provastatin, simvastatin, fluvastatin, atorvastatin, cerivastatin and their analogs thereof. Suitable fibric acid derivative may be gemfibrozil, clofibrate, fenofibrate, ciprofibrate, benzafibrate and their analogs thereof

The present invention also provides a pharmaceutical composition, containing a compound of the general formula (I) or compounds of formula (IIIm), as defined above, their tautomeric forms, their stereoisomers, their polymorphs, their pharmaceutically acceptable salts, their pharmaceutically acceptable solvates in combination with the usual pharmaceutically employed carriers, diluents, excipients or solvates.

The pharmaceutical composition may be in the forms normally employed, such as tablets, capsules, powders, syrups, solutions, suspensions and may contain flavourants, sweeteners etc. in suitable solid or liquid carriers or diluents, or in suitable sterile media to form injectable solutions or suspensions. Such compositions typically contain from 1 to 20%, preferably 1 to 10% by weight of active compound, the remainder of the composition being pharmaceutically acceptable carriers, excipients, diluents, solvents and the like.

The compounds of formula (I) or the compounds of formula (IIIm) as defined above are clinically administered to mammals, including man, via either oral or parenteral routes. Administration by the oral route is preferred, being more convenient and avoiding the possible pain and irritation of injection. However, in circumstances where the patient cannot swallow the medication, or absorption following oral administration is impaired, as by disease or other abnormality, it is essential that the drug be administered parenterally. By either route, the dosage is in the range of about 0.01 to about 100 mg/kg body weight of the subject per day or preferably about 0.01 to about 30 mg/kg body weight per day administered singly or as a divided dose. However, the optimum dosage for the individual subject being treated will be determined by the person responsible for treatment, generally smaller doses being administered initially and thereafter increments made to determine the most suitable dosage.

Suitable pharmaceutically acceptable carriers include solid fillers or diluents and sterile aqueous or organic solutions. The active compound will be present in such pharmaceutical compositions in the amounts sufficient to provide the desired dosage in the range as described above. Thus, for oral administration, the compounds can be combined with a suitable solid or liquid carrier or diluent to form capsules, tablets, powders, syrups, solutions, suspensions and the like. The pharmaceutical compositions, may, if desired, contain additional components such as flavourants, sweeteners, excipients and the like. For parenteral administration, the compounds can be combined with sterile aqueous or organic media to form injectable solutions or suspensions. For example, solutions in sesame or peanut oil, aqueous propylene glycol and the like can be used, as well as aqueous solutions of water-soluble pharmaceutically-acceptable acid addition salts or salts with base of the compounds. The injectable solutions prepared in this manner can then be administered intravenously, intraperitoneally, subcutaneously, or intramuscularly, with intramuscular administration being preferred in humans.

The invention is explained in detail in the examples given below which are provided by way of illustration only and therefore should not be construed to limit the scope of the invention.

**Preparation 1**

**Ethyl 2-ethoxy-3-[4-(2-azidoethoxy)phenyl]-2-propenoate**

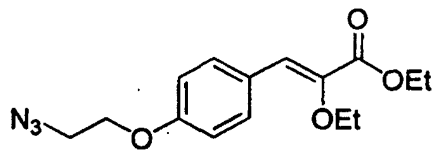

**Method A**

To a stirred suspension of sodium hydride (390 mg, 9.83 mmol, 60%) in dry THF (20 mL) was added a solution of ethyl (diethylphosphono)ethoxyacetate (2.28 g, 8.52 mmol) in THF (10.0 mL) at 0-5°C dropwise and stirred for 30 min at 5-25°C. To the reaction mixture was added a solution of 4-(2-azidoethoxy)benzaldehyde (2.0 g, 6.56 mmol) in THF (5.0 mL) at 25-30°C and stirred further for 30 min. After completion of the reaction (tlc monitored), THF was removed and the resultant residue was diluted with water (50 mL) and extracted with ethyl acetate (3 X 25 mL). The combined organic extracts were washed with brine, dried over anhydrous $Na_2SO_4$ and concentrated to yield the title compound (3.0 g, 94%) as a mixture of E/Z isomers.

**Method B**

To a stirred solution of ethyl 2-ethoxy-3-[4-(2-bromoethoxy)phenyl]-2-propenoate (7.0 g, 20 mmol) prepared as disclosed in U.S. Patent Application Serial No. 09/012,585, sodium azide (2.0 g, 31 mmol) in dry DMF (40 mL) was added at *ca* 25°C and stirred for 16 h. Water was added and extracted with ethyl acetate (3 + 50 mL). The combined organic extracts were washed with brine, dried over anhydrous $Na_2SO_4$ and concentrated to yield the title compound as a colorless liquid (5.6 g, 92%).

$^1H$ NMR ($CDCl_3$): δ 7.76 and 7.15 (d, J = 8.70 Hz, 2H), 7.02 - 6.75 (m, 2.6H), 6.08 (s, 0.4H), 4.35 - 3.80 (m, 6H), 3.72 - 3.61 (m, 2H), 1.48 - 1.20 (m, 6H).

**Preparation 2**

**Ethyl 2-phenoxy-3-(4-benzyloxyphenyl)-2-propenoate**

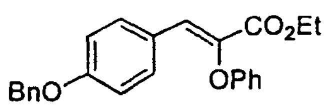

The title compound (1.66 g, 47%) as a mixture of *E*/*Z* isomers was obtained from 4-benzyloxybenzaldehyde (2.0 g, 9.4 mmol), ethyl (diethylphosphono) phenoxyacetate (3.0 g, 9.4 mmol) (*J. Org. Chem*. **1983**, *48*, 3407) and NaH (273 mg, 11.39 mmol, 95%) by a similar procedure to that described in preparation 1 (method A).

$^1H$ NMR ($CDCl_3$): δ 7.73 (d, J = 8.40 Hz, 1H), 7.60 - 7.25 (m, 9H), 7.18 - 6.92 (m, 5H), 5.1.2 and 5.08 (s, 2H), 4.22 and 4.15 (q, J = 7.05 Hz, 2H), 1.24 and 1.10 (t, J = 7.05 Hz, 3H).

**Preparation 3**

**(±)-Ethyl 2-ethoxy-3-[4-(2-azidoethoxy)phonyl]propanoate**

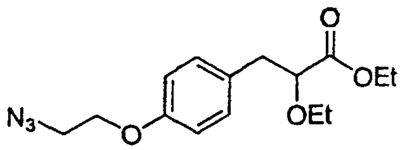

The title compound was obtained (16.8 g, 86%) from (±)-ethyl 2-ethoxy-3-[4-(2-bromoethoxy)phenylpropanoate (22.0 g, 63 mmol) prepared as disclosed in U.S. Patent 6,054,453 and sodium azide (6.2 g, 95 mmol) by a similar procedure to that described in preparation 1 (method B).

$^1H$ NMR ($CDCl_3$): δ 7.17 (d, J = 8.63 Hz, 2H), 6.83 (d, J = 8.62 Hz, 2H), 4.25 - 4.05 (m, 4H), 3.96 (t, J = 6.57 Hz, 1H), 3.64 - 3.50 (m, 3H), 3.42 - 3.23 (m, 1H), 2.95 (d, J = 6.64 Hz, 2H), 1.30 -1.09 (m, 6 H).

**Preparation 4**

**(±)-Ethyl 2-ethoxy-3-[4-(2-aminoethoxy)phenyl]propanoate**

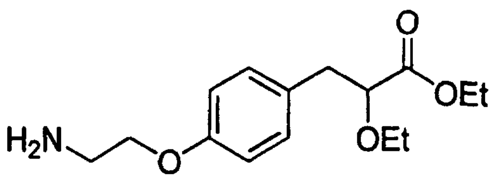

**Method A**

A solution of (±)-ethyl 2-ethoxy-3-[4-(2-azidoethoxy)phenyl]propanoate (1.0 g, 3.25 mmol) obtained in preparation 3, in 1,4-dioxane (20 mL) was reduced with hydrogen in the presence of 10% palladium charcoal (100 mg) at 50 psi for 10 h. The reaction mixture was filtered through a bed of celite and the celite bed was washed with dioxane. The filtrate was evaporated to dryness under reduced pressure to yield the title compound (600 mg, 65%).

**Method B**

The title compound (450 mg, 49%) was obtained from ethyl 2-ethoxy-3-[4-(2-azidoethoxy)phenyl]2-propenoate (1.0 g, 3.27 mmol) obtained in preparation 1 and 10% Pd/C (500 mg) by a similar procedure to that described in method A above.

$^1H$ NMR ($CDCl_3$): δ 7.15 (d, J = 7.82 Hz, 2H), 6.83 (d, J = 7.82 Hz, 2H), 4.10 (q, J = 7.02 Hz, 2H), 3.97 (t, J = 5.60 Hz, 2H), 3.70 - 3.50 (m, 1H), 3.50 - 3.25 (m, 1H), 3.40 - 2.95 (m, 2H), 3.07 (t, J = 4.77 Hz, 1H), 2.95 (d, J = 6.64 Hz, 2H), 0.95 (bs, 2H, $D_2O$ exchangeable), 1.23 (t, J = 6.64 Hz, 3H), 1.17 (t, J = 7.05 Hz, 3H).

**Preparation 5**

**(±)-Ethyl 2-phenoxy-3-(4-hydroxyphenyl)propanoate**

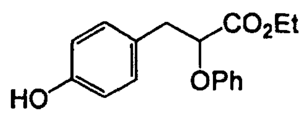

The title compound (1.03 g, 82%) was obtained from ethyl 2-phenoxy-3-(4-benzyloxyphenyl) -2-propenoate (1.65 g, 4.4 mmol) obtained in preparation 2 and 5% Pd-C (3.30 g) by a similar procedure to that described in preparation 4 (Method A).

$^1H$ NMR ($CDCl_3$): δ 7.38 - 7.08 (m, 3H), 7.08 - 6.80 (m, 4H), 6.73 (d, J = 8.3 Hz, 2H), 4.73 (t, J = 6.43 Hz, 1H), 4.16 (q, J = 7.15 Hz, 2H), 3.16 (d, J = 6.40 Hz, 2H), 1.18 (t, *J* = 7.15 Hz, 3H).

**Preparation 6**

**(±)-Ethyl 2-ethoxy-3-[4-[2-N-(2-aminobenzoyl)aminoethoxy]phenyl]propanoate**

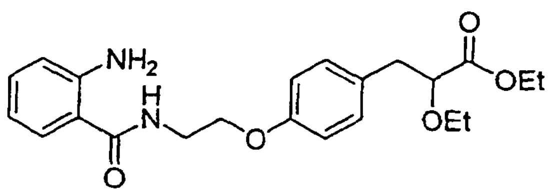

To a stirred solution of isatoic anhydride (1.57 g, 9.6 mmol) in 1,4-dioxane (30 mL) was added a solution of (±)-ethyl 2-ethoxy-3-[4-(2-aminoethoxy)phenyl] propanoate (3.0 g, 10.7 mmol) obtained in preparation 4 in 1,4-dioxane (10 mL) and stirred at room temperature for 2 h. Dioxane was removed under reduced pressure to yield the title compound as a brown coloured gummy liquid (3.8 g, 99%).

$^1H$ NMR ($CDCl_3$): δ 7.34 (d, J = 7.91 Hz, 1H), 7.28 - 7.12 (m, 1H), 7.17 (d, J = 8.40 Hz, 2H), 6.83 (d, J = 8.40 Hz, 2H), 6.70 - 6.50 (m, 2H), 4.21 - 4.02 (m, 4H), 3.97 (t, J = 6.43 Hz, 1H), 3.81 (q, J = 5.07 Hz, 2H), 3.65 - 3.48 (m, 1H), 3.48 - 3.22 (m, 1H), 2.95 (d, J = 6.64 Hz, 2H), 1.23 (t, J = 7.06 Hz, 3H), 1.16 (t, J = 7.05 Hz, 3H).

**Example 1**

**(±)-Ethyl 2-ethoxy-3-[4-[[3-methyl-4-oxo-3,4-dihydro-2-quinazolinyl] methoxy] phenyl]propanoate**

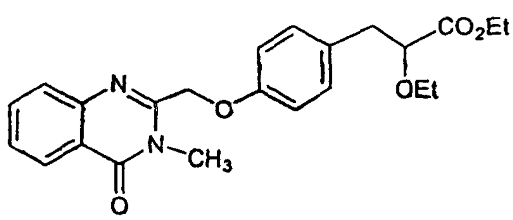

To a stirred solution of (±)-ethyl 2-ethoxy-3-(4-hydroxyphenyl)propanoate (7.6 g, 31.9 mmol) (disclosed in U. S. Patent 6,054,453) potassium carbonate (8.81 g, 63.8 mmol) in dry DMF (60 mL) was added and stirred for 0.5 h at 30°C. To the reaction mixture was added 2-chloromethyl-3-methyl-4-oxo-3,4-dihydroquinazoline (10.0 g, 47.8 mmol)

in one portion and stirred for 15 h at the same temperature. Water (100 mL) was added and extracted with ethyl acetate (3 + 100 mL). The combined ethylacetate extracts were washed with water, saturated sodium carbonate solution, brine, dried over anhydrous $Na_2SO_4$ and concentrated to yield the title compound (10.0 g, 70%). mp: 71-73°C.

$^1H$ NMR ($CDCl_3$): δ 8.31 (d, J = 7.89 Hz, 1H), 7.84 - 7.65 (m, 2H), 7.52 (t, J = 7.90 Hz, 1H), 7.20 (d, J = 8.63 Hz, 2H), 6.98 (d, J = 8.63 Hz, 2H), 5.17 (s, 2H), 4.17 (q, J = 7.06 Hz, 2H), 3.97 (t, J = 6.41 Hz, 1H), 3.75 (s, 3H), 3.70 - 3.48 (m, 1H), 3.48 - 3.25 (m, 1H), 3.02 - 2.82 (m, 2H), 1.36 (m, 6H).

## Example 2

**(±)-2-Ethoxy-3-[4-[[3-methyl-4-oxo-3,4-dihydro-2-quinazolinyl]methoxy]phenyl] propanoic acid**

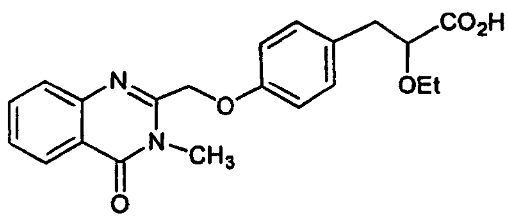

To a stirred solution of (±)-ethyl 2-ethoxy-3-[4-[[3-methyl-4-oxo-3,4-dihydro-2-quinazolinyl]methoxy]phenyl] propanoate (10.0 g, 24.3 mmol) obtained in Example 1, in methanol (70 mL) was added a solution of sodium carbonate (12.93 g, 0.122 mmol) in water (70 mL) and stirred for 8 h at 25-30 °C. Methanol was removed under reduced pressure and the aqueous layer was washed with ethyl acetate (2 X 75 mL). The aqueous layer was acidified to pH 2.0 with 2N HCl. The white solid precipitated was filtered and dried to yield the title compound (8.0 g, 85.8%). mp: 80°C.

$^1H$ NMR (DMSO-$d_6$): δ 8.29 (d, J = 7.89 Hz, 1H), 7.85 - 7.65 (m, 2H), 7.51 (t, J = 6.32 Hz, 1H), 7.19 (d, J = 8.63 Hz, 2H), 6.97 (d, J = 8.63 Hz, 2H), 5.16 (s, 2H), 4.04 (dd, J = 7.10 and 4.57 Hz, 1H), 3.34 (s, 3H), 3.72 - 3.50 (m, 1H), 3.50 - 3.35 (m, 1H), 3.15 - 2.85 (m, 2H), 1.16 (t, J = 7.94 Hz, 3H).

## Example 3

**(±)-Sodium 2-ethoxy-3-[4-[[3-methyl-4-oxo-3,4-dihydro-2-quinazolinyl]methoxy] phenyl]propanoate**

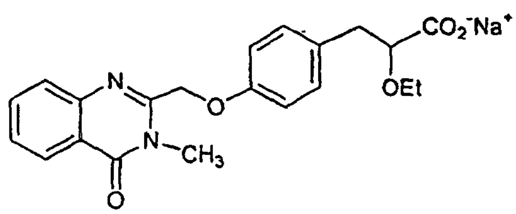

To a stirred suspension of (±)-2-ethoxy-3-[4-[[3-methyl-4-oxo-3,4-dihydro-2-quinazolinyl]methoxy]phenyl]propanoic acid (4.0 g, 10.5 mmol), obtained in Example 2 in methanol (50 mL) was added a solution of sodium methoxide (2.27 g, 42 mmol) in methanol (10 mL) dropwise at 30°C. The reaction mixture was stirred for further 1 h. Diethyl ether (50 mL) was added and the white solid precipitated was filtered and dried to afford the title compound (3.2 g, 76%), mp: 210°C.

$^1H$ NMR ($CDCl_3$): δ 8.17 (d, J = 7.06 Hz, 1H), 7.85 (t, J = 7.06 Hz, 1H), 7.69 (d, J = 7.88 Hz, 1H), 7.58 (t, J = 7.88 Hz, 1H), 7.17 (d, J = 8.62 Hz, 2H), 6.98 (d, J = 8.62 Hz, 2H), 5.24 (s, 2H), 3.68 (s, 3H), 3.60 - 3.48 (m, 1H), 3.25 - 3.00 (m, 1H), 2.85 (dd, J = 14.11 and 3.74 Hz, 1H), 2.62 (dd, J = 14.11 and 8.72 Hz, 1H), 2.60 - 2.48 (m, 1H), 0.97 (t, J = 7.06 Hz, 3H).

**Comparative Example 4**

**[2R, N(1S)] 2-ethoxy-3-[4-[[3-Methyl-4-oxo-3,4-dihydro-2-quinazolinyl]methoxy] phenyl]-N-(2-hydroxy-1-phenylethyl)propanamide (4a)**

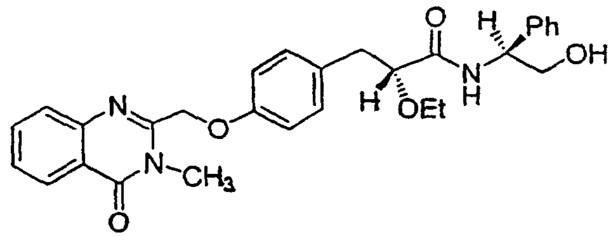

**[2S, N(1S)] 2-ethoxy-3-[4-[[3-Methyl-4-oxo-3,4-dihydro-2-quinazolinyl] methoxy] phenyl]-N-(2-hydroxy-1-phenylethyl)propanamide (4b)**

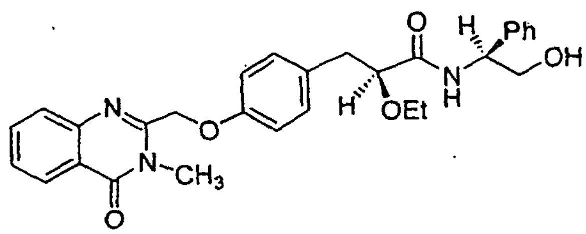

To a stirred solution of (±)-2-ethoxy-3-[4-[[3-methyl-4-oxo-3,4-dihydro-2-quinazolinyl]methoxy]phenyl]propanoic acid (8.0 g, 20.9 mmol), obtained in Example 2, in dry dichloromethane (150 mL) was added triethylamine (7.28 mL, 5.29 g, 52.0 mmol) at 0°C, followed by addition of pivaloyl chloride (3.12 mL, 2.77 g, 23.0 mmol) and stirred for 30 min. at the same temperature. To this reaction mixture was added a solution of (S)-2-phenyl glycinol (2.87 g, 20.9 mmol) in dichloromethane (5 mL) containing triethylamine (5.8 mL, 41.8 mmol). After stirring for 1 h dichloromethane (600 mL) was added and the mixture was washed with water, brine, dried over anhydrous $Na_2SO_4$ and evaporated. The residue was chromatographed on silica gel using a gradient of 10-50% ethyl acetate in pet. ether as eluent to afford firstly a diastereomer tentatively assigned as [2R, N(1S)] 2-ethoxy-3-[4-[[3-methyl-4-oxo-3,4-dihydro-2-quinazolinyl]methoxy]phenyl]-N-[2-hydroxy-1-phenylethyl]propanamide **(4a)** (4.5 g) followed by [2S, N(1S)] 2-ethoxy-3-[4-[[3-methyl-4-oxo-3,4-dihydro-2-quinazolinyl]methoxy]phenyl]-N-[2-hydroxy- I -phenylethyl]propanamide (**4b**).

Spectral data for (**4a**):

$[\alpha]_D^{25}$ = + 41.60 (c = 0.5, MeOH), mp: 136-138°C.

$^1$H NMR ($CDCl_3$): δ 8.29 (d, J = 7.50 Hz, 1H), 7.82 - 7.62 (m, 2H), 7.51 (t, J = 7.50 Hz, 1H), 7.40 - 7.10 (m, 7H), 7.0 (d, J = 8.62 Hz, 2H), 5.18 (s, 2H), 5.00 - 4.88 (m, 1H), 3.98 (dd, J = 6.23 and 3.78 Hz, 1H), 3.75 (s, 3H), 3.70 - 3.55 (m, 2H), 3.50 (q, J = 7.01 Hz, 2H), 3.13 (dd, J = 14.12 and 3.78 Hz, 1H), 2.96 (dd, J = 14.12 and 6.23 Hz, 1H), 1.13 (t, J = 7.01 H, 3H).

Spectral data for (**4b**):

$[\alpha]_D^{25}$ = - 9.9 (c = 1.0, MeOH) mp: 126-128°C.

$^1$H NMR ($CDCl_3$): δ 8.30 (d, J = 8.89 Hz, 1H), 7.68 - 7.81 (m, 2H), 7.51 (t, J = 6.41 Hz,1H), 7.03 - 7.35 (m, 7H), 6.90 (d, J = 8.39 Hz, 2H), 5.13 (s, 2H), 4.91 - 5.01 (m, 1H), 3.99 (dd, J = 3.88 and 6.78 Hz, 1H), 3.85 (t, J = 5.35 Hz, 2H), 3.74 (s, 3H), 3.44 - 3.61 (m, 2H), 3.08 (dd, J = 3.88 and 14.12 Hz, 1 H), 2.87 (dd, J = 6.78 and 14.12 Hz, 1H), 1.17 (t, J = 7.01 Hz, 3H).

**Example 5**

**(+)-2-Ethoxy-3-[4-[[3-methyl-4-oxo-3,4-dihydro-2-quinazolinyl]methoxy]phenyl] propanoic acid**

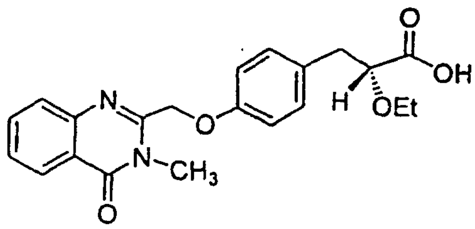

A solution of [2R, N(1S)] 2-ethoxy-3-[4-[(3-methyl-4-oxo-3,4-dihydro-2-quinazolinyl] methoxy] phenyl]-N-[2-hydroxy-1-phenylethyl]propanamide (8.25 g, 16.50 mmol) obtained in Comparative Example 4a in a mixture of 1M sulphuric acid (212 mL) and dioxane/water (1 : 1, 1.7 L) was heated at 100°C for 16 h. The reaction mixture was cooled to *ca* 25°C and dioxane was removed under reduced pressure. The remaining solution was cooled in an ice bath and the white solid precipitated was filtered and dried to afford the title compound (3.6 g, 58%).

mp: 170°C.

$[\alpha]_D^{25}$ = 21.2 (c = 0.5, MeOH).

$^1$H NMR ($CDCl_3$): δ 8.29 (d, J = 7.88 Hz, 1H), 7.81 - 7.68 (m, 2H), 7.51 (t, J = 6.27 Hz, 1H), 7.19 (d, J = 8.62 Hz, 2H), 6.94 (d, J = 8.62 Hz, 2H), 5.16 (s, 2H), 4.04 (dd, J = 4.52 and 7.33 Hz, 1H), 3.70 (s, 3H), 3. 70 - 3.51 (m, 1H), 3.34 - 3.51 (m, 1H), 2.90 - 3.14 (m, 2H), 1.16 (t, J = 6.92 Hz, 3H).

**Example 6**

**(-)-2-Ethoxy-3-[4-[[3-methyl-4-oxo-3,4-dihydro-2-quinazolinyl]methoxy] phenyl] propanoic acid**

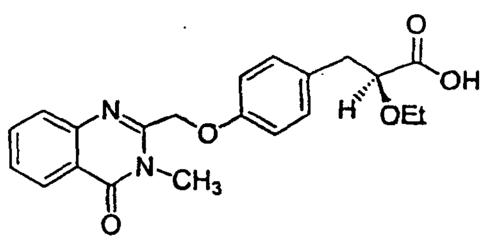

The title compound (3.0 g, 87%) was obtained from [2S, N(1S)] 2-ethoxy-3-[4-[[3-methyl-4-oxo-3,4-dihydro-2-quinazolinyl]methoxy]phenyl]-N-[2-hydroxy-1-phenylethyl]propanamide (4.5 g, 8.9 mmol) obtained in Comparative example 4b, by a similar procedure to that described in Example 5.

mp: 133-135°C.

$[\alpha]_D^{25}$ = - 20.84 (c = 1.0, MeOH).

$^1$H NMR ($CDCl_3$+DMSO-$d_6$): δ 8.22 (d, J = 7.56 Hz, 1H), 7.88 - 7.68 (m, 2H), 7.54 (t, J = 7.54 Hz, 1H), 7.20 (d, J = 8.62 Hz, 2H), 7.00 (d, J = 8.62 Hz, 2H), 5.24 (s, 2H), 3.93 (dd, J = 7.56 and 4.89 Hz, 1H), 3.71 (s, 3H), 3.70 - 3.50 (m, 1H), 3.42 - 3.22 (m, 1H), 3.05 - 2.78 (m, 2H), 1.12 (t, J = 7.06 Hz, 3H).

**Example 7**

**(-)-Sodium 2-ethoxy-3-[4-[[3-methyl-4-oxo-3,4-dihydro-2-quinazolinyl]methoxy] phenyl]propanoate**

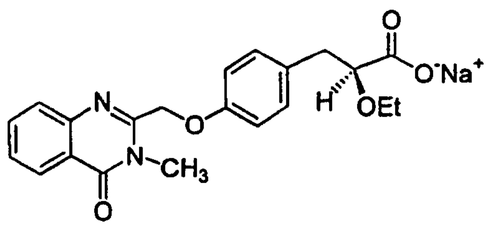

The title compound (1.9 g, 85.5%) was obtained from (-)-2-ethoxy-3-[4-[[3-methyl-4-oxo-3,4-dihydro-2-quinazolinyl]methoxy]phenyl]propanoic acid (2.1 g, 5.49 mmol) obtained in example 6 and 10% sodium methoxide solution (1.39 mL) by a similar procedure to that described in Example 3.

mp: 190°C.

$[\alpha]_D^{25}$ = -29.2 ( c = 0.75 , MeOH).

$^1$H NMR (DMSO-$d_6$): δ 8.15 (d, J = 7.89 Hz, 1H), 7.83 (t, J = 7.47 Hz, 1H), 7.68 (d, J = 7.89 Hz, 1H), 7.56 (t, J = 7.31 Hz, 1H), 7.15 (d, J = 8.53 Hz, 2H), 6.96 (d, J = 8.63 Hz, 2H), 5.22 (s, 2H), 3.61 (s, 3H), 3.42 - 3.58 (m, 2H), 3.01 - 3.19 (m, 1H), 2.84 (dd, J = 3.64 and 14.12 Hz, 1H), 2.61 (dd, J = 9.04 and 14.12 Hz, 1H), 0.96 (t, J = 7.01 Hz, 3H).

**Example 8**

**(±)-(Morpholine-4-yl) 2-ethoxy-3-[4-[[3-methyl-4-oxo-3,4-dihydro-2-quinazolinyl] methoxy]phenyl] propanamide**

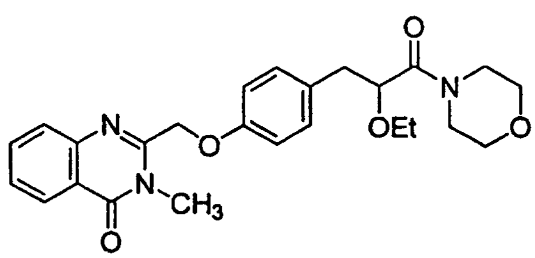

To a stirred solution of (±)-2-ethoxy-3-[4-[[3-methyl-4-oxo-3,4-dihydro-2-quinazolinyl] methoxy]phenyl]propanoic acid (0.2 g, 0.52 mmol) obtained in Example 2 in dichloromethane (2 mL) was added triethylamine (182 μL, 0.13 g, 1.3 mmol) dropwise at 0°C. After stirring for 5 min was added pivaloyl chloride (78 μL, 69 mg, 0.57 mmol) and stirring continued for further 30 min at 0°C. To this reaction mixture was added a solution of morpholine (45 mL, 46 mg, 0.52 mmol) in dichloromethane containing triethylamine (145 μL, 1.0 mmol) at 25°C and stirred for 1 h at 25-30°C. To the reaction mixture chloroform (10 mL) was added and washed with water (2 + 10 mL), brine, dried over anhydrous $Na_2SO_4$ and concentrated. The crude compound was purified by column chromatography using ethyl acetate and pet. ether (1:1) as eluent to afford the title compound (184 mg, 78%).

mp: 115°C.

$^1$H NMR ($CDCl_3$): δ 8.26 (d, J = 7.57 Hz, 1H), 7.80 - 7.65 (m, 2H), 7.51 (t, J = 4.05 Hz, 1H), 7.15 (d, J = 8.58 Hz, 2H), 6.95 (d, J = 8.58 Hz, 2H), 5.14 (s, 2H), 4.24 (t, J = 6.75 Hz, 1H), 3.71 (s, 3H), 3.61 - 3.31 (m, 10 H), 2.95 (d, J = 6.75 Hz, 2H), 1.13 (t, J = 7.01 Hz, 3H).

**Example 9**

**(±)-2-Ethoxy-3-[4-[[3-methyl-4-oxo-3,4-dihydro-2-quinazolinyl]methoxy]phenyl]-N-(2-fluorophenyl) propanamide**

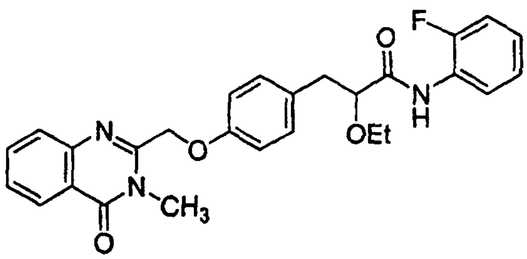

The title compound (110 mg, 44%) was obtained from (±)-2-ethoxy-3-[4-[[3-methyl-4-oxo-3,4-dihydro-2-quinazolinyl]methoxy]phenyl]propanoic acid (200 mg, 0.52 mmol) obtained in Example 2 and 2-fluoroaniline (50 μL, 58 mg, 0.52 mmol) by a similar procedure to that described in Example 8.

mp: 138-140°C.

$^1H$ NMR ($CDCl_3$): δ 8.33 (t, J = 7.42 Hz, 2H), 7.83 - 7.69 (m, 2H), 7.52 (t, J = 6.43 Hz, 1H), 7.29 - 6.92 (m, 7H), 5.16 (s, 2H), 4.02 (dd, J = 7.89 and 3.4 Hz, 1H), 3.74 (s, 3H), 3.65 - 3.40 (m, 2H), 3.18 (dd, J = 14.11 and 3.41 Hz, 1H), 2.94 (dd, J = 14.11 and 7.89 Hz, 1H), 1.20 (t, J = 7.01 Hz, 3H).

**Example 10**

**(±)-Ethyl 2-methoxy-3-[4-[[3-methyl-4-oxo-3,4-dihydro-2-quinazolinyl] methoxy] phenyl]propanoate**

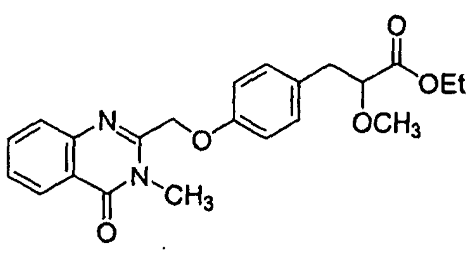

To a stirred suspension of sodium hydride (270 mg, 10.46 mmol, 95%) in dry DMF (2 mL) was added a solution of (±)-ethyl 2-hydroxy-3-[4-[[3-methyl-4-oxo-3,4-dihydro-2-quinazolinyl]methoxy] phenyl]propanoate (2.0 g, 5.23 mmol) at 0°C and stirred for 30 min. To the reaction mixture was added methyl iodide (1.62 mL, 26.15 mmol) at the same temperature and stirring continued for further 1h. After completion of the reaction, diluted with ethyl acetate (150 mL), washed with brine (3 x 100 mL), dried over anhydrous $Na_2SO_4$ and concentrated. The crude compound was purified by column chromatography using ethyl acetate and pet. Ether (1:9) as eluent to afford the title compound as a liquid (480 mg, 23%).

$^1H$ NMR ($CDCl_3$): δ 8.28 (d, J = 8.89 Hz, 1H), 7.70 - 7.68 (m, 2H), 7.53 (t, J = 4.06 Hz, 1H), 7.19 (d, J = 8.40 Hz, 2H), 6.97 (d, J = 8.63 Hz, 2H), 5.15 (s, 2H), 4.18 (q, J = 7.10 Hz, 2H), 3.98 (dd, J = 4.56 and 7.06 Hz, 1H), 3.73 (s, 3H), 3.39 (s, 3H), 3.12 - 2.99 (m, 2H), 1.25 (t, J = 7.10 Hz, 3H).

## Example 11

**(±)-2-Methoxy-3-[4-[[3-methyl-4-oxo-3,4-dihydro-2-quinazolinyl]methoxy]phenyl] propanoic acid**

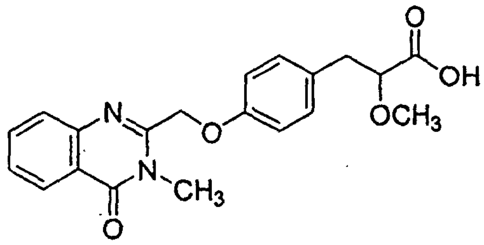

The title compound (355 mg, 80%) was obtained from (±)-ethyl 2-methoxy-3-[4-[[3-methyl-4-oxo-3,4-dihydro-2-quinazolinyl]methoxy]phenyl]propanoate (480 mg, 1.21 mmol) obtained in example 10 and sodium carbonate (640 mg. 6.06 mmol) by a similar procedure to that described in Example 2.

mp: 99-101°C.

$^1$H NMR ($CDCl_3$): δ 8.29 (d, J = 7.89 Hz, 2H), 7.82 - 7.68 (m, 2H), 7.55 (t, J = 7.89 $H_2$, 1H), 7.19 (d, J = 8.62 Hz, 2H), 6.97 (d, J = 8.62 Hz, 2H), 5.15 (s, 2H), 3.98 (dd, J = 7.06 and 4.56 Hz, 1H), 3.74 (s, 3H), 3.39 (s, 3H), 3.18 - 2.82 (m, 2H).

## Example 12

**(±)-Ethyl 2-propoxy-3-[4-[[3-methyl-4-oxo-3,4-dihydro-2-quinazolinyl]methoxy] phenyl]propanoate**

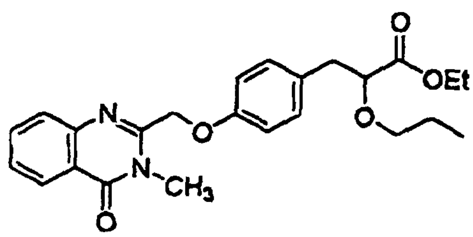

The title compound (1.23 g, 55%) was obtained as a liquid from (±)-ethyl 2-hydroxy-3-[4-[[3-methyl-4-oxo-3,4-dihydro-2-quinazolinyl] methoxy] phenyl] propanoate ($^1$H NMR ($CDCl_3$): δ 8.30 (d, J = 7.47 Hz,1H), 7.77 - 7.72 (m, 2H), 7.53 (t, J = 3.51 Hz, 1H), 7.18 (d, J = 8.57 Hz, 2H), 6.98 (d, J = 8.57 Hz, 2H), 5.16 (s, 2H), 4.41 (m, 1H), 4.23 (q, J = 7.10 Hz, 2H), 3.73 (s, 3H), 2.93 - 3.05 (m, 2H), 1.27 (t, J = 7.10 Hz, 3H) mp: 112-118°C, 2.0 g, 5.23 mmol) which was obtained from (±)-ethyl 2-hydroxy-3-(4-hydroxyphenyl) propanoate (5.0 g, 23.8 mmol) (DE 26 625 163), 2-chloromethyl-3-methyl-4-oxo-3,4-dihydro-quinazoline (5.0 g, 23.8 mmol) and potassium carbonate (6.57 g, 47.6 mmol) as a base by a similar procedure to that described in Example 1; propylbromide (2.5 ml, 23.17 mmol) and sodium hydride (270 mg, 10.46 mmol) as a base by a similar procedure to that described in Example 10.

$^1$H NMR ($CDCl_3$): δ 8.29 (d, J = 8.12 Hz, 1H), 7.80 - 7.65 (m, 2H), 7.50 (t, J = 7.50 Hz, 1H), 7.18 (d, J = 8.40 Hz, 2H), 6.96 (d, J = 8.30 Hz, 2H), 5.15 (s, 2H), 4.16 (q, J = 7.35 Hz, 2H), 3.95 (t, J = 6.32 Hz, 1H), 3.74 (s, 3H), 3.53 - 3.49 (m, 1H), 3.22 - 3.18 (m, 1H), 2.96 (d, J = 6.32 Hz, 2H), 1.70 - 1.40 (m, 2H), 1.20 (t, J = 7.25 Hz, 3H), 0.82 (t, J = 7.35 Hz, 3H).

**Example 13**

**(±)-2-Propoxy-3-[4-[[3-methyl-4-oxo-3,4-dihydro-2-quinazolinyl]methoxy] phenyl]propanoic acid**

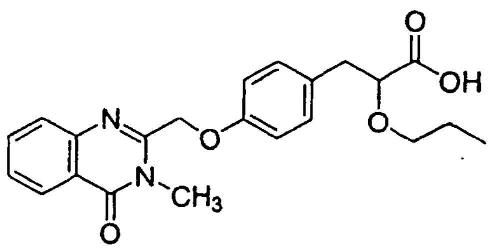

The title compound (310 mg, 81%) was obtained from (±)-ethyl 2-propoxy-3-[4-[[3-methyl-4-oxo-3,4-dihydro-2-quinazolinyl]methoxy]phenyl]propanoate (410 mg, 0.97 mmol) obtained in Example 12 and sodium carbonate (512 mg, 4.83 mmol) by a similar procedure to that described in Example 2.

mp: 167-168°C.

$^1$H NMR ($CDCl_3$): δ 8.29 (d, J = 7.88 Hz, 1H), 7.82 - 7.63 (m, 2H), 7.53 (t, J = 7.88 Hz, 1H), 7.19 (d, J = 8.62 Hz, 2H), 6.97 (d, J = 8.62 Hz, 2H), 5.16 (s, 2H), 4.05 (dd, J = 7.28 and 4.25 Hz, 1H), 3.74 (s, 3H), 3.60 - 3.40 (m, 1H), 3.40 - 3.25 (m, 1H), 3.20 - 2.90 (m, 2H), 1.56 (s, J = 7.05 Hz, 2H), 0.85 (t, J = 7.43 Hz, 3H).

**Comparative Example 14**

**[2S, N(1S)] 2-propoxy-3-[4-[[3-methyl-4-oxo-3,4-dihydro-2-quinazolinyl] methoxy]phenyl]-N-(2-hydroxy-1-phenylethyl)propanamide (14a)**

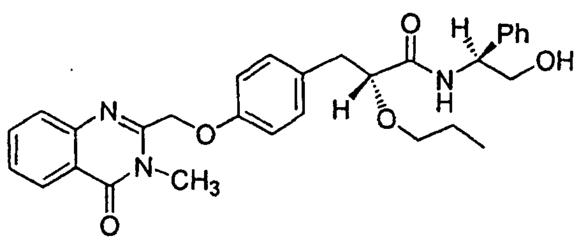

**[2R, N(1S)] 2-Propoxy-3-[4-[[3-methyl-4-oxo-3,4-dihydro-2-quinazolinyl] methoxy]phenyl]-N-(2-hydroxy-1-phenylethyl)propanamide (14b)**

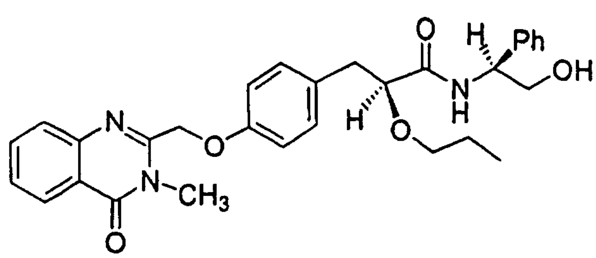

The title compounds [2S, N(1S)] propanamide **(14a)** and [2R, N(1S)] propanamide **(14b)** were obtained from

(±)-2-propoxy-3-[4-[[3-methyl-4-oxo-3,4-dihydro-2-quinazolinyl]methoxy] phenyl]propanoic acid (30 mg, 0.075 mmol) obtained in Example 13, triethylamine (47 µL, 0.33 mmol), pivaloyl chloride (11 µL, 0.083 mmol) and S-(+)-2-phenyl glycinol (10 mg, 0.075 mmol) by a similar procedure to that described in Comparative Example 4.

Spectral data for (**14a**):

$^1$H NMR ($CDCl_3$): δ 8.31 (d, J = 8.31 Hz, 1H), 7.69 - 7.82 (m, 2H), 7.50 - 7.60 (m, 1H), 7.12 - 7.39 (m, 7H), 6.94 (d, J = 6.23 Hz, 2H), 5.19 (s, 2H), 4.89 - 5.01 (m, 1H), 3.98 (dd, J = 3.73 and 5.90 Hz, 1H), 3.76 (s, 3H), 3.60 - 3.67 (m, 2H), 3.38 (q, J = 2.89 Hz, 2H), 3.13 (dd, J = 3.73 and 14.12 Hz, 1H), 2.95 (dd, J = 5.90 and 14.12 Hz, 1H), 1.54 (q, J = 7.16 Hz, 2H), 0.84 (t, J = 7.40 Hz, 3H).

Spectral Data for **(14b)**:

$^1$H NMR ($CDCl_3$): δ 8.32 (d, J = 7.89 Hz, 1H), 7.86 - 7.70 (m, 2H), 7.58 - 7.49 (m, 1H), 7.39 - 7.08 (m, 7H), 6.92 (d, J = 8.40 Hz, 2H), 5.15 (s, 2H), 5.08 - 4.91 (m, 1H), 4.00 (dd, J = 3.73 and 6.73 Hz, 1H), 3.87 (d, J = 4.89 Hz, 2H), 3.76 (s, 3H), 3.44 (q, J = 3.46 Hz, 2H), 3.10 (dd, J = 3.73 and 14.11 Hz, 1H), 2.90 (dd, J = 6.73 and 14.11 Hz, 1H), 1.58 (q, J = 6.95 Hz, 2H), 0.90 (t, J = 7.42 Hz, 3H).

## Example 15

**(±)-Ethyl 2-(n-butoxy)-3-[4-[[3-methyl-4-oxo-3,4-dihydro-2-quinazolinyl] methoxy]phenyl]propanoate**

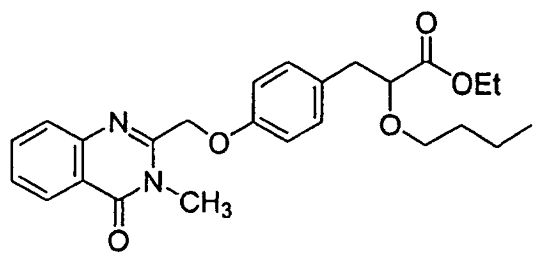

The title compound (270 mg, 47%) was obtained as a liquid from (±)-ethyl 2-hydroxy-3-[4-[[3-methyl-4-oxo-3,4-dihydro-2-quinazolinyl] methoxy] phenyl] propanoate ($^1$H NMR ($CDCl_3$): δ 8.30 (d, J = 7.47 Hz,1H), 7.77 - 7.72 (m, 2H), 7.53 (t, J = 3.51 Hz, 1H), 7.18 (d, J = 8.57 Hz, 2H), 6.98 (d, J = 8.57 Hz, 2H), 5.16 (s, 2H), 4.41 (m, 1H), 4.23 (q, J = 7.10 Hz, 2H), 3.73 (s, 3H), 2.93 - 3.05 (m, 2H), 1.27 (t, J = 7.10 Hz, 3H) mp:112-118°C, 500 mg, 1.31 mmol) which was obtained from (±)-ethyl 2-hydroxy-3-(4-hydroxyphenyl) propanoate (5.0 g, 23.8 mmol) (DE 26 625 163), 2-chloromethyl-3-methyl-4-oxo-3,4-dihydro-quinazoline (5.0 g, 23.8 mmol) and potassium carbonate (6.57 g, 47.6 mmol) as a base by a similar procedure to that described in Example 1; butyl bromide (0.7 mL, 0.64 mmol) and sodium hydride (50 mg, 1.96 mmol) as a base by a similar procedure to that described in Example 10.

$^1$H NMR ($CDCl_3$): δ 8.30 (d, J = 7.47 Hz, 1H), 7.81 - 7.65 (m, 2H), 7.58 - 7.43 (m, 1H), 7.18 (d, J = 8.62 Hz, 2H), 6.96 (d, J = 8.62 Hz, 2H), 5.16 (s, 2H), 4.15 (q, J = 7.15 Hz, 2H), 3.93 (t, J = 6.40 Hz, 1H), 3.74 (s, 3H), 3.60 - 3.45 (m, 1H), 3.30 - 3.15 (m, 1H), 2.95 (d, J = 6.40 Hz, 2H), 1.78 - 1.40 (m, 4H), 1.21 (t, J = 7.15 Hz, 3H), 0.83 (t, J = 7.35 Hz, 3H).

**Example 16**

**(±)-2-(n-Butoxy)-3-[4-[[3-methyl-4-oxo-3,4-dihydro-2-quinazolinyl]methoxy] phenyl]propanoic acid**

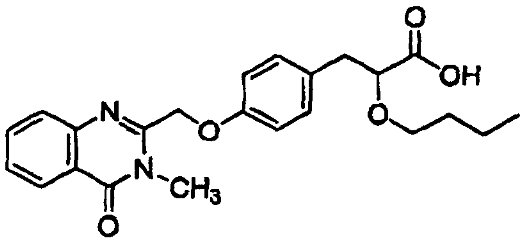

The title compound (320 mg, 80%) was obtained from (±)-ethyl 2-(n-butoxy)-3-[4-[[3-methyl-4-oxo-3,4-dihydro-2-quinazolinyl]methoxy]phenyl]propanoate (430 mg, 0.98 mmol), obtained in Example 15 and sodium carbonate (520 mg, 4.91 mmol) by a similar procedure to that described in Example 2.

mp: 145°C.

$^1$H NMR ($CDCl_3$): δ 8.30 (d, J = 7.85 Hz, 1H), 7.85 - 7.60 (m, 2H), 7.54 (t, J = 7.85 Hz, 1H), 7.20 (d, J = 8.62 Hz, 2H), 6.98 (d, J = 8.62 Hz, 2H), 5.17 (s, 2H), 4.05 (dd, J = 7.28 and 4.25 Hz, 1H), 3.74 (s, 3H), 3.65 - 3.48 (m, 1H), 3.28 - 3.32 (m, 1H), 3.20 - 2.86 (m, 2H), 1.65 - 1.40 (m, 2H), 1.40 - 1.20 (m, 2H), 0.87 (t, J = 7.15 Hz, 3H).

**Example 17**

**(±)-Ethyl 2-(n-octyloxy)-3-[4-[[3-methyl-4-oxo-3,4-dihydro 2-quinazolinyl] methoxy]phenyl] propanoate**

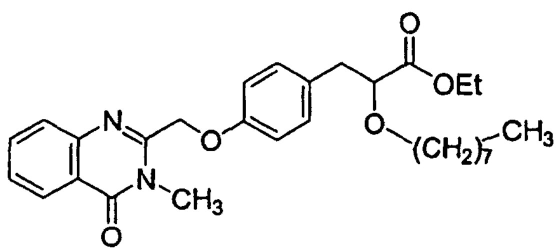

The title compound (240 mg, 38%) was obtained as a liquid from (±)-ethyl 2-hydroxy-3-[4-[[3-methyl-4-oxo-3,4-dihydro-2-quinazolinyl]methoxy] phenyl] propanoate (500 mg, 1.31 mmol), n-octylbromide (1.1 mL, 6.54 mmol) and sodium hydride (50 mg, 1.96 mmol, 95% ) as a base by a similar procedure to that described in Example 10.

$^1$H NMR ($CDCl_3$): δ 8.30 (d, J = 8.3 Hz, 1H), 7.85 - 7.65 (m, 2H), 7.51 (t, J = 8.02 Hz, 1H), 7.19 (d, J = 8.62 Hz, 2H), 6.96 (d, J = 8.62 Hz, 2H), 5.15 (s, 2H), 4.16 (q, J = 7.15 Hz, 2H), 3.94 (t, J = 6.52 Hz, 1H), 3.74 (s, 3H), 3.60 - 3.48 (m, 1H), 3.31 - 3.18 (m, 1H), 2.95 (d, J = 6.32 Hz, 2H), 1.80 - 1.40 (m, 4H), 1.40 - 1.05 (m, 11 H), 0.87 (t, J = 6.67 Hz, 3H).

**Example 18**

**(±) 2-(n-Octyloxy)-3-[4-[[3-methyl-4-oxo-3,4-dihydro-2-quinazolinyl] methoxy] phenyl]propanoic acid**

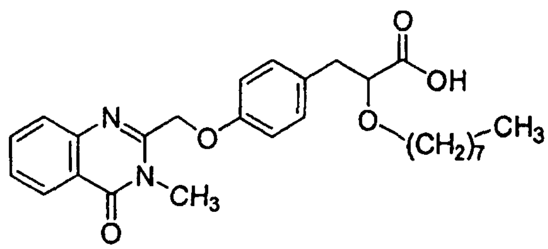

The title compound (350 mg, 88%) was obtained from (±)-ethyl 2-(n-octyloxy) - 3-[4-[[3-methyl-4-oxo-3,4-dihydro-2-quinazolinyl]methoxy] phenyl]propanoate (437 mg, 0.88 mmol) obtained in Example 17 and sodium carbonate (468 mg, 4.40 mmol) by a similar procedure to that described in Example 2.

mp: 99-100°C.

$^1$H NMR ($CDCl_3$): δ 8.31 (d, J = 8.3 Hz, 1H), 7.82 - 7.65 (m, 2H), 7.51 (t, J = 8.02 Hz, 1H), 7.19 (d, J = 8.62 Hz, 2H), 6.97 (d, J = 8.62 Hz, 2H), 5.16 (s, 2H), 4.10 - 4.00 (m, 1H), 3.74 (s, 3H), 3.62 - 3.45 (m, 1H), 3.45 - 3.28 (m, 1H), 3.18 - 2.88 (m, 2H), 1.68 - 1.42 (m, 2H), 1.42 - 1.12 (m, 10 H), 0.88 (t, J = 5.88 Hz, 3H).

**Example 19**

**(±)-Ethyl 2-benzyloxy-3-[4-[[3-methyl-4-oxo-3,4-dihydro-2-quinazolinyl] methoxy]phenyl] propanoate**

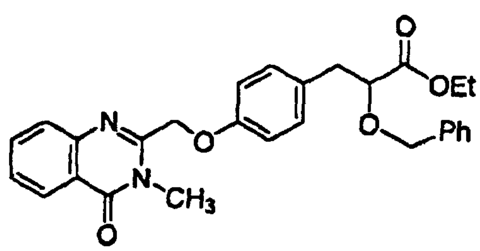

The tide compound (1.40 g, 57%) was obtained as a liquid from (±)-ethyl 2-hydroxy-3-[4-[[3-methyl-4-oxo-3,4-dihydro-2-quinazolinyl]methoxy] phenyl] propanoate ($^1$H NMR ($CDCl_3$): δ 8.30 (d, J = 7.47 Hz,1H), 7.77 - 7.72 (m, 2H), 7.53 (t, J = 3.51 Hz, 1H), 7.18 (d, J = 8.57 Hz, 2H), 6.98 (d, J = 8.57 Hz, 2H), 5.16 (s, 2H), 4.41 (m, 1H), 4.23 (q, J = 7.10 Hz, 2H), 3.73 (s, 3H), 2.93 - 3.05 (m, 2H), 1.27 (t, J = 7.10 Hz, 3H) mp: 112-118°C, 2.0 g, 5.23 mmol) obtained from (±)-ethyl 2-hydroxy-3-(4-hydroxyphenyl) propanoate (5.0 g, 23.8 mmol) (DE 26 625 163), 2-chloromethyl-3-methyl-4-oxo-3,4-dihydro-quinazoline (5.0 g, 23.8 mmol) and potassium carbonate (6.57 g, 47.6 mmol) as a base by a similar procedure to that described in Example 1; benzyl bromide (1.07 g, 6.28 mmol) and sodium hydride (260 mg, 10.46 mmol, 95%) by a similar procedure to that described in Example 10.

$^1$H NMR ($CDCl_3$): δ 8.30 (d, J= 7.89 Hz, 1H), 7.76 - 7.63 (m, 2H), 7.54 (t, J = 7.89 Hz, 1H), 7.46 - 7.04 (m, 5H), 7.20 (d, J = 8.72 Hz, 2H), 6.96 (d, J = 6.89 Hz, 2H), 5.16 (s, 2H), 4.66 (d, J = 11.85 Hz, 1H), 4.36 (d, J = 11.85 Hz, 1H), 4.30 - 4.00 (m, 3H), 3.74 (s, 3H), 3.08 - 2.92 (m, 2H), 1.24 (q, J = 7.15 Hz, 3H).

**Example 20**

**(±)-2-Benzyloxy-3-[4-[[3-methyl-4-oxo-3,4-dihydro-2-quinazolinyl] methoxy] phenyl]propanoic acid**

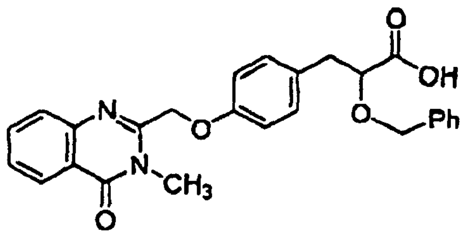

The title compound (1.0 g, 77%) was obtained from (±)-ethyl 2-benzyloxy-3-[4-[[3-methyl-4-oxo-3,4-dihydro-2-quinazolinyl]methoxy]phenyl]propanoate (1.40 g, 2.97 mmol) obtained in Example 19 and sodium carbonate (1.57 g, 14.81 mmol) by a similar procedure to that described in Example 2.

mp: 152-154°C.

$^1$H NMR ($CDCl_3$): δ 8.30 (d, J = 7.89 Hz, 1H), 7.82 - 7.68 (m, 2H), 7.53 (t, J = 7.89 Hz, 1H), 7.35 - 7.18 (m, 5H), 7.19 (d, J = 8.72 Hz, 2H), 6.97 (d, J = 8.72 Hz, 2H), 5.16 (s, 2H), 4.64 (d, J = 11.62 Hz, 1H), 4.43 (d, J = 11.2 Hz, 1H), 4.16 (dd, J = 7.45 and 4.55 Hz, 1H), 3.74 (s, 3H), 3.20 - 2.91 (m, 2H).

**Example 21**

**(±)-Ethyl 2-phenoxy 3-[4-[[3-methyl-4-oxo-3,4-dihydro-2-quinazolinyl] methoxy] phenyl]propanoate**

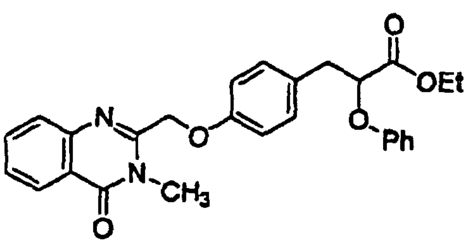

The title compound (900 mg, 95%) was obtained as a liquid from (±)-ethyl 2-phenoxy-3-(4-hydroxyphenyl) propanoate (660 mg, 2.3 mmol) obtained in preparation 5 , 2-chloromethyl-3-methyl-4-oxo-3,4-dihydroquinazoline (563 mg, 2.7 mmol) and potassium carbonate (637 mg, 4.61 mmol) as a base by a similar procedure described in Example 1.

$^1$H NMR ($CDCl_3$): δ 8.29 (d, J = 7.89 Hz, 1H), 7.65 - 7.80 (m, 2H), 7.50 (t, J = 7.10 Hz, 1H), 7.15 - 7.32 (m, 5H), 6.92 - 7.05 (m, 2H), 6.84 (d, J = 7.98 Hz, 2H), 5.16 (s, 2H), 4.75 (t, J = 6.39 Hz, I H), 4.16 (q, J = 6.36 Hz, 2H), 3.72 (s, 3H), 3.20 (d, J = 6.64 Hz, 2H), 1.17 (t, J = 7.15 Hz, 3H).

## Example 22

**(±)-2-Phenoxy-3-[4-[[3-methyl-4-oxo-3,4-dihydro-2-quinazolinyl]methoxy]phenyl] propanoic acid**

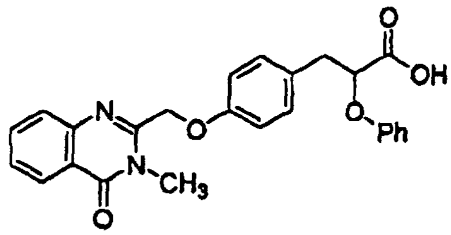

The title compound (0.45 g, 53%) was obtained from (±)-ethyl 2-phenoxy-3-[4-[[3-methyl-4-oxo-3,4-dihydro-2-quinazolinyl]methoxy]phenyl]propanoate (900 mg, 1.96 mmol) obtained in Example 21 and sodium carbonate (1.04 g, 9.82 mmol) by a similar procedure to that described in Example 2.

mp: 156-158°C.

$^1$H NMR ($CDCl_3$): δ 8.31 (d, J = 7.89 Hz, 1H), 7.85 - 7.65 (m, 2H), 7.52 (t, J = 6.39 Hz, 1 H), 7.35 - 7.21 (m, 5H), 7.02 - 6.96 (m, 2H), 6.87 (d, J = 7.93 Hz, 2H), 5.15 (s, 2H), 4.85 (t, J = 6.02 Hz, 1H), 3.73 (s, 3H), 3.26 (d, J = 6.14 Hz, 2H).

## Example 23

**(±)-Ethyl 2-(2-methoxyethoxy)-3-[4-[[3-methyl-4-oxo-3,4-dihydro-2-quinazolinyl] methoxy]phenyl]propanoate**

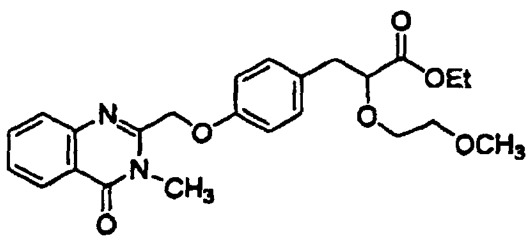

The title compound (560 mg, 83%) was obtained from (±)-ethyl 2-(2-methoxy-ethoxy)-3-(4-hydroxyphenyl) propanoate (410 mg, 1.529 mmol), 2-chloromethyl-3-methyl-4-oxo-3,4-dihydroquinazoline (640 mg, 3.058 mmol) and potassium carbonate (634 mg, 4.58 mmol) as a base by a similar procedure to that described in Example 1.

$^1$H NMR ($CDCl_3$): δ 8.31 (d, J = 7.45 Hz, 1H), 7.84 - 7.68 (m, 2H), 7.51 (t, J = 6.41 Hz, 1H), 7.20 (d, J = 8.61 $H_2$, 2H), 6.98 (d, J = 8.61 Hz, 2H), 5.18 (s, 2H), 4.21 - 4.02 (m, 4H), 3.76 (s, 3H), 3.75 - 3.66 (m, 1H), 3.65 - 3.40 (m, 3H), 3.31 (s, 3H), 3.01 - 2.96 (m, 1H), 1.22 (t, J = 7.15 Hz, 3H).

## Example 24

**(±)-2-(2-Methoxyethoxy)-3-[4-[[3-methyl-4-oxo-3,4-dihydro-2-quinazolinyl] methoxy]phenyl]propanoic acid**

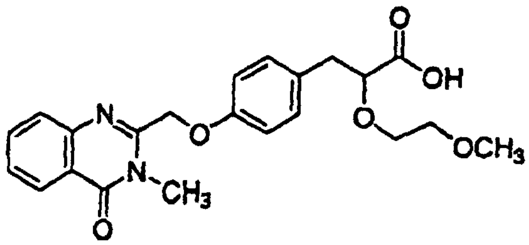

The title compound (270 mg, 51%) was obtained from (±)-ethyl 2-(2-methoxy-ethoxy)-3-[4-[[3-methyl-4-oxo-3,4-dihydro-2-quinazolinyl] methoxy] phenyl] propanoate (560 mg, 1.27 mmol) obtained in Example 23 and sodium carbonate (675 mg, 6.36 mmol) by a similar procedure to that described in Example 2.

mp: 146-148°C.

$^1$H NMR ($CDCl_3$): δ 8.30 (d, J = 7.47 Hz, 1H), 7.82 - 7.69 (m, 2H), 7.52 (t, *J* = 6.41 Hz, 1H), 7.21 (d, J = 8.63 Hz, 2H), 6.99 (d, J = 8.63 Hz, 2H), 5.17 (s, 2H), 4.06 (dd, J= 3.46 and 8.76 Hz, 1H), 3.75 (s, 3H), 3.71 - 3.42 (m, 4H), 3.40 (s, 3H), 3.19 (dd, J = 3.46 and 14.16 Hz, 1H), 2.91 (dd, J = 8.76 and 14.16 Hz, 1H).

## Example 25

**(±)-Ethyl 2-ethoxy-3-[4-[2-[2-ethyl-4-oxo-3,4-dihydro-3-quinazolinyl]ethoxy] phenyl]propanoate**

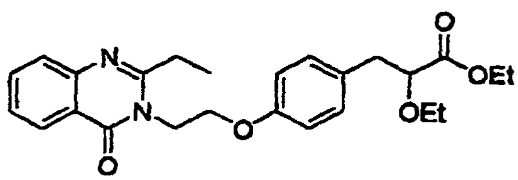

### Method A

To a stirred solution of 2-ethyl-4-oxo-3,4-dihydroquinazoline (200 mg, 1.15 mmol) in DMF (3 mL) was added potassium carbonate (317 mg, 230 mmol) and stirred for 30 min. To this reaction mixture was added a solution of (±) ethyl 2-ethoxy -3-[4-(2-bromoethoxy)phenyl]propanoate (475 mg, 1.38 mmol) (disclosed in U.S. Patent Application Serial No. 09/012,585 now U.S. Patent 6,054,453) in DMF (2 mL) and stined for 24 h at 30°C. The reaction mixture was diluted with water and extracted with ethyl acetate (3 + 10 mL). The combined organic extracts were wasbed with brine, dried over anhydrous $Na_2SO_4$ and concentrated to yield the title compound (260 mg, 51 %).

### Method B

To a stirred suspension of sodium hydride (1.15 g, 28.7 mmol, 60%) in dry DMF (60 mL) was added 2-ethyl-4-oxo-3,4-dihydroquinazoline (5.0 g, 28.7 mmol) at 0°C and stirred for 0.5 h at the same temperature. To the reaction mixture was added lithium bromide (5.0 g, 57.47 mmol) in one portion and stirring continued for further 0.5 h at 0°C. A solution of (±) ethyl 2-ethoxy-3-[4-(2-bromoethoxy) phenyl] propanoate (14.87 g, 43.1 mmol), in dry DMF (20 mL) was added and stirred for 5 h at 30°C. The reaction mixture was diluted with water and extracted with ethyl acetate (3 + 10 mL). The combined organic layers were washed with brine, dried over anhydrous $Na_2SO_4$ and concentrated to yield the title compound (6.1 g, 48%).

**Method C**

To a stirred solution of (±) ethyl 2-ethoxy-3-[4-[2-[N-(2-aminobenzoyl) amino-ethoxy]phenyl] propanoate (15 g, 37.5 mmol) obtained in preparation 6, in a mixture of xylene (50 mL) and propionic acid (50 mL) was added triethylamine (10.4 mL, 7.5 g, 75 mmol) followed by addition of propanoyl chloride (3.6 mL, 3.8 g, 41 mmol) at ca 30°C and stirred for 2 h. The reaction mixture was immersed in pre-heated oil bath at 160°C and stirred for 24 h. at the same temperature. Water was added to the reaction mixture and extracted with ethyl acetate (3 + 100 mL). The organic extracts were washed with brine, dried over anhydrous $Na_2SO_4$ and concentrated. The crude compound was crystallised from diisopropyl ether to yield the title compound (11.8 g, 72%).

mp: 86-88°C.

$^1H$ NMR ($CDCl_3$): δ 8.25 (d, J = 7.89 Hz, 1H), 7.80 - 7.60 (m, 2H), 7.43 (t, J = 7.89 Hz, 1H), 7.12 (d, J = 8.62 Hz, 2H), 6.76 (d, J = 8.62 Hz, 2H), 4.54 (t, J = 5.07 Hz, 2H), 4.30 (t, J = 5.07 Hz, 2H),4.15 (q, J = 7.06 Hz, 2H), 3.92 (t, J = 6.4 Hz, 1H), 3.68 - 3.48 (m, 1H), 3.40 - 3.20 (m, 1H), 3.11 (q, J = 7.38 Hz, 2H), 2.91 (d, J = 6.64 Hz, 2H), 1.44 (t, J = 3.8 Hz, 3H), 1.21 (t, J = 7.06 Hz, 3H), 1.14 (t, J = 7.38 Hz, 3H).

**Example 26**

**(±)-2-Ethoxy-3-[4-[2-[2-ethyl-4-oxo-3,4-dihydro-3-quinazolinyl] ethoxy] phenyl]propanoic acid**

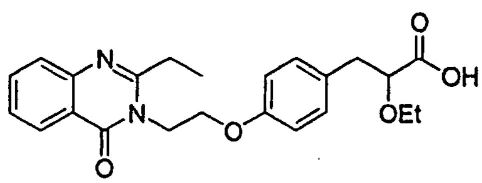

The title compound (72 mg, 70%) was obtained from (±)-ethyl 2-ethoxy-3-[4-[2-[2-ethyl-4-oxo-3,4-dihydro-3-quinazolinyl]ethoxy]phenyl]propanoate (110 mg, 0.25 mmol) obtained in Example 25 and sodium carbonate (133 mg, 1.25 mmol) by a similar procedure to that described in Example 2.

mp 140-141°C.

$^1H$ NMR (DMSO-$d_6$): δ 8.13 (d, J = 7.89 Hz, 1H), 7.82 (t, J = 7.01 Hz, 1H), 7.64 (d, J = 8.21 Hz, 1H), 7.50 (t, J = 7.26 Hz, 1H), 7.13 (d, J = 8.50 Hz, 2H), 6.84 (d, J = 8.50 Hz, 2H), 4.47 (t, J = 5.19 Hz, 2H), 4.26 (t, J = 5.19 Hz, 2H), 3.99 - 3.84 (m, 1H), 3.60 - 3.40 (m, 1H), 3.40 - 3.20 (m, 1H), 3.06 (q, J = 6.96 Hz, 2H), 2.88 (q, J = 6.64 Hz, 2H), 1.32 (t, J = 7.17 Hz, 3H), 1.02 (t, J = 6.96 Hz, 3H).

**Comparative Example 27**

**[2R, N(1S)] 2-ethoxy-3-[4-[2-[2-ethyl-4-oxo-3,4-dihydro-3-quinazolinyl]ethoxy]-N-(2-hydroxy-1-phenylethyl) propanamide (27a)**

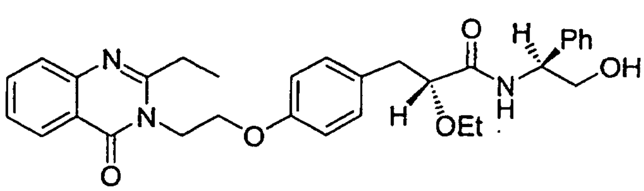

**[2S, N(1S)] 2-ethoxy-3-[4-[2-[2-ethyl-4-oxo-3,4-dihydro-3-quinazolinyl]ethoxy]phenyl]-N-(2-hydroxy-1-phenylethyl)propanamide (27b)**

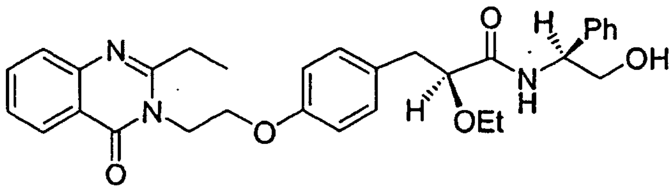

The title compounds [2R, N(1S)] propanamide (27a) and [2S, N(1S)] propanamide (27a) were obtained from (±)-2-ethoxy-3-[4-[2-[2-ethyl-4-oxo-3,4-dihydro-3-quinazolinyl]ethoxy]phenyl]propanoic acid (187 mg, 0.45 mmol) obtained in Example 26, triethylamine (288 μL, 2.05 mmol), pivaloyl chloride (61 μL, 0.5 mmol) and S-(+)-2-phenylglycinol (62 mg, 0.45 mmol) by a similar procedure to that described in Comparative Example 4.

Spectral data for (27a):

mp: 128-130°C; $[\alpha]_D^{25}$ = + 46.1 (c = 1.0, MeOH).

$^1H$ NMR ($CDCl_3$): δ 8.26 (d, J = 7.89 Hz, 1H), 7.80 - 7.68 (m, 2H), 7.46 (t, J = 7.24 Hz, 1H), 7.38 - 6.96 (m, 7H), 6.79 (d, J = 8.49 Hz, 2H), 5.01 - 4.90 (m, 1H), 4.56 (t, J = 4.98 Hz, 2H), 4.34 (t, J = 4.98 Hz, 2H), 3.95 (dd, J = 3.80 and 6.66 Hz, 1H), 3.68 (d, J = 5.40 Hz, 2H), 3.48 (q, J = 6.95 Hz, 2H), 3.21 - 3.10 (m, 2H), 3.10 - 2.84 (m, 2H), 1.46 (t, J = 7.31 Hz, 3H), 1.13 (t, J = 7.05 Hz, 3H).

Spectral data for (**27a**):

mp: 156-158°C; $[\alpha]_D^{25}$ = + 4.1 (c = 1.0, MeOH).

$^1H$ NMR ($CDCl_3$): δ 8.28 (d, J = 7.89 Hz, 1H), 7.81 - 7.68 (m, 2H), 7.46 (t, J = 7.24 Hz, 1H), 7.26 - 7.00 (m, 7H), 6.70 (d, J = 8.49 Hz, 2H), 5.02 - 4.91 (m, 1H), 4.57 (t, J = 5.14 Hz, 2H), 4.30 (t, J = 5.14 Hz, 2H), 3.98 (dd, J = 3.80 and 6.66 Hz, 1H), 3.85 (d, J = 4.25 Hz, 2H), 3.60 - 3.45 (m, 2H), 3.16 (q, J = 7.19 Hz, 2H), 3.10 - 2.80 (m, 2H), 1.47 (t, J = 7.36 Hz, 3H), 1.17 (t, J = 7.01 Hz, 3H).

## Example 28

**(+)-2-Ethoxy-3-[4-[2-[2-ethyl-4-oxo-3,4-dihydro-3-quinazolinyl]ethoxy]phenyl] propanoic acid**

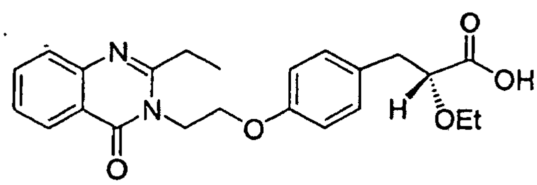

The title compound (83 mg, 71 %) was obtained from [2R, N(1S)] 2-ethoxy-3-[4-[2-[2-ethyl-4-oxo-3,4-dihydro-3-quinazolinyl]ethoxy]phenyl]-N-(2-hydroxy-1-phenylethyl)propanamide (150 mg, 0.283 mmol) obtained in Comparative Example **27a** by a similar procedure to that described in Example 6.

mp: 120-122°C.

$[\alpha]_D^{25}$ = + 19.2 (c = 1.0, MeOH).

$^1H$ NMR ($CDCl_3$): δ 8.24 (d, J = 7.88 Hz, 1H), 7.80 - 7.63 (m, 2H), 7.43 (t, J = 7.21 Hz, 1H), 7.11 (d, J = 8.63 Hz, 2H), 6.77 (d, J = 8.63 Hz, 2H), 4.53 (t, J = 4.98 Hz, 2H), 4.30 (t, J = 4.98 Hz, 2H), 4.01 (dd, J = 4.47 and 7.38 Hz, 1H), 3.69 - 3.34 (m, 2H), 3.19 - 2.85 (m, 4H), 1.42 (t, J = 7.42 Hz, 3H), 1.14 (t, J = 6.94 Hz, 3H).

**Example 29**

**(-)-2-Ethoxy-3-[4-[2-[2-ethyl-4-oxo-3,4-dihydro-3-quinazolinyl]ethoxy]phenyl]propanoic acid**

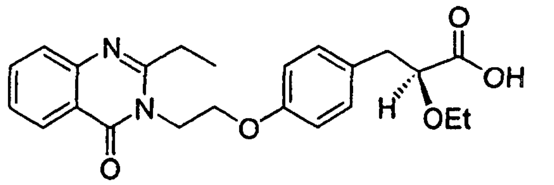

The title compound (170 mg, 82 %) was obtained from [2S, N(1S)] 2-ethoxy-3-[4-[2-[2-ethyl-4-oxo-3,4-dihydro-3-quinazolinyl]ethoxy]phenyl]-N-(2-hydroxy-1-phenylethyl)propanamide (267 mg, 0.504 mmol) obtained in Comparative Example **27b** by a similar procedure to that described in Example 6.

mp: 134-136°C

$[\alpha]_D^{25}$ = -19.2 (c = 1.0, MeOH).

$^1$H NMR ($CDCl_3$): δ 8.24 (d, J = 7.89 Hz, 1H), 7.79 - 7.61 (m, 2H), 7.43 (t, J = 7.66 Hz, 1H), 7.11 (d, J = 8.63 Hz, 2H), 6.77 (d, J = 8.63 Hz, 2H), 4.53 (t, J = 4.98 Hz, 2H), 4.31 (t, J = 4.98 Hz, 2H), 4.02 (dd, J = 4.22 and 7.12 Hz, 1H), 3.61 - 3.32 (m, 2H), 3.16 - 2.82 (m, 4H), 1.43 (t, J = 7.36 Hz, 3H), 1.15 (t, J = 6.96 Hz, 3H).

**Example 30**

**(+)-Ethyl 2-ethoxy-3-[4-[2-[2-ethyl-4-oxo-3,4-dihydro-3-quinazolinyl]ethoxy]phenyl]** propanoate

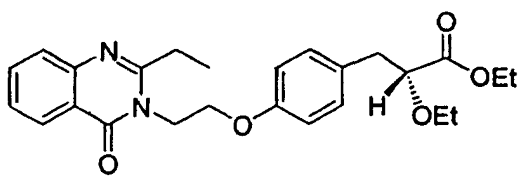

To a stirred suspension of potassium carbonate (172 mg, 1.24 mmol) in DMF (2 mL) was added a solution of (+)-2-ethoxy-3-[4-[2-[2-ethyl-4-oxo-3,4-dihydro-3-quinazolinyl]ethoxy]phenyl]propanoic acid (256 mg, 0.624 mmol) obtained in Example 28 in dry DMF (2 mL) and stirred at ca 30°C for 30 min. To this reaction mixture was added ethyl bromide (93 mL, 1.24 mmol) slowly dropwise and stirred for 1 h at *ca* 30°C. The reaction mixture was poured into water (15 mL) and extracted with ethyl acetate (2 x 15 mL). The combined ethyl acetate extracts were washed with water, brine, dried over anhydrous $Na_2SO_4$ and concentrated. The crude product was pacified by column chromatography using ethyl acetate and pet ether (4 : 6) as eluent to afford the title compound (92 mg, 86 %).

mp: 114-116 °C.

$[\alpha]_D^{25}$ = 11.1 (c = 1.0, MeOH).

$^1$H NMR ($CDCl_3$) δ 8.26 (d, J = 7.88 Hz, 1H), 7.66 - 7.85 (m, 2H), 7.45 (t, J = 6.68 Hz, 1H), 7.13 (d, J = 8.39 Hz, 2H), 6.78 (d, J = 8.62 Hz, 2H), 4.55 (t, *J* = 5.14 Hz, 2H), 4.32 (t, J = 5.03 Hz, 2H), 4.16 (q, J = 7.09 Hz, 2H), 3.93 (t, J = 6.57 Hz, 1H), 3.69 - 3.50 (m, 1H), 3.42 - 3.25 (m, 1H), 3.12 (q, J = 7.36 Hz, 2H), 2.93 (d, J = 6.55 Hz, 2H), 1.45 (t, J = 7.33 Hz, 3H), 1.32 - 1.11 (m, 6H).

## Example 31

**(-)-Ethyl 2-ethoxy-3-[4-[2-[2-ethyl-4-oxo-3,4-dihydro-3-quinazolinyl]ethoxy] phenyl]propanoate**

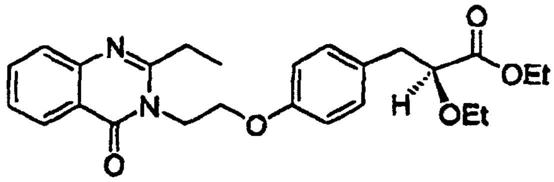

The title compound (75 mg, 70%) was obtained from (-)-2-ethoxy-3-[4-[2-[2-ethyl-4-oxo-3,4-dihydro-3-quinazolinyl]ethoxy]phenyl]propanoic acid (100 mg, 0.243 mmol) obtained in Example 29, ethyl bromide (36 mL, 0.487 mmol) and potassium carbonate as a base by a similar procedure to that described in Example 30.

mp: 108-110°C

$[\alpha]_D^{25}$ = - 11.68 (c = 0.50, MeOH).

$^1H$ NMR ($CDCl_3$): δ 8.26 (d, J = 7.47 Hz, 1H), 7.83 - 7.64 (m, 2H), 7.45 (t, J = 7.26 Hz, 1H), 7.13 (d, J = 8.62 Hz, 2H), 6.78 (d, J = 8.62 Hz, 2H), 4.55 (t, J = 5.14 Hz, 2H), 4.32 (t, J = 5.03 Hz, 2H), 4.16 (q, J = 7.10 Hz, 2H), 3.93 (t, J = 6.55 $H_2$, 1H), 3.69 - 3.51 (m, 1H), 3.42 - 3.26 (m, 1H), 3.12 (q, J = 7.38 Hz, 2H), 2.93 (d, J = 6.59 Hz, 2H), 1.45 (t, J = 7.33 Hz, 3H), 1.30 - 1.11 (m, 6H).

## Example 32

**(±)-Ethyl 2-ethoxy-3-[4-[2-[2-methyl-4-oxo-3,4-dihydro-3-quinazolinyl]ethoxy]phenyl] propanoate**

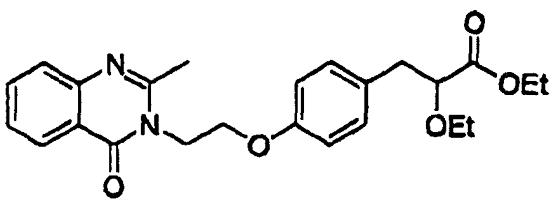

The title compound (229 mg, 33%) was obtained as a liquid from 2-methyl-4-oxo-3,4-dihydroquinazoline (0.25 g, 1.56 mmol), potassium carbonate (431 mg, 3.12 mmol) and (±)-ethyl 2-ethoxy-3-[4-(2-bromoethoxy)phenyl]propanoate (647 mg, 1.87 mmol) (disclosed in U.S. Patent Application Serial No. 09/012,585 now U.S. Patent 6,054,453) by a similar procedure to that described in Example 25.

$^1H$ NMR ($CDCl_3$): δ 8.24 (d, J = 8.31 Hz, 1H), 7.79 - 7.60 (m, 2H), 7.44 (t, J = 6.85 Hz, 1H), 7.12 (d, J = 8.63 Hz, 2H), 6.77 (d, J = 8.63 Hz, 2H), 4.52 (t, J = 4.93 Hz, 2H), 4.32 (t, J = 4.82 Hz, 2H), 4.12 (q, J = 5.65 Hz, 2H), 3.92 (t, J = 6.64 Hz, 1H), 3.63 - 3.50 (m, 1H), 3.39 - 3.21 (m, 1H), 2.92 (d, J = 6.65 Hz, 2H), 2.81 (s, 3H), 1.29 - 1.09 (m, 6H).

## Example 33

**(±)-2-Ethoxy-3-[4-[2-[2-methyl-4-oxo-3,4-dihydro-3-quinazolinyl]ethoxy]phenyl] propanoic acid**

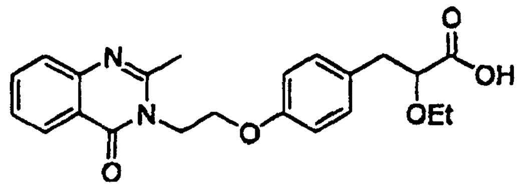

The title compound (100 mg, 61%) was obtained from (±)-ethyl 2-ethoxy 3-[4-[2-[2-methyl-4-oxo-3,4-dihydro-3-quinazolinyl]ethoxy]phenyl]propanoare (175 mg, 0.41 mmol) obtained in Example 32 and sodium carbonate (219 mg, 2.06 mmol) by a similar procedure to that described in Example 2.

mp: 124-126 °C.

$^1$H NMR ($CDCl_3$): δ 8.25 (d, J = 7.89 Hz, 1H), 7.79 - 7.60 (m, 2H), 7.45 (t, J = 7.47 Hz, 1H), 7.13 (d, J = 8.62 Hz, 2H), 6.79 (d, J = 8.62 Hz, 2H), 4.53 (t, J = 4.82 Hz, 2H), 4.33 (t, J = 4.98 Hz, 2H), 4.08 - 3.99 (m, 1H), 3.62 - 3.39 (m, 2H), 3.12 - 2.86 (m, 2H), 2.81 (s, 3H), 1.16 (t, J = 7.05 Hz, 3H).

## Comparative Example 34

**[2R, N(1S)] 2-ethoxy-3-[4-[2-[2-methyl-4-oxo-3,4-dihydro-3-quinazolinyl]ethoxy]phenyl]-N-(2-hydroxy-1-phenylethyl)propanamide (34a) :**

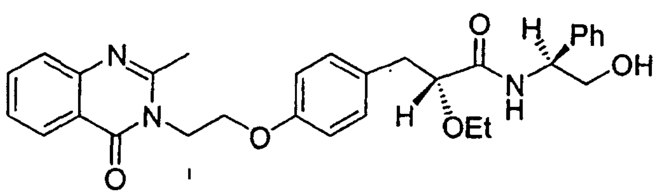

**[2S, N(1S)] 2-ethoxy-3-[4-[2-[2-methyl-4-oxo-3,4-dihydro-3-quinazolinyl]ethoxy]phenyl]-N-(2-hydroxy-1-phenylethyl)propanamide (34b) :**

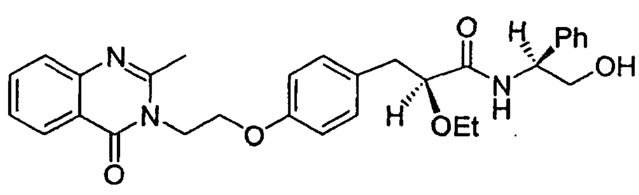

The title compounds [2R, N(1S)] propanamide (34a) and [2S, N(1S)] propanamide (34b) were obtained from (±)-2-ethoxy-3-[4-[2-[2-methyl-4-oxo-3,4-dihydro-3-quinazolinyl]ethoxy]phenyl]propanoic acid (1.5 g, 3.78 mmol) obtained in Example 33, triethylamine (2.37 mL, 16.87 mmol), pivaloyl chloride (0.56 mL, 4.17 mmol) and S-(+)-2-phenylglycinol (520 mg, 3.78 mmol) by a similar procedure to that described in Comparative Example 4.

Spectral data for (**34a**):

mp: 150-152 °C. $[\alpha]_D^{25}$ = 43.0 (c = 0.4, MeOH).

$^1$H NMR ($CDCl_3$): δ 8.23 (d, J = 7.89 Hz, 1H), 7.79 - 7.59 (m, 2H), 7.43 (t, J = 7.89 Hz, 1 H), 7.79 - 7.59 (m, 7H), 7.43 (t, J = 8.4 Hz, 2H), 4.99 - 4.90 (m, 1 H), 4.52 (t, J = 4.82 Hz, 2 H), 4.33 (t, J = 4.82 Hz, 2 H), 3.93 (dd, J =

6.18 and 3.78 Hz, 1 H), 3.69 - 3.60 (m, 2H), 3.45 (q, J = 7.02 Hz, 2H), 3.08 (dd, J = 16.11 and 3.74 Hz, 1 H), 2.91 (dd, J = 14.11 and 6.46 Hz, 1 H), 2.81 (s, 3 H), 1.10 (t J = 6.94 Hz, 3 H).

Spectral data for (**34b**):

mp: 158-160 °C. $[\alpha]_D^{25}$ = 7.5 (c = 0.4, MeOH).

$^1$H NMR ($CDCl_3$): δ 8.27 (d, J = 7.89 Hz, 1H), 7.80 - 7.61 (m, 2H), 7.45 (t, J = 7.38 Hz, 1H), 7.29 - 6.99 (m, 7H), 6.70 (d, J = 8.67 Hz, 2H), 5.01 - 4.92 (m, 1H), 4.55 (t, J = 4.93 Hz, 2H), 4.31 (t, J = 4.93 Hz, 2H), 3.97 (dd, J = 6.59 and 3.88 Hz, 1H), 3.88 - 3.80 (m, 2H), 3.59 - 3.43 (m, 2H), 3.05 (dd, J = 14.11 and 3.50 Hz, 1H), 2.92 - 2.80 (m, 3H), 2.82 (s, 3H), 1.17 (t, J = 6.94 Hz, 2H)

## Example 35

**(+)-2-Ethoxy-3-[4-[2-[2-methyl-4-oxo-3,4-dihydro-3-quinazolinyl]ethoxy]phenyl] propanoic acid**

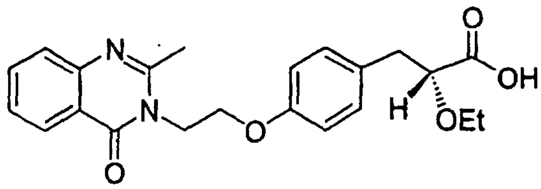

The title compound (330 mg, 81%) was obtained from [2R, N(1S)] 2-ethoxy-3-[4-[2-[2-methyl-4-oxo-3,4-dihydro-3-quinazolinyl]ethoxy]phenyl]-N-(2-hydroxy-1-phenylethyl]propanamide (530 mg, 1.03 mmol) obtained in Comparative Example **34a** by a similar procedure to that described in Example 6.

mp: 110-112 °C.

$[\alpha]_D^{25}$ = 16.7 (c = 1.0, MeOH).

$^1$H NMR ($CDCl_3$) : δ 8.23 (d, J = 7.89 Hz, 1H), 7.79 - 7.60 (m, 2H), 7.43 (t, J = 7.31 Hz, 1H), 7.13 (d, J = 8.3 Hz, 2H), 6.77 (d, J = 8.39 Hz, 2H), 4.52 (t, J = 4.77 Hz, 2H), 4.31 (t, J = 4.77 Hz, 2H), 4.00 (dd, J = 7.47 and 4.56 Hz, 1H), 3.66 - 3.31 (m, 2H), 3.05 (dd, J = 14.16, 4.5 Hz, 1H), 2.89 (dd, J = 14.16, 7.47 Hz, 1H), 2.81 (s, 3H), 1.15 (t, J = 7.01 Hz, 3H).

## Example 36

**(-)-2-Ethoxy-3-[4-[2-[2-methyl-4-oxo-3,4-dihydro-3-quinazolinyl]ethoxy]phenyl] propanoic acid:**

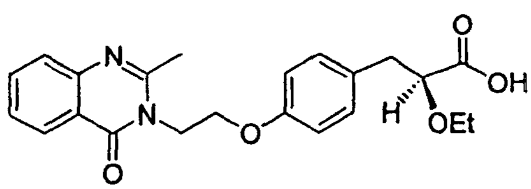

The title compound (340 mg, 78%) was obtained from [2S, N(1S)]-2-ethoxy-3-[4-[2-[2-methyl-4-oxo-3,4-dihydro-3-quinazolinyl]ethoxy]phenyl]-N-(2-hydroxy-1-phenylethyl)propanamide (570 mg, 1.1 mmol) obtained in Comparative Example **34b** by a similar procedure to that described in Example 6.

mp: 132-134°C

$[\alpha]_D^{25}$ = -16.4 (c= 1.0, MeOH).

$^1$H NMR ($CDCl_3$): δ 8.24 (d, J = 7.89 Hz, 1H), 7.78 - 7.60 (m, 2H), 7.43 (t, J = 7.35 Hz, 1H), 7.12 (d, J = 8.53 Hz, 2H), 6.77 (d, J = 8.49 Hz, 2H), 4.52 (t, J = 4.88 Hz, 2H), 4.32 (t, J = 4.93 Hz, 2H), 4.01 (dd, J = 7.4 and 4.54 Hz, 1H), 3.65 - 3.31 (m, 2H), 3.05 (dd, J = 14.11 and 4.47 Hz, 1H), 2.92 (dd,14.11 and 7.47 Hz, 1H), 2.81 (s, 3H), 1.15 (t, J = 6.96 Hz, 3H).

**Example 37**

**(+)-Ethyl 2-ethoxy-3-[4-[2-[2-methyl-4-oxo-3,4-dihydro-3-quinazolinyl]ethoxy] phenyl] propanoate**

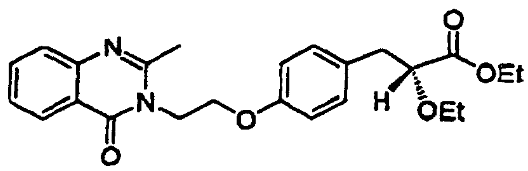

The title compound (50 mg, 78%) was obtained from (+)-2-ethoxy-3-[4-[2-[2-methyl-4-oxo-3,4-dihydro-3-quinazolinyl]ethoxy]phenyl]propanoic acid (60 mg, 0.15 mmol) obtained in Example 35, potassium carbonate (42 mg, 0.30 mmol), and ethyl bromide (33 mg, 0.30 mmol) by a similar procedure to that described in Example 30.

mp: 108-110°C.

$[\alpha]_D^{25}$ = 12.8 (c = 0.5, MeOH).

$^1H$ NMR ($CDCl_3$) : δ 8.26 (d, J = 8.3 Hz, 1H), 7.80 - 7.60 (m, 2H), 7.46 (t, J = 7.47 Hz, 1H), 7.15 (d, J = 8.3 Hz, 2H), 6.79 (d, J = 8.42 Hz, 2H), 4.54 (t, J = 4.81 Hz, 2H), 4.34 (t, J = 4.82 Hz, 2H), 4.17 (q, J = 7.05 Hz, 2H), 3.94 (t, J = 6.59 Hz, 1H), 3.68 - 3.50 (m, 1H), 3.41 - 3.22 (m, 1H), 2.94 (d, J = 6.44 Hz, 2H), 2.84 (s, 3H), 1.30 - 1.10 (m, 6H).

**Example 38**

**(-)-Ethyl 2-ethoxy-3-[4-[2-[2-methyl-4-oxo-3,4-dihydro-3-quinazolinyl]ethoxy]phenyl] propanoate**

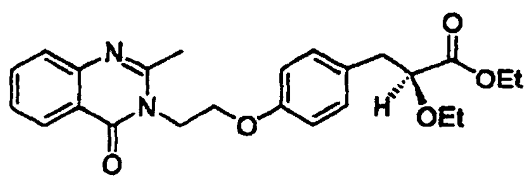

The title compound (51 mg, 79%) was obtained from (-)-2-ethoxy-3-[4-[2-[2-methyl-4-oxo-3,4-dihydro-3-quinazolinyl]ethoxy]phenyl]propanoic acid (60 mg, 0.15 mmol) obtained in Example 38, ethyl bromide (33 mg, 0.30 mmol) and potassium carbonate (42 mg, 0.30 mmol) as a base by a similar procedure to that described in Example 30.

mp: 112-114°C

$[\alpha]_D^{25}$ = 12.8 (c = 0.50, MeOH).

$^1H$ NMR ($CDCl_3$): δ 8.27 (d, J = 7.89 Hz, 1H), 7.80 - 7.61 (m, 2H), 7.46 (t, J = 7.42 Hz, 1H), 7.15 (d, J = 8.10 $H_2$, 2H), 6.79 (d, J = 8.63 Hz, 2H), 4.54 (t, J = 4.89 Hz, 2H), 4.34 (t, J= 4.82 Hz, 2H), 4.17 (q, J = 7.09 Hz, 2H), 3.94 (t, J = 6.64 Hz, 1H), 3.69 - 3.51 (m, 1H), 3.40 - 3.23 (m, 1H), 2.94 (d, J = 6.31 Hz, 2H), 2.84 (s, 3H), 1.32 - 1.11 (m, 6H).

Example 39

**(±)-Ethyl 2-ethoxy-3-[4-[2-[4-oxo-3,4-dihydro-3-quinazolinyl]ethoxy] phenyl]propanoate**

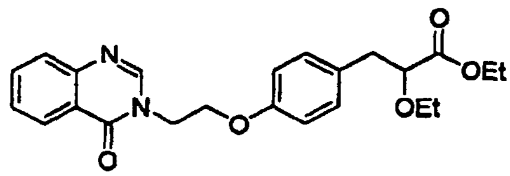

The title compound (525 mg, 62%) was obtained from 4-oxo-3,4-dihydro-quinazoline (300 mg, 2.05 mmol), potassium carbonate (0.567 g, 4.1 mmol) and ethyl 2-echoxy-3-[4-(2-bromoethoxy)phenyl]propanoate (0.851 g, 2.46 mmol) (disclosed in U.S. Patent Application Serial No. 09/012,585 now U.S. Patent 6,054,453), by a similar procedure to that described in Example 25.

mp: 90-92°C.

$^1$H NMR ($CDCl_3$): δ 8.30 (d, J = 8.3 Hz, 1H), 8.21 (s, 1H), 7.81 - 7.71 (m, 2H), 7.50 (t, J = 6.22 Hz, 1H), 7.12 (d, J = 8.39 Hz, 2H), 6.78 (d, J = 8.39 Hz, 2H), 4.40 (t, J = 4.77 Hz, 2H), 4.27 (t, J = 4-61 Hz, 2H), 4.14 (q, J = 7.11 Hz, 2H), 3.92 (t, J = 6.64 Hz, 1H), 3.68 - 3.51 (m, 1H], 3.40 - 3.22 (m, 1H), 2.91 (d, J = 6.64 Hz, 2H), 1.29 - 1.10 (m, 6H).

Example 40

**(±)-2-Ethoxy-3-[4-[2-[4-oxo-3,4-dihydro-3-quinazolinyl]ethoxy]phenyl]propanoic acid**

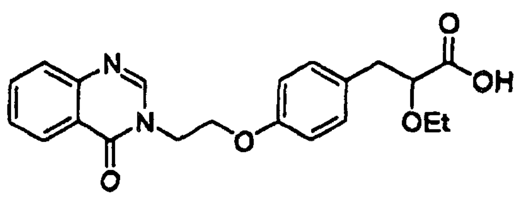

The title compound (125 mg, 67%) was obtained from (±)-ethyl 2-ethoxy-3-[4-[2-[4-oxo-3,4-dihydro-3-quinazolinyl]ethoxy]phenyl]propanoate (0.2 g, 0.487 mmol) obtained in Example 39 and sodium carbonate (0.258 g, 2.44 mmol) by a similar procedure to that described in Example 2.

mp: 160-162°C.

$^1$H NMR ($CDCl_3$): δ 8.42 (s, 1H), 8.19 (d, J = 7.89 Hz, 1H), 7.86 (t, J = 7.63 Hz, 1H), 7.71 (d, J = 7.98 Hz, 1H), 7.58 (t, J = 7.47 Hz, 1H), 7.13 (d, J = 7.98 Hz, 2H), 6.86 (d, J = 7.98 Hz, 2H), 4.38 (d, J = 4.98 Hz, 2H), 4.28 (d, J = 4.66 Hz, 2H), 3.93 (t, J = 6.27 Hz, 1H), 3.58 - 3.42 (m, 2H), 2.82 (d, J = 7.98 Hz, 2H), 1.03 (t, J = 7.05 Hz, 3H).

Example 41

**(±)-Ethyl 2-phenoxy-3-[4-[2-[2-ethyl-4-oxo-3,4-dihydro-3-quinazolinyl]ethoxy]phenyl] propanoate**

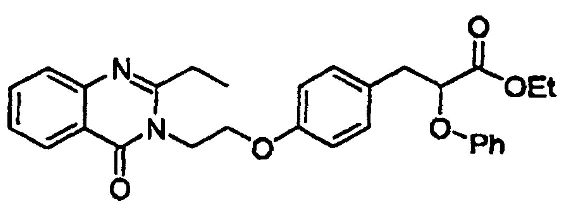

The title compound (140 mg, 20%) was obtained from (±)-2-ethyl-4-oxo-3,4-dihydroquinazoline (250 mg, 1.43 mmol), potassium carbonate (396 mg, 2.87 mmol) and ethyl 2-phenoxy-3-[4-(2-bromoethoxy)phenyl]propanoate (677 mg, 1.72 mmol) by a similar procedure to that described in Example 25.

mp: 142-144°C.

$^1$H NMR ($CDCl_3$): δ 8.20 (d, J 8.30 Hz, 1H), 7.60 (t, J = 5.44 Hz, 2H), 7.45 (t, J = 6.73 Hz, 1H), 7.28 - 7.12 (m, 4H), 6.90 (t, J = 6.25 Hz, 1H), 6.81 - 6.71 (m, 4H), 4.70 (m, 1H), 4.52 (t, J = 5.64 Hz, 2H), 4.26 (t, J = 5.19 Hz, 2H), 4.14 (q, J = 7.09 Hz, 2H), 3.18 - 3.00 (m, 4H), 1.42 (t, J = 7.36 Hz, 3H), 1.17 (t, J = 7.08 Hz, 3H).

## Example 42

**(±)-2-Phenoxy-3-[4-[2-[2-ethyl-4-oxo-3,4-dihydro-3-quinazolinyl]ethoxy]phenyl]propanoic acid**

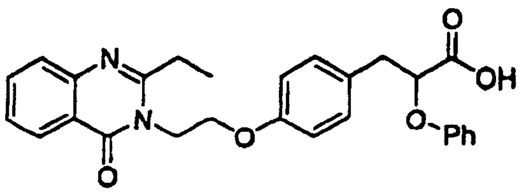

The title compound (80 mg, 0.17 mmol) was obtained from (±)-ethyl 2-phenoxy-3-[4-[2-[2-ethyl-4-oxo-3,4-dihydro-3-quinazolinyl]ethoxy]phenyl]propanoate (150 mg, 0.308 mmol) obtained in Example 41 and sodium carbonate (163 mg, 1.54 mmol) by a similar procedure to that described in Example 2.

mp: 174-176°C.

$^1$H NMR (DMSO-$d_6$) : δ 8.13 (d, J = 7.89 Hz, 1H), 7.78 (t, J = 7.93 Hz, 1H), 7.62 (d, J = 8.21 Hz, 1H), 7.51 (t, J = 7.42 Hz, 1H), 7.36 - 7.20 (m, 4H), 6.99 - 6.80 (m, 5H), 4.83 (m, 1H), 4.47 (t, J = 6.30 Hz, 2H), 4.27 (t, J = 5.08 Hz, 2H), 3.15 - 3.00 (m, 4H), 1.32 (t, J = 7.10 Hz, 3H).

## Example 43

**(±)-Ethyl 2-phenoxy-3-[4-[2-[2-methyl-4-oxo-3,4-dihydro-3-quinazolinyl]ethoxy]phenyl] propanoate**

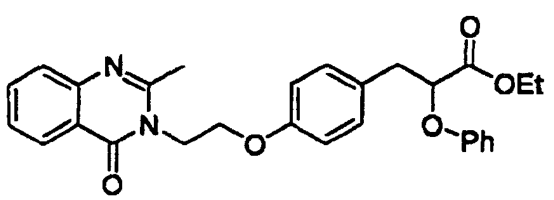

The title compound (950 mg, 42%) was obtained from 2-methyl-4-oxo-3,4-dihydroquinazoline (760 mg, 4.77 mmol), ethyl 2-phenoxy-3-[4-(2-bromoethoxy) phenyl]propanoate (2250 mg, 5.72 mmol) and potassium carbonate (1.32 g, 9.55 mmol) as a base by a similar procedure to that described in Example 25.

mp: 98-00°C.

$^1$H NMR ($CDCl_3$): δ 8.23 (d, J = 8.12 Hz, 1H), 7.78 - 7.58 (m, 2H), 7.43 (t, J = 7.35 Hz, 1H), 7.30 - 7.15 (m, 4H), 6.99 - 6.72 (m, 5H), 4.69 (t, J= 6.43 Hz, 1H), 4.51 (t, J = 4.82 Hz, 2H), 4.30 (t, J = 4.82 Hz, 2H), 4.15 (q, J = 6.09 Hz, 2H), 3.14 (d, J = 6.64 Hz, 2H), 2.08 (s, 3H), 1.69 (t, J = 7.08 Hz, 3H).

**Example 44**

**(±)-2-Phenoxy-3-[4-[2-[2-methyl-4-oxo-3,4-dihydro-3-quinazolinyl] ethoxy] phenyl]propanoic acid**

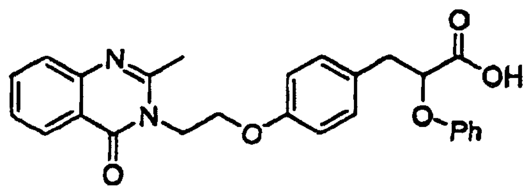

The title compound (90 mg, 64%) was obtained from (±)-ethyl 2-phenoxy-3-[4-[2-[2-methyl-4-oxo-3,4-dihydro-3-quinazolinyl]ethoxy]phenyl]propanoate (150 mg, 0.3 mmol) obtained in Example 43 and sodium carbonate (168 mg, 1.5 mmol) by a similar procedure to that described in Example 2.

mp: 206-210°C.

$^1H$ NMR ($CDCl_3$): δ 8.22 (d, J = 7.89 Hz, 1H), 7.72 (t, J = 6.89 Hz, 1H), 7.60 (d, J = 7.89 Hz, 1H), 7.43 (t, J = 7.36 Hz, 1H), 7.27 - 7.11 (m, 4H), 6.94 - 6.71 (m, 5H), 4.67 (t, J = 6.29 Hz, 1H), 4.51 (t, J = 4.9 Hz, 2H), 4.30 (t, J = 4.93 Hz, 2H), 3.17 (d, J = 5.82 Hz, 2H), 2.80 (s, 3H).

**Example 45**

**(±)-Ethyl 2-ethoxy-3-[4-[2-[2-ethyl-4-methyl-6-oxo-1-pyrimidinyl] ethoxy] phenyl]propanoate**

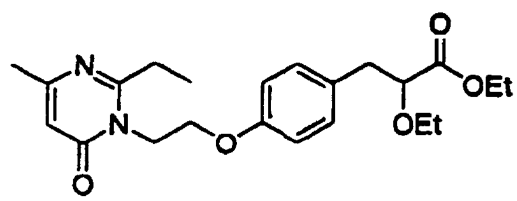

The title compound (430 mg, 59%) was obtained as a liquid from (±)-2-ethyl-4-methyl-6-pyrimidone (250 mg, 1.81 mmol) and ethyl 2-ethoxy-3-[4-(2-bromo-ethoxy)phenyl]propanoate (750 mg, 2.17 mmol) (disclosed in U.S. Patent Application Serial No. 09/012,585 now U.S. Patent 6,054,453), sodium hydride (44 mg, 1.9 mmol, 95 %) as a base, by a similar procedure to that described in Example 25.

$^1H$ NMR ($CDCl_3$): δ 7.16 (d, J = 8.62 Hz, 2H), 6.86 (d, J = 8.62 Hz, 2H), 6.43 (s, 1H), 4.70 (t, J = 4.77 Hz, 2H), 4.28 (t, J = 4.77 Hz, 2H), 4.17 (q, J = 7.11 Hz, 2H), 3.96 (t, J = 6.55 Hz, 1H), 3.70 - 3.50 (m, 1H), 3.42 - 3.22 (m, 1H), 2.95 (d, J = 6.55 Hz, 2H), 2.83 (q, J = 7.60 Hz, 2H), 2.40 (s, 3H), 1.32 (t, J = 7.60 Hz, 3H), 1.23 (t, J = 7.11 Hz, 3H), 1.63 (q, J = 6.90 Hz, 3H).

**Example 46**

**(±)-2-Ethoxy-3-[4-[2-[2-ethyl-4-methyl-6-oxo-1-pyrimidinyl]ethoxy]phenyl] propanoic acid**

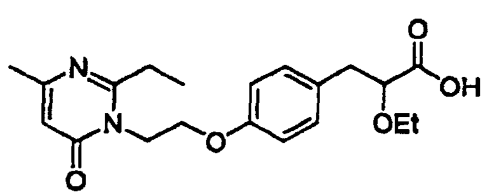

The title compound (100 mg, 50%) was obtained from (±)-ethyl 2-ethoxy-3-[4-[2-[2-ethyl-4-methyl-6-oxo-1-pyrimidinyl]ethoxy]phenyl]propanoate (215 mg, 0.53 mmol) obtained in Example 45 and sodium carbonate (265 mg, 2.5 mmol) by a similar procedure to that described in Example 2.

mp: 100-103°C.

$^1$H NMR ($CDCl_3$): δ 7.19 (d, J = 8.62 Hz, 2H), 6.88 (d, J = 8.62 Hz, 2H), 6.45 (s, 1H), 4.73 (t, J = 4.79 Hz, 2H), 4.30 (t, J = 4.79 Hz, 2H), 4.06 (dd, J = 7.28,4.56 Hz, 1H), 3.70 - 3.40 (m, 2H), 3.11 (dd, J = 14.16,4.56 Hz, 1H), 2.97 (dd, J = 14.16 and 7.28 Hz, 1H), 2.85 (q, J = 7.58 Hz, 2H), 2.42 (s, 3H), 1.33 (t, J = 7.58 Hz, 3H), 1.20 (t, J = 7.01 Hz, 3 H).

## Example 47

**(±)-Ethyl 2-ethoxy-3-[4-[[3-phenyl-4-oxo-3,4-dihydro-2-quinazolinyl]methoxy] phenyl]propanoate**

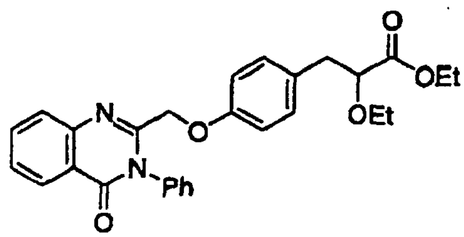

The title compound (420 mg, 87.5%) was obtained as a liquid from (±)-ethyl 2-ethoxy-3-(4-hydroxyphenyl) propanoate (220 mg, 0.92 mmol) (disclosed in U.S. Patent Application Serial No. 09/012,585 now U.S. Patent 6,054,453), 2-chloromethyl-3-phenyl-4-oxo-3,4-dihydroquinazoline (275 mg, 1.01 mmol) and potassium carbonate (383 mg, 2.77 mmol) as a base by a similar procedure to that described in Example 1.

$^1$H NMR ($CDCl_3$) δ 8.30 (d, J = 7.89 Hz, 1H), 7.79 (d, J = 7.83 Hz, 1H), 7.41 - 7.57 (m, 5H), 7.36 (d, J = 7.56 Hz, 2H), 7.07 (d, J = 8.40 Hz, 2H), 6.68 (d, J = 8.40 Hz, 2H), 4.74 (s, 2H), 4.12 (q, J = 7.08 Hz, 2H), 3.93 (t, J= 6.53 Hz, 1H), 3.50 - 3.68 (m, 1H), 3.22 - 3.40 (m, 1H), 2.90 (d, J = 6.65 Hz, 2H), 1.10 - 1.29 (m, 6H).

## Example 48

**(±)-2-Ethoxy-3-[4-[[3-phenyl-4-oxo-3,4-dihydro-2-quinazolinyl]methoxy]phenyl] propanoic acid**

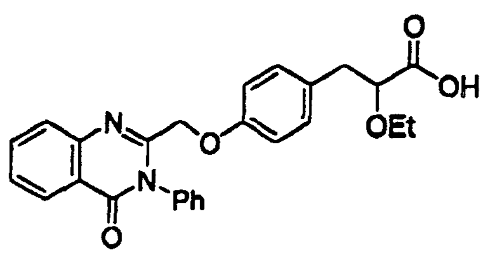

The title compound (120 mg, 58%) was obtained from (±)-ethyl 2-ethoxy-3-[4-[[3-phenyl-4-oxo-3,4-dihydro-2-quinazolinyl]methoxy]phenyl]propanoate (220 mg, 0.466 mmol) obtained in Example 47 and sodium carbonate (247 mg, 2.33 mmol) by a similar procedure to that described in Example 2.

mp: 173 °C.

$^1$H NMR (DMSO-$d_6$) : δ 8.47 (d, J = 8.21 Hz, 1H), 7.87 (d, J = 7.47 Hz, 1H), 7.76 (d, J = 8.39 Hz, 2H), 7.59 (t, J = 7.68 Hz, 1H), 7.41 (t, J = 7.82 Hz, 1H), 7.34 - 7.11 (m, SH), 6.98 (d, J = 8.39 Hz, 2H), 4.69 (s, 2H), 3.94 (dd, J = 5.12 and 7.42 Hz, 1H), 3.58 - 3.40 (m, 1H), 3.39 - 3.20 (m, 1H), 2.98 - 2.76 (m, 2H), 1.03 (t, J = 7.01 Hz, 3H).

**Example 49**

**(±)-Ethyl 2-ethoxy-3-[4-[[3-methyl-4-oxo-3,4-dihydro-6,7-dimethoxy-2-quinazolinyl]methoxy]phenyl] propanoate**

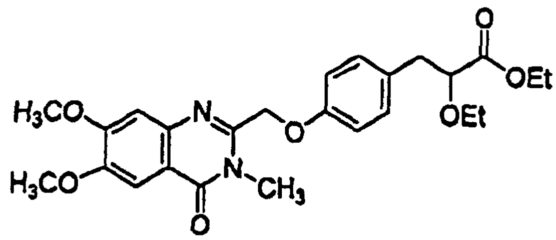

The title compound (420 mg, 79.9%) was obtained as a liquid from (±)-ethyl 2-ethoxy-3-(4-hydroxyphenyl) propanoate (292 mg, 1.23 mmol) (disclosed in U.S. Patent Application Serial No. 09/012,585 now U.S. Patent 6,054,453), 2-chloromethyl-3-methyl-4-oxo-3,4-dihydro-6,7-dimethoxy quinazoline (300 mg, 1.12 mmol) and potassium carbonate (464 mg, 3.36 mmol) as a base by a similar procedure to that described in Example 1.

$^1$H NMR ($CDCl_3$) : δ 7.60 (s, 1H), 7.19 (d, J = 8.63 Hz, 2H), 7.11 (s, 1H), 6.96 (d, J = 8.63 Hz, 2H), 5.12 (s, 2H), 4.15 (q, J = 7.13 Hz, 2H), 4.00 ( S,6H ) 4.01- 3.91 (m, 1H), 3.73 (s, 3H), 3.70 - 3.51 (m, 1H), 3.41 - 3.24 (m, 1H), 2.95 (d, J = 6.64 Hz, 2H), 1.28 - 1.10 (m, 6H).

**Example 50**

**(±)- 2-Ethoxy-3-[4-[[3-methyl-4-oxo-3,4-dihydro-6,7-dimethoxy-2-quinazolinyl]methoxy] phenyl]propanoic acid**

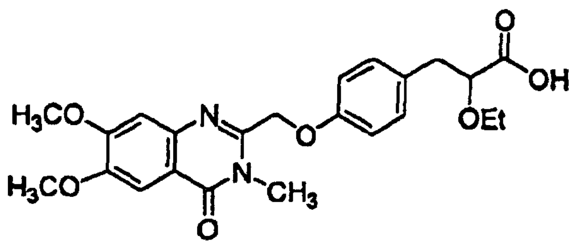

The title compound (300 mg, 79.7%) was obtained from (±)-ethyl 2-ethoxy-3-[4-[[3-methyl-4-oxo-3,4-dihydro-6,7-dimethoxy-2-quinazolinyl]methoxy] phenyl] propanoate (400 mg, 0.85 mmol) obtained in Example 49 and sodium carbonate (451 mg, 4.25 mmol) by a similar procedure to that described in Example 2.

mp: 187°C.

$^1$H NMR ($CDCl_3$): δ 7.61 (s, 1H), 7.19 (d, J = 8.62 Hz, 2H), 7.12 (s, 1H), 6.97 (d, J = 8.62 Hz, 2H), 5.13 (s, 2H), 4.11 - 3.94 (m, 7H), 3.73 (s, 3H), 3.69 - 3.53 (m, 1H), 3.53 - 3.40 (m, 1H), 3.13 - 2.89 (m, 2H), 1.18 (t, J = 6.94 Hz, 3H).

**Example 51**

**(±)-Ethyl 2-ethoxy-3-[4-[[3-(4-methylphenyl)-4-oxo-3,4-dihydro-2-quinazolinyl]methoxy]phenyl]propanoate**

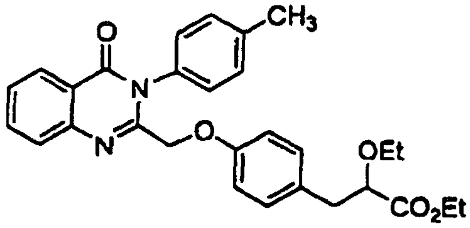

The title compound (310 mg, 60.5%) was obtained from (±)-ethyl 2-ethoxy-3-(4-hydroxy phenyl)propanoate (276 mg, 1.16 mol) (described in U.S. Patent Application Serial No. 09/012,585 now U.S. Patent 6,054,453), 2-chloromethyl-3-(4-methylphenyl)-4-oxo-3,4-dihydroquinazoline (300 mg, 1.05 mmol) and potassium carbonate (435 mg, 3.16 mmol) as a base by a similar procedure to that described in Example 1.

mp: 81°C.

$^1$H NMR ($CDCl_3$): δ 8.30 (d, J = 7.89 Hz, 1H), 7.79 (d, J = 3.73 Hz, 1H), 7.52 (t, J = 6.09 Hz, 1H), 7.32 - 7.18 (m, 5H), 7.09 (d, J = 8.3 Hz, 2H), 6.72 (d, J = 8.30 Hz, 2H), 4.75 (s, 2H), 4.13(q, J = 7.09 Hz, 2H), 3.93 (t, J = 9.94 Hz, 1H), 3.69 - 3.50 (m, 1H), 3.40 - 3.24 (m, 1H), 2.91 (d, J = 6.41 Hz, 2H), 2.40 (s, 3H), 1.25 - 1.10 (m, 6H).

**Example 52**

**(±)-2-Ethoxy-3-[4-[[3-(4-methylphenyl)-4-oxo-3,4-dihydro-2-quinazolinyl] methoxy]phenyl]propanoic acid**

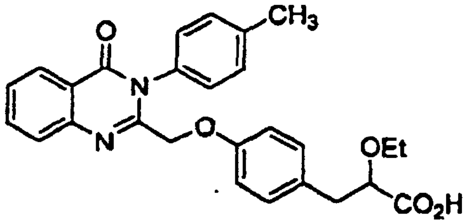

The title compound (85 mg, 69%) was obtained from (±)-ethyl 2-ethoxy-3-[4-[[3-(4-methylphenyl)-4-oxo-3,4-dihydro-2-quinazolinyl]methoxy]phenyl]propanoate (130 mg, 0.267 mmol) obtained in Example 51 and sodium carbonate (142 mg, 1.33 mmol) by a similar procedure to that described in Example 2.

mp: 178°C.

$^1$H NMR ($CDCl_3$): δ 8.30 (d, J = 7.89 Hz, 1H), 7.79 (d, J = 3.5 Hz, 1H), 7.54 (t, J = 5.97 Hz, 1H), 7.47 - 6.90 (m, 7H), 6.72 (d, J = 8.62 Hz, 2H), 4.74 (s, 2H), 4.01 (dd, J = 4.26 and 7.16 Hz, 1H), 3.64 - 3.30 (m, 2H), 3.09 - 2.88 (m, 2H), 2.39 (s, 3H), 1.12 (t, J = 5.65 Hz, 3H).

**Example 53**

**(±)-Ethyl 2-ethoxy-3-[4-[[3-(4-methoxyphenyl)-4-oxo-3,4-dihydro-2-quinazolinyl]methoxy] phenyl]propanoate**

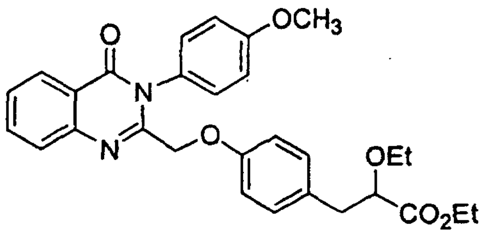

The title compound (350 mg, 60%) was obtained as a liquid from ethyl 2-ethoxy-3-(4-hydroxy phenyl)propanoate (305 mg, 1.28 mmol) (described in U.S. Patent Application Serial No. 09/012,585 now U.S. Patent 6,054,43), 2-chloromethyl-3-(4-methoxyphenyl)-4-oxo-3,4-dihydroquinazoline (350 mg, 1.16 mmol) and potassium carbonate (482 mg, 3.49 mmol) as a base by a similar procedure to that described in Example 1.

$^1$H NMR ($CDCl_3$): δ 8.30 (d, J = 7.89 Hz, 1H), 7.78 (d, J = 3.73 Hz, 1H), 7.53 (t, J = 6.22 Hz, 2H), 7.23 (d, J = 8.62 Hz, 2H), 7.09 (d, J = 8.21 Hz, 2H), 7.01 (d, J = 8.72 Hz, 2H), 6.72 (d, J = 8.62 Hz, 2H), 4.75 (s, 2H), 4.15 (q, J = 7.13 Hz, 2H), 3.93 (t, J = 6.65 Hz, 1H), 3.82 (s, 3H), 3.64 - 3.50 (m, 1H), 3.40 - 3.22 (m, 1H), 2.91 (d, J = 6.64 Hz, 2H), 1.25 (t, J = 7.10 Hz, 3H), 1.13 (t, J = 7.40 Hz, 3H).

**Example 54**

**(±)-2-Ethoxy-3-[4-[[3-(4-methoxyphenyl)-4-oxo-3,4-dihydro-2-quinazolinyl] methoxy]phenyl]propanoic acid**

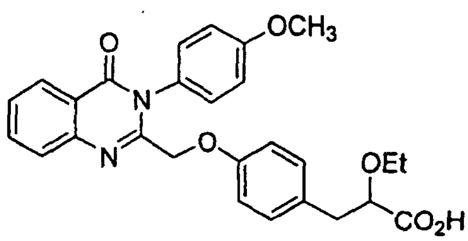

The title compound (200 mg, 78.4%) was obtained from (±)-ethyl 2-ethoxy-3-[4-[[3-(4-methoxy phenyl)-4-oxo-3,4-dihydro-2-quinazolinyl]methoxy] phenyl] propanoate (270 mg, 0.537 mmol) obtained in example 53 and sodium carbonate (285 mg, 2.68 mmol) by a similar procedure to that described in Example 2.

mp: 171°C.

$^1$H NMR ($CDCl_3$): δ 8.32 (d, J = 7.89 Hz, 1H), 7.81 (d, J = 3.41 Hz, 2H), 7.64 - 7.50 (m, 1H), 7.25 (d, J = 8.53 Hz, 2H), 7.29 - 6.92 (m, 4H), 6.74 (d, J = 8.53 Hz, 2H), 4.77 (s, 2H), 4.03 (dd, J = 4.19 and 7.00 Hz, 1H), 3.85 (s, 3H), 3.64 - 3.56 (m, 1H), 3.48 - 3.40 (m, 1H), 3.10 - 2.84 (m, 2H), 1.17 (t, J = 7.01 Hz, 3H).

**Example 55**

**(±)-Ethyl 2-ethoxy-3-[4-[[3-benzyl-4-oxo-3,4-dihydro-2-quinazolinyl] methoxy]phenyl] propanoate**

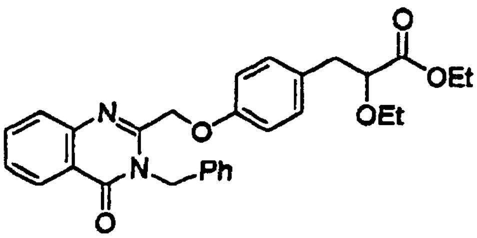

The title compound (450 mg, 75%) was obtained as a liquid from (±)-ethyl 2-ethoxy-3-(4-hydroxyphenyl)propanoate (322 mg, 1.35 mmol) (disclosed in U.S. Patent Application Serial No. 09/012,585 now U.S. Patent 6,054,453), 2-chloromethyl-3-benzyl-4-oxo-3,4-dihydro-quinazoline (350 mg, 1.23 mmol) and potassium carbonate (509 mg, 3.69 mmol) as a base by a similar procedure to that described in Example 1.

$^1$H NMR ($CDCl_3$): δ 8.35 (d, J = 7.89 Hz, 1H), 7.85 - 7.70 (m, 2H), 7.54 (t, J = 6.36 Hz, 1H), 7.36 - 7.10 (m, 7H), 6.87 (d, J = 8.63 Hz, 2H), 5.59 (s, 2H), 5.00 (s, 2H), 4.15 (q, J = 7.08 Hz, 2H), 3.96 (t, J = 6.57 Hz, 1H), 3.69 - 3.50 (m, 1H), 3.41 - 3.25 (m, 1H), 2.94 (d, J = 6.32 Hz, 2H), 1.29 - 1.11 (m, 6H).

**Example 56**

**(±)-2-Ethoxy-3-[4-[[3-benzyl-4-oxo-3,4-dihydro-2-quinazolinyl]methoxy] phenyl]propanoic acid**

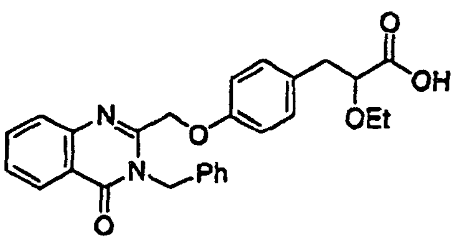

The title compound (280 mg, 80%) was obtained from (±)-ethyl 2-ethoxy-3-[4-[[3-benzyl-4-oxo-3,4-dihydro-2-quinazolinyl]methoxy]phenyl]propanoate (370 mg, 0.76 mmol) obtained in Example 55 and sodium carbonate (403 mg, 3.8 mmol) by a similar procedure to that described in Example 2.

mp: 160°C.

$^1$H NMR ($CDCl_3$): δ 8.35 (d, J = 8.31 Hz, 1H), 7.81 - 7.70 (m, 2H), 7.54 (t, J = 6.43 Hz, 1H), 7.38 - 7.10 (m, 7H), 6.88 (d, J = 8.53 Hz, 2H), 5.59 (s, 2H), 5.01 (s, 2H), 4.05 (dd, J = 4.15 and 7.21 Hz, 1H), 3.66 - 3.39 (m, 2H), 3.09 (dd, J = 4.15 and 14.21 Hz, 1H), 2.94 (dd, J = 7.21 and 14.21 Hz, 1H), 1.17 (t, J = 7.05 Hz, 3H)

**Example 57**

**(±)-Ethyl 2-ethoxy-3-[4-[[3-(3-chlorophenyl)-4-oxo-3,4-dihydro-2-quinazolinyl] methoxy]phenyl]propanoate**

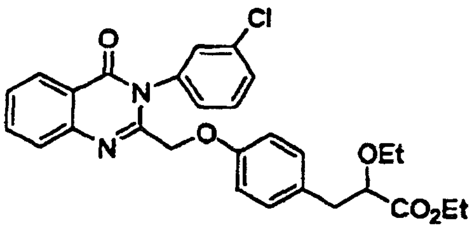

The title compound (300 mg, 49%) was obtained as a liquid from (±)-ethyl 2-ethoxy-3-(4-hydroxyphenyl)propanoate (288 mg, 1.21 mmol), 2-chloromethyl-3-(3-chlorophenyl)-4-oxo-3,4-dihydroquinazoline (370 mg, 1.21 mmol) and potassium carbonate (502 mg, 3.63 mmol) as a base by a similar procedure to that described in Example 1.

$^1H$ NMR ($CDCl_3$): δ 8.31 (d, J = 7.88 Hz, 1H), 7.90 - 7.78 (m, 2H), 7.61 - 7.20 (m, 5H), 7.10 (d, J = 8.63 Hz, 2H), 6.71 (d, J = 8.63 Hz, 2H), 4.85 (s, 2H), 4.15 (q, J = 7.07 Hz, 2H), 3.94 (t, J = 6.64 Hz, 1H), 3.70 - 3.51 (m, 1H), 3.41 - 3.25 (m, 1H), 2.92 (d, J = 6.55 Hz, 2H), 1.28 - 1.10 (m, 6H).

**Example 58**

**(±)-2-Ethoxy-3-[4-[[3-(3-chlorophenyl)-4-oxo-3,4-dihydro-2-quinazolinyl] methoxy]phenyl]propanoic acid**

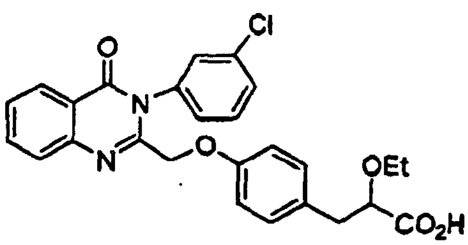

The title compound (125 mg, 66%) was obtained from (±)-ethyl 2-ethoxy-3-[4-[[3-(3-chlorophenyl)-4-oxo-3,4-dihydro-2-quinazolinyl]methoxy]phenyl]propanoate (200 mg, 0.39 mmol) obtained in Example 57 and sodium carbonate (209 mg, 1.97 mmol) by a similar procedure to that described in Example 2.

mp: 157°C.

$^1H$ NMR ($CDCl_3$): δ 8.52 (d, J = 8.31 Hz, 1H), 8.30 (bs, 1H), 7.90 - 7.79 (m, 1H), 7.62 - 6.92 (m, 8H), 6.35 (d, J = 8.3 Hz, 1H), 4.59 (s, 2H), 4.03 (dd, J = 4.47 and 7.05 Hz, 1H), 3.62 - 3.31 (m, 2H), 3.12 - 2.82 (m, 2H), 1.18 (t, J = 3.41 Hz, 3H).

**Example 59**

**(±)-Ethyl 2-ethoxy-3-[4-[[3-(3-chloro-4-fluorophenyl)-4-oxo-3,4-dihydro-2-quinazolinyl] methoxy]phenyl] propanoate**

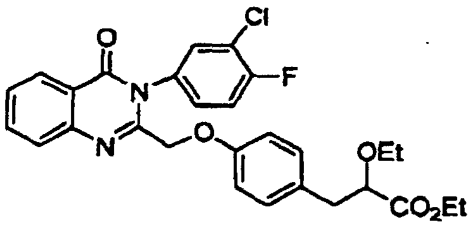

The title compound (250 mg, 57%) was obtained as a liquid from (±)-ethyl 2-ethoxy-3-(4-hydroxyphenyl)propanoate (218 mg, 0.919 mmol) (disclosed in U.S. Patent Application Serial No. 09/012,585 now U.S. Parent 6,054,453), 2-chlommethyl-3-(3-chloro-4-fluoro-phenyl)-4-oxo-3,4-dihydroquinazoline (270 mg, 0.835 mmol) and potassium carbonate (380 mg, 2.5 mmol) as a base by a similar procedure to that described in Example 1.

$^1H$ NMR ($CDCl_3$): δ 8.29 (d, J = 7.98 Hz, 1H), 7.77 - 7.83 (m, 2H), 7.50 - 7.60 (m, 1H), 7.44 (d, J = 5.31 Hz, 1H), 7.23 (d, J = 6.32 Hz, 2H), 7.11 (d, J = 8.62 Hz, 2H), 6.71 (d, J = 8.49 Hz, 2H), 4.80 (s, 2H), 4.12 (q, J = 4.75 Hz, 2H), 3.93 (t, J = 6.60 Hz, 1H), 3.50 - 3.68 (m, 1H), 3.24 - 3.41 (m, 1H), 2.91 (d, J = 6.64 Hz, 2H), 1.10 - 1.28 (m, 6H).

**Example 60**

**(±)-2-Ethoxy-3-[4-[[3-(3-chloro-4-fluorophenyl)-4-oxo-3,4-dihydro-2-quinazolinyl]methoxy]phenyl]propanoic acid**

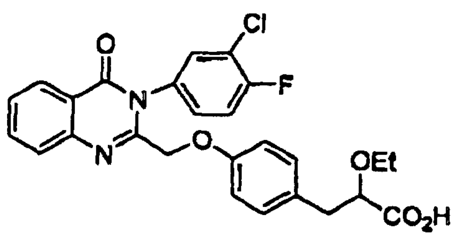

The title compound (85 mg, 50%) was obtained from (±)-ethyl 2-ethoxy-3-[4-[[3-(3-chloro-4-fluorophenyl)-4-oxo-3,4-dihydro-2-quinazolinyl]methoxy]phenyl] propanoate (180 mg, 0.343 mmol) obtained in Example 59 and sodium carbonate (181 mg, 1.71 mmol) by a similar procedure to that described in Example 2.

mp: 175°C.

$^1H$ NMR ($CDCl_3$): δ 8.60 (d, J = 8.07 Hz, 1H), 8.00 (d, J = 4.48 Hz, 1H), 7.70 (d, J = 7.89 Hz, 2H), 7.80 - 7.09 (m, 5H), 6.98 (d, J = 8.39 Hz, 2H), 4.60 (s, 2H), 3.96 (dd, J = 4.61 and 7.43 Hz, 1H), 3.70 - 3.52 (m, 1H), 3.41 - 3.24 (m, 1H), 3.08 - 2.84 (m, 2H), 1.15 (t, J = 6.85 Hz, 3H).

**Example 61**

**(±)-Ethyl 2-ethoxy-3-[4-[2-[N-(2-propanamido) benzoyl]aminoethoxy] phenyl]propanoate**

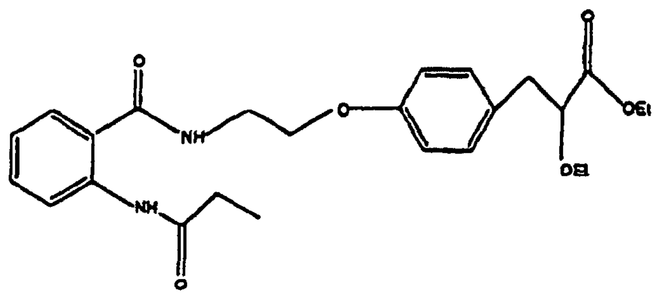

To a stirred solution of (±)-Ethyl 2-ethoxy-3-[4-[2-N-(2-aminobenzoyl) aminoethoxy]phenyl] propanoate (1.5 g, 3.75 mmol) obtained in preparation 6 in a mixture of xylene (5ml) and propanoic acid (5 ml) was added triethylamine (1.04 ml, 0.75 g, 7.5 mmol) followed by addition of propanoyl chloride (0.36 ml, 0.388 g, 4.1 mmol) at *ca* 30°C and stirred 2h. Water was added to the reaction mixture and extracted with ethyl acetate (3 x 10 ml). The combined organic extracts were washed with brine, dried over anhydrous $Na_2SO_4$ and concentrated. The crude compound was chromatographed on silica gel using 20% EtoAC/petroleum ether as eluent to afford the title compound (1.18g, 72%).

$^1H$ NMR ($CDCl_3$): δ 8.64 (d, J = 8.63 Hz, 1H), 7.51 - 7.42 (m 2H), 7.18 (d, J = 8.40 Hz,2H), 7.07 (t, J = 7.68 Hz, 1H), 6.84 (d, J = 8.40 Hz, 2H), 4.25 - 4.08 (m, 4H), 3.97 (t, J = 6.57 Hz, 1H), 3.88 - 3.80 (m, 2H), 3.70 - 3.52 (m, 1H), 3.42 - 3.26 (m, 1H), 2.96 (d, J = 6.32 Hz, 2H), 2.45 (q, J = 7.53Hz, 2H), 1.32 - 1.10 (m,9H).

**Example 62**

**(±)-Ethyl 2-ethoxy-3-[4-[2-[N-(2 -trifluoroacetamido) benzoyl]aminoethoxy] phenyl]propanoate**

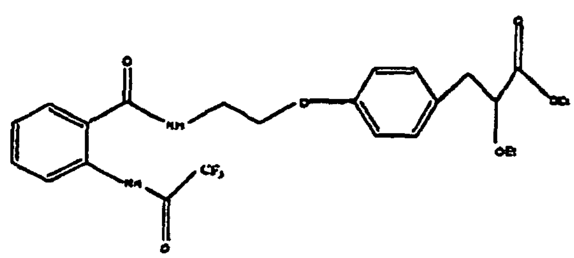

The title compound (456 mg, 92%) was obtained from (±)-Ethyl 2-ethoxy-3-[4-[2-N-(2-aminobenzoyl)aminoethoxy]phenyl] propanoate (400mg, 1.0 mmol) obtained in preparation 6 and trifluoroacetic anhydride (314 mg, 1.5 mmol) by a similar procedure described in Example 61.

mp: 70-72°C.

$^1H$ NMR ($CDCl_3$): δ 12.72 (bs, $D_2O$ exchangeable, 1H), 8.60 (d, J = 8.30 Hz, 0.5H), 7.68 -7.42 (m, 2H), 7.30 - 7.12 (m, 0.5H), 7.00 - 6.80 (m, 1H), 7.16 (d, J = 8.40 Hz, 2H), 6.83 (d, J = 8.40 Hz, 2H), 4.25 - 4.02 (m, 4H), 3.96 (t, J = 6.55 Hz, I H), 3.86 (q, J = 5.10 Hz, 2H), 3.70 - 3.42 (m, 1H), 3.42 - 3.20 (m, 1H), 2.95 (d, J = 6.55 Hz, 2H), 1.28 - 1.02 (m, 6H).

The compounds of the present invention lowered random blood sugar level, triglyceride, total cholesterol, LDL, VLDL and increased HDL. This was demonstrated by *in vitro* as well as *in vivo* animal experiments.

Demonstration of Efficacy of Compounds

***A) In vitro***

**a) Determination of hPPARα activity**

Ligand binding domain of hPPARα was fused to DNA binding domain of Yeast transcription factor GAL4 in eucaryotic expression vector. Using superfect (Qiagen, Germany) as transfecting reagent HEK-293 cells were transfected with this plasmid and a reporter plasmid harboring the luciferase gene driven by a GAL4 specific promoter. Compound was added at different concentrations after 42 hrs of

transfection and incubated overnight. Luciferase activity as a function of compound binding/activation capacity of PPARα was measured using Packard Luclite kit (Packard, USA) in Top Count (Ivan Sadowsld, Brendan Bell, Peter Broag and Melvyn Hollis. Gene. 1992. 118: 137 -141; Superfect Transfection Reagent Handbook. February, 1997. Qiagen, Germany).

**b) Determination of hPPARγ activity**

Ligand binding domain of hPPARγ1 was fused to DNA binding domain of Yeast transcription factor GAL4 in eucaryotic expression vector. Using lipofectamine (Gibco BRL, USA) as transfecting reagent HEK-293 cells were transfected with this plasmid and a reporter plasmid harboring the luciferase gene driven by a GAL4 specific promoter, Compound was added at 1 μM concentration after 48 hrs of transfection and incubated overnight. Luciferase activity as a function of drug binding/activation capacity of PPARγ1 was measured using Packard Luclite kit (Packard, USA) in Packard Top Count (Ivan Sadowski, Brendan Bell, Peter Broag and Melvyn Hollis. Gene. 1992. 118:137-141; Guide to Eukaryotic Transfections with Cationic Lipid Reagents. Life Technologies, GIBCO BRL, USA).

| Example No | Concentration | PPARα | Concentration | PPARγ |
|---|---|---|---|---|
| Example 25 | 50 μM | 5 | 1 μM | 19 |
| Example 18 | 50 μM | 5 | 1 μM | 3 |

**c) Determination of HMG CoA reductase inhibition activity**

Liver microsome bound reductase was prepared from 2% cholestyramine fed rats at mid-dark cycle. Spectrophotometric assays were carried out in 100 mM $KH_2PO_4$, 4 mM DTT, 0.2 mM NADPH, 0.3 mM HMG CoA and 125 μg of liver microsomal enzyme. Total reaction mixture volume was kept as I ml. Reaction was started by addition of HMG CoA. Reaction mixture was incubated at 37 °C for 30 min and decrease in absorbance at 340 nm was recorded. Reaction mixture without substrate was used as blank (Goldstein, J. L and Brown, M. S. Progress in under-standing the LDL receptor and HMG CoA reductase, two membrane proteins that regulate the plasma cholesterol. J. Lipid Res. 1984,25: 1450 - 1461). The test compounds inhibited the HMG CoA reductase enzyme.

***B) In vivo***

**a) Efficacy in genetic models**

Mutation in colonies of laboratory animals and different sensitivities to dietary regimens have made the development of animal models with non-insulin dependent diabetes and hyperlipidemia associated with obesity and insulin resistance possible. Genetic models such as db/db and ob/ob (Diabetes, (1982) 31(1): 1- 6) mice and zucker fa/fa rats have been developed by the various laboratories for understanding the pathophysiology of disease and testing the efficacy of new antidiabetic compounds (Diabetes, (1983) 32: 830-838; Annu. Rep. Sankyo Res. Lab. (1994). 46 : 1-57). The homozygous animals, C57 BL/KsJ-db/db mice developed by Jackson Laboratory, US, are obese, hyperglycemic, hyperinsulinemic and insulin resistant (J. Clin. Invest., (1990) 85 : 962-967), whereas heterozygous are lean and normoglycemic. In db/db model, mouse progressively develops insulinopenia with age, a feature commonly observed in late stages of human type II diabetes when blood sugar levels are insufficiently controlled. The state of pancreas and its course vary according to the models. Since this model resembles that of type II diabetes mellitus, the compounds of the present invention were tested for blood sugar and triglycerides lowering activities.

Male C57BL/KsJ-db/db mice of 8 to 14 weeks age, having body weight range of 35 to 60 grams, bred at Dr. Reddy's Research Foundation (DRF) animal house, were used in the experiment. The mice were provided with standard

feed (National Institute of Nutrition (NIN), Hyderabad, India) and acidified water, ad libitum. The animals having more than 350 mg / dl blood sugar were used for testing. The number of animals in each group was 4.

Test compounds were suspended on 0.25 % carboxymethyl cellulose and administered to test group at a dose of 0.001 mg to 30 mg / kg through oral gavage daily for 6 days. The control group received vehicle (dose 10 ml / kg). On 6th day the blood samples were collected one hour after administration of test compounds / vehicle for assessing the biological activity.

The random blood sugar and triglyceride levels were measured by collecting blood (100 μl) through orbital sinus, using heparinised capillary in tubes containing EDTA which was centrifuged to obtain plasma. The plasma glucose and triglyceride levels were measured spectrometrically, by glucose oxidase and glycerol-3-$PO_4$ oxidase/peroxidase enzyme (Dr. Reddy's Lab. Diagnostic Division Kits, Hyderabad, India) methods respectively.

The blood sugar and triglycerides lowering activities of the test compound was calculated according to the formula.

No adverse effects were observed for any of the mentioned compounds of invention in the above test.

| Compound | Dose (mg / kg) | Reduction in Blood Glucose Level (%) | Triglyceride Lowering (%) |
|---|---|---|---|
| Example 42 | 3 mg | 52 | 31 |
| Example 13 | 3 mg | 72 | 69 |
| Example 16 | 3 mg | 49 | 40 |
| Example 46 | 3 mg | 52 | 19 |

The ob/ob mice were obtained at 5 weeks of age from Bomholtgard, Denmark and were used at 8 weeks of age. Zucker fa/fa fatty rats were obtained from IffaCredo, France at 10 weeks of age and were used at 13 weeks of age. The animals were maintained under 12 hour light and dark cycle at 25 ± 1 °C. Animals were given standard laboratory chow (NIN, Hyderabad, India) and water, *ad libitum* (Fujiwara, T., Yoshioka, S., Yoshioka, T., Ushiyama, I and Horikoshi, H. Characterization of new oral antidiabetic agent CS-045. Studies in KK and ob/ob mice and Zucker fatty rats. Diabetes. 1988. 37: 1549-1558).

The test compounds were administered at 0.1 to 30 mg/kg/day dose for 9 days. The control animals received the vehicle (0.25 % carboxymethylcellulose, dose 10 ml/kg) through oral gavage.

The blood samples were collected in fed state 1 hour after drug administration on 0 and 9 day of treatment The blood was collected from the retro-orbital sinus through heparinised capillary in EDTA containing tubes. After centrifugation, plasma sample was separated for triglyceride, glucose, free fatty acid, total cholesterol and insulin estimations. Measurement of plasma triglyceride, glucose, total cholesterol were done using commercial kits (Dr. Rcddy's Laboratory, Diagnostic Division kits, Hyderabad, India). The plasma free fatty acid was measured using a commercial kit form Boehringer Mannheim, Germany. The plasma insulin was measured using a RIA kit (BARC, India). The reduction of various parameters examined are calculated according to the formula.

In ob/ob mice oral glucose tolerance test was performed after 9 days treatment. Mice were fasted for 5 hrs and challenged with 3 gm/kg of glucose orally. The blood samples were collected at 0, 15, 30, 60 and 120 min for estimation of plasma glucose levels.

The experimental results from the db/db mice, ob/ob mice, Zucker fa/fa rats suggest that the novel compounds of the present invention also possess therapeutic utility as a prophylactic or regular treatment for diabetes, obesity, cardiovascular disorders such as hypertension, hyperlipidaemia and other diseases; as it is known from the literature that such diseases are interrelated to each other.

Blood glucose level and triglycerides are also lowered at doses greater than 10 mg/kg. Normally, the quantum of reduction is dose dependent and plateaus at certain dose.

**b) Cholesterol lowering activity in hypercholesterolemic rat models**

Male Sprague Dawley rats (NIN stock) were bred in DRF animal house. Animals were maintained under 12 hour light and dark cycle at 25 ± 1°C. Rats of 180 - 200 gram body weight range were used for the experiment. Animals were made hypercholesterolemic by feeding 2% cholesterol and 1% sodium cholate mixed with standard laboratory chow [National Institute of Nutrition (NIN), Hyderabad, India] for 6 days. Throughout the experimental period the animals were maintained on the same diet (Petit, D., Bonnefis, M. T., Rey, C and Infante, R. Effects of ciprofibrate on liver lipids and lipoprotein synthesis in normo- and hyperlipidemic rats. Atherosclerosis. 1988. 74: 215-225).

The test compounds were administered orally at a dose 0.1 to 30 mg/kg/day for 3 days. Control group was treated with vehicle alone (0.25 % Carboxy-ethylcellulose; dose 10 ml/kg).

The blood samples were collected in fed state 1 hour after drug administration on 0 and 3 day of compound treatment. The blood was collected from the retro-orbital sinus through hepariniscd capillary in EDTA containing tubes. After centrifugation, plasma sample was separated for total cholesterol, HDL and triglyceride estimations. Measurement of plasma triglyceride, total cholesterol and HDL were done using commercial kits (Dr. Reddy's Laboratory, Diagnostic Division, India). LDL and VLDL cholesterol were calculated from the data obtained for total cholesterol, HDL and triglyceride. The reduction of various parameters examined are calculated according to the formula.

| Compound | Dose mg/kg | Triglyceride (%)↓ | Total Cholesterol (%)↓ | HDL↑ (%) | LDL (%)↓ | VLDL(%)↓ |
|---|---|---|---|---|---|---|
| Example 6 | 3 mg | 45 | 11 | NE | 11 | 25 |
| Example 13 | 10 mg | 38 | 20 | 4 | 21 | 33 |
| ↓ = reduction; ↑= increase; NE = no effect | | | | | | |

c) **Plasma triglyceride and total cholesterol lowering activity in Swiss albino mice and Guinea pigs**

Male Swiss albino mice (SAM) and male Guinea pigs were obtained from NIN and housed in DRF animal house. All these animals were maintained under 12 hour light and dark cycle at 25 ± 1°C. Animals were given standard laboratory chow (NIN, Hyderabad, India) and water, *ad libitum*. SAM of 20 - 25 g body weight range and Guinea pigs of 500 - 700 g body weight range were used (Oliver, P., Plancke, M. O., Marzin, D., Clavey, V., Sauzieres, J and Fruchart, J. C. Effects of fenofibrate, gemfibrozil and nicotinic acid on plasma lipoprotein levels in normal and hyperlipidemic mice. Atherosclerosis. 1988. 70: 107-114).

The test compounds were administered orally to Swiss albino mice at 0.3 to 30 mg/kg/day dose for 6 days. Control mice were treated with vehicle (0.25% Carboxymethylcellulose; dose 10 ml/kg). The test compounds were administered orally to Guinea pigs at 0.3 to 30 mg/kg/day dose for 6 days. Control animals were treated with vehicle (0.25% Carboxymethylcellulose; dose 5 ml/kg).

The blood samples were collected in fed state I hour after drug administration on 0 and 6 day of treatment. The blood was collected from the retro-orbital sinus through heparinised capillary in EDTA containing tubes. After centrifugation, plasma sample was separated for triglyceride and total cholesterol (Wieland, O. Methods of Enzymatic analysis. Bergermeyer, H. O., Ed., 1963. 211 - 214; Trinder, P. Ann. Clin. Biochem. 1969. 6 : 24 - 27). Measurement of plasma triglyceride, total cholesterol and HDL were done using commercial kits (Dr. Reddy's Diagnostic Division, Hyderabad, India).

| Compound | Dose (mg / kg) | Triglyceride (%)↓ |
|---|---|---|
| Example 40 | 3 mg | 59 |
| Example 33 | 1 mg | 56 |
| Example 26 | 10 mg | 70 |
| Example 38 | 10 mg | 61 |
| Example 62 | 10mg | 57 |

**Formulae for calculation:**

1. Percent reduction in Blood sugar / triglycerides / total cholesterol were calculated according to the formula :

$$\text{Percent reduction (\%)} = \left[ 1 - \frac{TT/OT}{TC/OC} \right] \times 100$$

OC = Zero day control group value
OT = Zero day treated group value

TC = Test day control group value

TT = Test day treated group value

2. LDL and VLDL cholesterol levels were calculated according to the formula :

$$\text{LDL cholesterol in mg/dl} = [\text{Total cholesterol - HDL cholesterol} - \frac{\text{Triglyceride}}{5}]\ \text{mg/dl}$$

VLDL cholesterol in mg/dl=[Total cholesterol-HDL cholesterol-LDL cholesterol] mg/dl

## Claims

**1.** A compound of formula (I)

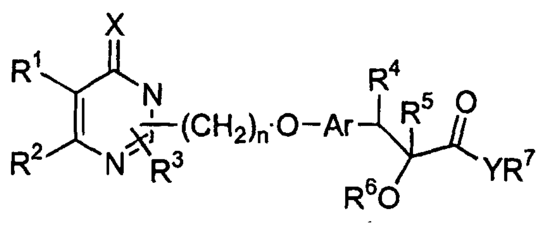

(I)

its tautomeric forms, its stereoisomers, its polymorphs, its pharmaceutically acceptable salts, of its pharmaceutically acceptable solvates, wherein X represents O or S; the groups $R^1$, $R^2$ and group $R^3$ when present on carbon atom, are the same or different and represent hydrogen, halogen, hydroxy, or unsubstituted or substituted groups selected from $(C_1\text{-}C_6)$ alkoxy, $(C_1\text{-}C_6)$alkyl, or phenyl, wherein when said groups are substituted the substituent (s) is selected from halogen or hydroxy; or $R^1$, $R^2$ along with the adjacent atoms to which they are attached form a substituted or unsubstituted phenyl group, wherein when the phenyl group formed by $R^1$ and $R^2$ is substituted, the substituent(s) is selected from halogen, hydroxy, $(C_1\text{-}C_3)$ alkyl or $(C_1\text{-}C_3)$alkoxy; $R^3$ when attached to nitrogen atom represents hydrogen, or unsubstituted or substituted groups selected from $(C_1\text{-}C_6)$alkyl, phenyl, naphthyl, benzyl or phenethyl, wherein when the group $R^3$ attached to nitrogen is substituted, the substituent is selected from halogen, hydroxy, methyl, ethyl, isopropyl, n-propyl, n-butyl or $(C_1\text{-}C_3)$alkoxy; the linking group represented by $-(CH_2)_n$-O- may be attached either through nitrogen atom or through carbon atom where n is an integer ranging from 1 - 4; Ar represents phenylene ; $R^4$ represents hydrogen atom, methyl, ethyl, hydroxy, $(C_1\text{-}C_3)$ alkoxy or forms a bond with $R^5$; $R^5$ represents hydrogen methyl, ethyl, hydroxy, $(C_1\text{-}C_3)$ alkoxy or forms a bond with $R^4$; $R^6$ represents hydrogen, $(C_1\text{-}C_{12})$alkyl, $(C_1\text{-}C_6)$alkoxy$(C_1\text{-}C_6)$alkyl, phenyl or benzyl, with a provision that $R^6$ does not represent hydrogen when $R^7$ represents hydrogen or $(C_1\text{-}C_{12})$ alkyl group; $R^7$ represents hydrogen or unsubstituted or substituted group selected from $(C_1\text{-}C_{12})$ alkyl or phenyl, wherein when $R^7$ is substituted the substituent(s) on $R^7$ is selected from halogen ; Y represents oxygen or $NR^8$, where $R^8$ represents hydrogen or $(C_1\text{-}C_{12})$alkyl ; or $R^7$ and $R^8$ together with the nitrogen atom to which they are attached form a 5 or 6 membered cyclic structure containing carbon atoms, a nitrogen atom and optionally an oxygen atom.

**2.** A process for the preparation of compound of formula (I)

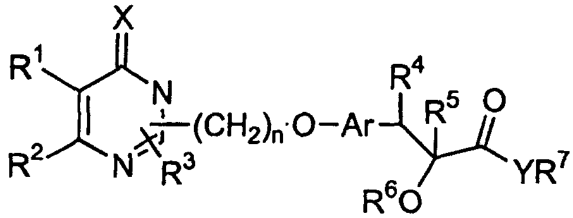

(I)

where X, $R^1$, $R^2$, $R^3$, $R^4$, $R^5$, $R^6$, Ar, n and $R^7$ are as defined in claim 1 and Y represents oxygen atom, which comprises:

a) reducing a compound of formula (IVa)

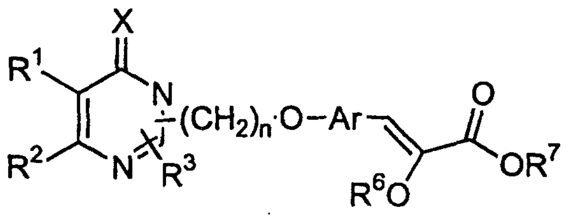

(IVa)

where all symbols are as defined earlier, to yield a compound of the formula (I) where $R^4$ and $R^5$ each represent hydrogen atom, and all other symbols are as defined above; or

b) reacting a compound of formula (IVb)

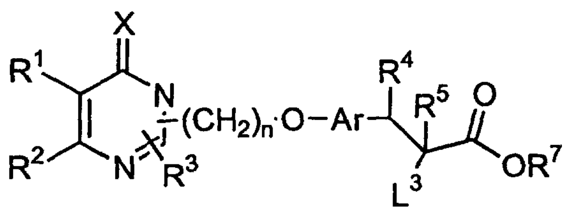

(IVb)

where all symbols are as defined above, $R^7$ is as defined above excluding hydrogen, and $L^3$ is a halogen atom, with an alcohol of formula (IVc),

$$R^6\text{-OH} \quad \text{(IVc)}$$

where $R^6$ represents $(C_1\text{-}C_{12})$alkyl, $(C_1\text{-}C_6)$alkoxy$(C_1\text{-}C_6)$alkyl, phenyl or benzyl, to produce a compound of the formula (I) defined above; or

c) reacting a compound of formula (IIIh)

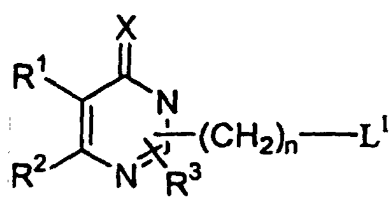

(IIIh)

where $L^1$ is a halogen atom, p-toluenesulfonate, methanesulfonate or trifluoromethanesulfonate, and all other symbols are as defined above, with a compound of formula (IIIi)

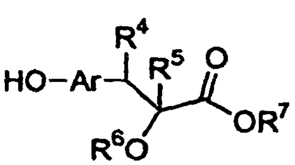

(IIIi)

where all symbols are as defined earlier, to produce a compound of the formula (I) defined above; or

d) reacting a compound of formula (IIIj)

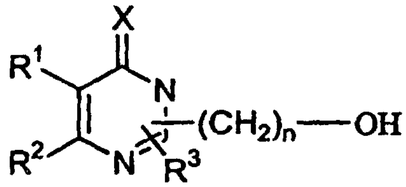

(IIIj)

where all symbols are as defined above, with a compound of formula (IIIi)

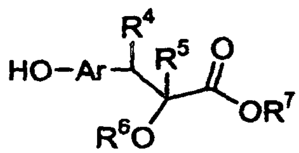

(IIIi)

where all symbols are as defined earlier, to produce a compound of the formula (I) defined above; or
e) reacting a compound of formula (IVd)

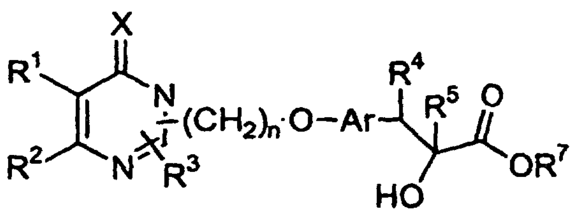

(IVd)

which represents a compound of formula (I) where $R^6$ represents hydrogen atom and all other symbols are as defined above, with a compound of formula (IVe)

$$R^6\text{-}L^3 \quad \text{(IVe)}$$

where $R^6$ represents $(C_1\text{-}C_{12})$alkyl, $(C_1\text{-}C_6)$alkoxy$(C_1\text{-}C_6)$alkyl, phenyl or benzyl, and $L^3$ is a halogen atom, to produce a compound of formula (I) defined above; or
f) reacting a compound of the formula (IIIa).

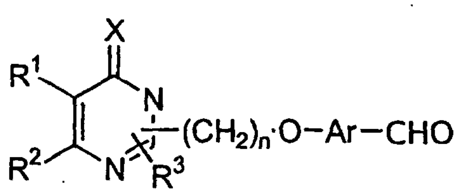

(IIIa)

where all symbols are as defined above, with a compound of formula (IIIg)

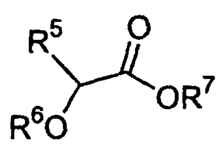

(IIIg)

where $R^5$ is hydrogen, and all other symbols are as defined above, to yield a compound of formula (I) as defined above after dehydroxylation; or
g) reacting a compound of formula (IIIc)

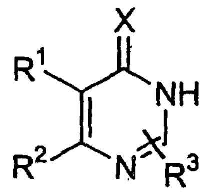

(IIIc)

where all symbols are as defined above, with a compound of formula (IIId)

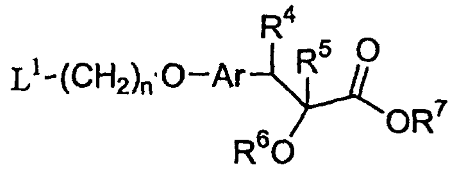

(IIId)

where $L^1$ is a halogen atom, p-toluenesulfonate, methanesulfonate or trifluoromethanesulfonate leaving group, and all other symbols are as defined above to produce a compound of formula (I) defined above, where the linker group $-(CH_2)_n$-O-is attached to nitrogen atom; or
h) reacting a compound of formula (IIIe)

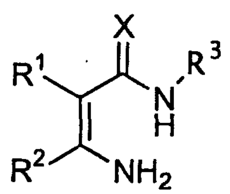

(IIIe)

where all symbols are as defined above, with a compound of formula (IIIf)

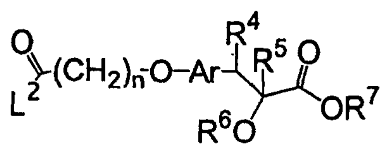

(IIIf)

where all symbols are as defined above, and $L^2$ is a halogen atom, to produce a compound of formula (I) defined above, where the linker group —$(CH_2)_n$-O- is attached to carbon atom; or
i) converting a compound of formula (IVf)

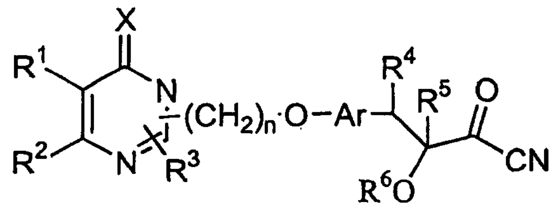

(IVf)

where all symbols are as defined above, to a compound of formula (I) defined above; or

j) reacting a compound of formula (IVg)

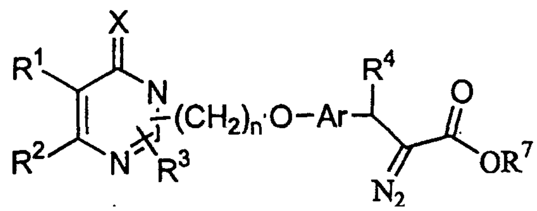

(IVg)

where $R^7$ is as defined above excluding hydrogen, and all other symbols are as defined above, with a compound of formula (IVc)

$$R^6\text{-OH} \qquad \text{(IVc)}$$

where $R^6$ represents $(C_1\text{-}C_{12})$alkyl, $(C_1\text{-}C_6)$alkoxy$(C_1\text{-}C_6)$alkyl, phenyl or benzyl , to produce a compound of the formula (I) defined above; or

k) cyclising a compound of formula (IIIm)

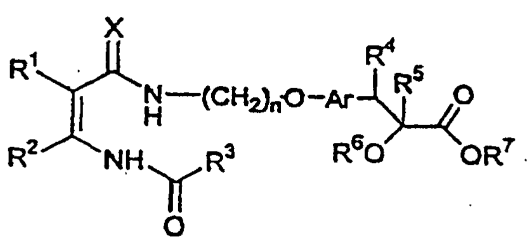

(IIIm)

where $R^7$ is as defined above excluding hydrogen, and all other symbols are as defined above, to produce a compound of formula (I) defined above where the linking group - $(CH_2)n$-O- is attached to nitrogen atom; and, if desired,

l) converting the compounds of formula (I) obtained in any of the processes described above into its pharmaceutically acceptable salts or its pharmaceutically acceptable solvates.

**3.** A process for the preparation of compound of formula (I)

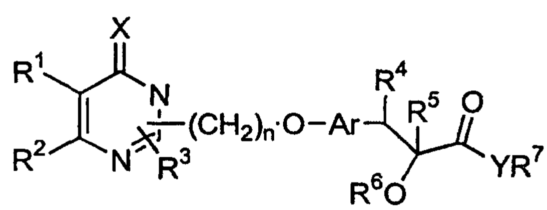

(I)

wherein X, $R^1$, $R^2$, $R^3$, $R^4$, $R^5$, $R^6$, Ar and n are as defined in claim 1 , $R^7$ represents hydrogen and Y represents oxygen atom, which comprises:

hydrolising a compound of formula (I) as defined in claim 2, where $R^7$ represents ($C_1$-$C_{12}$) alkyl or phenyl, optionally substituted with halogen.

**4.** A process for the preparation of compound of formula (I)

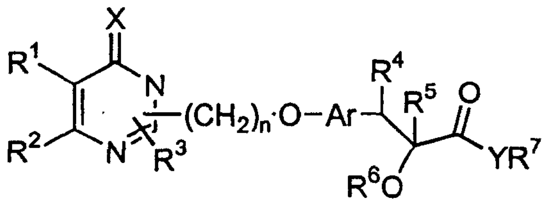

(I)

wherein X, $R^1$, $R^2$, $R^3$, $R^4$, $R^5$, $R^6$, $R^7$, Ar and n are as defined in claim 1, Y represents $NR^8$, where $R^8$ represents hydrogen or ($C_1$-$C_{12}$)alkyl, or $R^7$ and $R^8$ together with the nitrogen atom to which they are attached form a 5 or 6 membered cyclic structure containing carbon atoms, a nitrogen atom and optionally an oxygen atom, which comprises:

a) reacting a compound of formula (I)

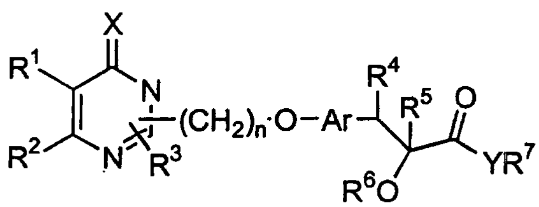

(I)

where all symbols are as defined above and Y represents oxygen and $R^7$ represents hydrogen or a ($C_1$-$C_{12}$) alkyl group or $YR^7$ represents a halogen atom, or $COYR^7$ represents a mixed anhydride group, with appropriate amines of the formula $NHR^7R^8$, where $R^7$ and $R^8$ are as defined earlier,
and, if desired,
b) converting the compounds of formula (I) obtained above into its pharmaceutically acceptable salts or its pharmaceutically acceptable solvates.

**5.** An intermediate of formula (IVf)

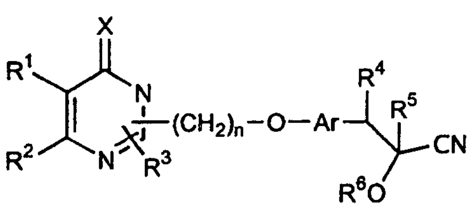

(IVf)

wherein X, $R^1$, $R^2$ ,$R^3$ , n, Ar , $R^4$ , $R^5$ and $R^6$ are as defined in claim 1.

**6.** A process for the preparation of the intermediate of formula (IVf) defined in claim 5 which comprises:

a) reacting a compound of formula (IIIa)

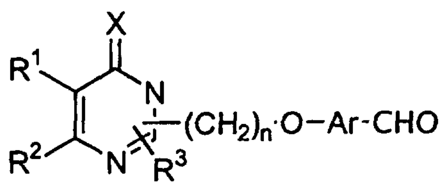

(IIIa)

where all symbols are as defined in claim 5, with a compound of formula (IVh)

$$R^6OCH_2P^+Ph_3{}^-Hal \quad (IVb)$$

where $R^6$ represents $(C_1-C_{12})$alkyl, $(C_1-C_6)$alkoxy$(C_1-C_6)$alkyl, phenyl or benzyl, and Hal represents a halogen atom, to yield a compound of formula (IVi)

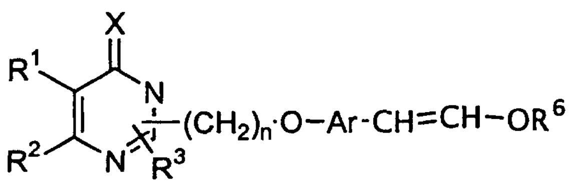

(IVi)

where all symbols are as defined above,
b) reacting the compound of formula (IVi) with an alcohol of the formula $R^6OH$ where $R^6$ represents $(C_1-C_{12})$ alkyl, $(C_1-C_6)$alkoxy$(C_1-C_6)$alkyl, phenyl or benzyl, to yield a compound of formula (IVj),

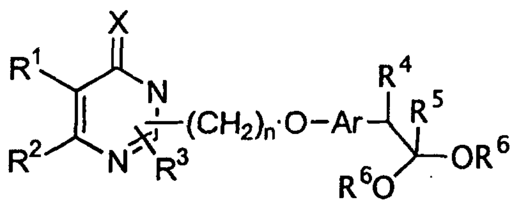

(IVj)

where $R^6$ is as defined above, $R^4$ and $R^5$ represent hydrogen, and all other symbols are as defined earlier, and
c) reacting the compound of formula (IVj) obtained above with trialkylsilyl cyanide to produce a compound of formula (TVf) where all symbols are as defined above.

**7.** An intermediate of formula (IVg)

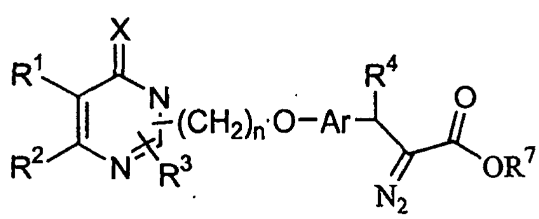

(IVg)

wherein X, $R^1$, $R^2$ , $R^3$ , n, Ar, $R^4$, and $R^7$ are as defined in claim 1.

**8.** A process for the preparation of the intermediate of formula (IVg) as defined in claim 7, which comprises:

a) reacting a compound of formula (IIIh)

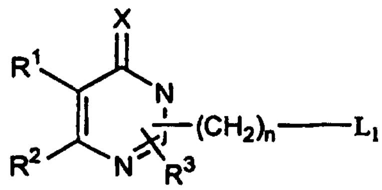

(IIIb)

where $L^1$ is a halogen atom, p-toluenesulfonate, methanesulfonate or trifluoromethanesulfonate, and all other symbols are as defined above, with a compound of formula (IV1)

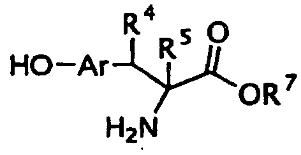

(IVl)

where $R^5$ is hydrogen atom and all other symbols are as defined above, to yield a compound of formula (IVk)

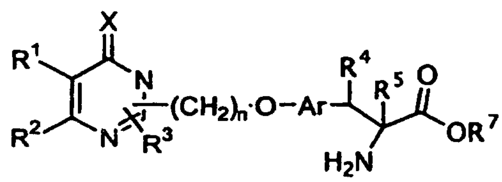

(IVk)

where $R^5$ is hydrogen atom and all other symbols are as defined above, and
b) reacting a compound of formula (IVk) obtained above with an diazotizing agent.

**9.** An intermediate of formula (IIIn)

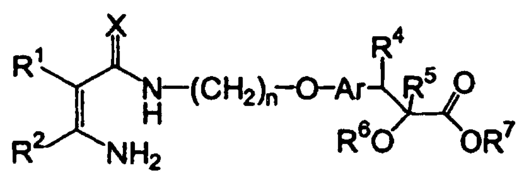

(IIIn)

wherein X, $R^1$, $R^2$ , n, Ar, $R^4$, $R^5$, $R^6$, and $R^7$ are as defined in claim 1.

**10.** A process for the preparation of the intermediate of formula (IIIn) defined in claim 9,

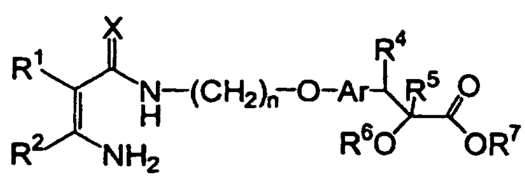

(IIIn)

which comprises reacting a compound of formula (IVm)

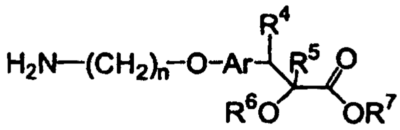

(IVm)

where all symbols are as defined in claim 9, with a compound of formula (IVo)

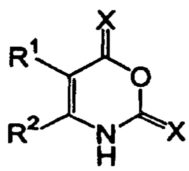

(IVo)

where $R^1$, $R^2$ and X are as defined earlier, to produce a compound of formula (IIIn) defined above.

**11.** An intermediate of formula (IIIm)

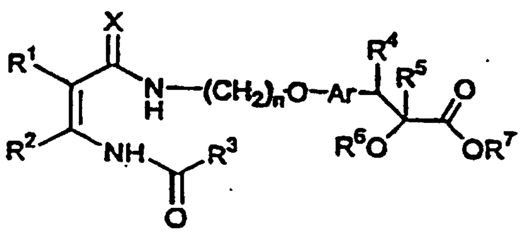

(IIIm)

wherein X, $R^1$, $R^2$, $R^3$, n, Ar, $R^4$ , $R^5$ , $R^6$ and $R^7$ are as defined in claim 1.

**12.** A process for the preparation of the intermediate of formula (IIIm) defined in claim 11.

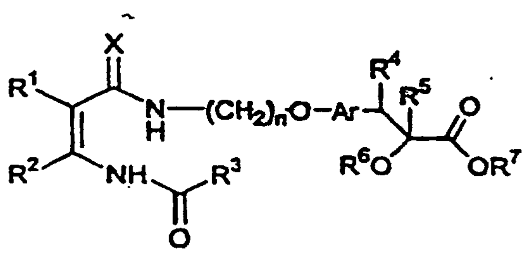

(IIIm)

which comprises reacting a compound of formula (IIIn)

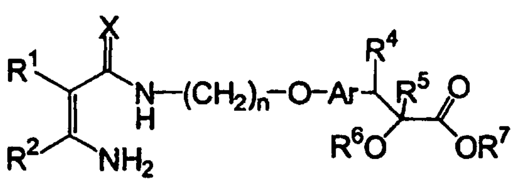

(IIIn)

where all symbols are as defined above, with a compound of formula (IIIo)

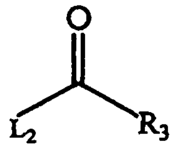

(IIIo)

where $L^2$ is a halogen atom and all other symbols are as defined above, to produce a compound of formula (IIIm) where all symbols are as defined above.

**13.** A compound according to claim 1 which is selected from:

(±)-Ethyl 2-ethoxy-3-[4-[[3-methyl-4-oxo-3,4-dihydro-2-quinazolinyl] methoxy]phenyl]propanoate;
(±)-2-Ethoxy-3-[4-[[3-methyl-4-oxo-3,4-dihydro-2-quinazolinyl] methoxy] phenyl]propanoic acid;
(±)-Sodium 2-ethoxy-3-[4-[[3-methyl-4-oxo-3,4-dihydro-2-quinazolinyl] methoxy]phenyl]propanoate;
(+)-2-Ethoxy-3-[4-[[3-methyl-4-oxo-3,4-dihydro-2-quinazolinyl] methoxy] phenyl]propanoic acid;
(-)-2-Ethoxy-3-[4-[[3-methyl-4-oxo-3,4-dihydro-2-quinazolinyl] methoxy] phenyl]propanoic acid;
(-)-Sodium 2-ethoxy-3-[4-[[3-methyl-4-oxo-3,4-dihydro-2-quinazolinyl] methoxy]phenyl]propanoate;
(±)-(Morpholine-4-yl) 2-ethoxy-3-[4-[[3-methyl-4-oxo-3,4-dihydro-2-quinazolinyl]methoxy]phenyl]propanamide;
(±)-2-Ethoxy-3-[4-[[3-methyl-4-oxo-3,4-dihydro-2-quinazolinyl] methoxy] phenyl]-N-(2-fluorophenyl)propanamide;
(±)-Ethyl 2-methoxy-3-[4-[[3-methyl-4-oxo-3,4-dihydro-2-quinazolinyl] methoxy]phenyl]propanoate;
(±)-2-Metboxy-3-[4-[[3-methyl-4-oxo-3,4-dihydro-2-quinazolinyl] methoxy] phenyl]propanoic acid;
(±)-Ethyl 2-propoxy-3-[4-[[3-methyl-4-oxo-3,4-dihydro-2-quinazolinyl) methoxy]phenyl]propanoate;
(±)-2-Propoxy-3-[4-[[3-methyl-4-oxo-3,4-dihydro-2-quinazolinyl) methoxy] phenyl]propanoic acid;
(±)-Ethyl 2-(n-butoxy)-3-[4-[[3-methyl-4-oxo-3,4-dihydro-2-quinazolinyl] methoxy] phenyl]propanoate;
(±)-2-(n-Butoxy)-3-[4-[[3-methyl-4-oxo-3,4-dihydro-2-quinazolinyl] methoxy] phenyl]propanoic acid;
(±)-Ethyl 2-(n-octyloxy)-3-[4-[[3-methyl-4-oxo-3,4-dihydro-2-quinazolinyl] methoxy]phenyl]propanoate;
(±)-2-(n-Octyloxy)-3-[4-[[3-methyl-4-oxo-3,4-dihydro-2-quinazolinyl] methoxy]phenyl]propanoic acid;
(±)-Ethyl 2-benzyloxy-3-[4-[[3-methyl-4-oxo-3,4-dihydro-2-quinazolinyl] methoxy]phenyl] propanoate;
(±)-2-Benzyloxy-3-[4-[(3-methyl-4-oxo-3,4-dihydro-2-quinazolinyl] methoxy] phenyl]propanoic acid;
(±)-Ethyl 2-phenoxy 3-[4-[[3-methyl-4-oxo-3,4-dihydro-2-quinazolinyl] methoxy]phenyl]propanoate;
(±)-2-Phenoxy-3-[4-[[3-methyl-4-oxo-3,4-dihydro-2-quinazolinyl] methoxy] phenyl]propanoic acid;
(±)-Ethyl 2-(2-methoxyethoxy)-3-[4-[[3-methyl-4-oxo-3,4-dihydro-2-quinazolinyl]methoxy]phenyl]propanoate;
(±)-2-(2-Methoxyethoxy)-3-[4-[[3-methyl4-oxo-3,4-dihydro-2-quinazolinyl] methoxy]phenyl]propanoic acid;
(±)-Ethyl 2-ethoxy-3-[4-[2-[2-ethyl-4-oxo-3,4-dihydro-3-quinazolinyl] ethoxy] phenyl]propanoate;
(±)-2-Ethoxy-3-[4-[2-[2-ethyl-4-oxo-3,4-dihydro-3-quinazolinyl]ethoxy] phenyl]propanoic acid;
(+)-2-Ethoxy-3-[4-[2-[2-ethyl-4-oxo-3,4-dihydro-3-quinazolinyl]ethoxy] phenyl]propanoic acid;
(-)-2-Ethoxy-3-[4-[2-[2-ethyl-4-oxo-3,4-dihydro-3-quinazolinyl]ethoxy] phenyl]propanoic acid;
(+)-Ethyl 2-ethoxy-3-[4-[2-[2-ethyl-4-oxo-3,4-dihydro-3-quinazolinyl]ethoxy] phenyl]propanoate;
(-)-Ethyl 2-ethoxy-3-[4-[2-[2-ethyl-4-oxo-3,4-dihydro-3-quinazolinyl] ethoxy] phenyl]propanoate;
(±)-Ethyl 2-ethoxy-3-[4-[2-[2-methyl-4-oxo-3,4-dihydro-3-quinazolinyl] ethoxy]phenyl]propanoate;
(±)-2-Ethoxy-3-[4-[2-[2-methyl-4-oxo-3,4-dihydro-3-quinazolinyl]ethoxy] phenyl]propanoic acid;
(+) -2-Ethoxy-3-[4-[2-[2-methyl-4-oxo-3,4-dihydro-3-quinazolinyl] ethoxy] phenyl]propanoic acid;
(-)-2-Ethoxy-3-[4-[2-[2-methyl-4-oxo-3,4-dihydro-3-quinazolinyl]ethoxy] phenyl]propanoic acid;
(+)-Ethyl 2-ethoxy-3-[4-[2-[2-methyl-4-oxo-3,4-dihydro-3-quinazolinyl] ethoxy]phenyl]propanoate;
(-)-Ethyl-2-ethoxy-3-[4-[2-[2-methyl-4-oxo-3,4-dihydro-3-quinazolinyl] ethoxy]phenyl]propanoate;
(±)-Ethyl 2-ethoxy-3-[4-[2-[4-oxo-3,4-dihydro-3-quinazolinyl]ethoxy] phenyl] propanoate;
(±)-2-Ethoxy-3-[4-[2-[4-oxo-3,4-dihydro-3-quinazolinyl]ethoxy]phenyl] propanoic acid;
(±)-Ethyl 2-phenoxy-3-[4-[2-[2-ethyl-4-oxo-3,4-dihydro-3-quinazolinyl] ethoxy]phenyl]propanoate;
(±)-2-Phenoxy-3-[4-[2-[2-ethyl-4-oxo-3,4-dihydro-3-quinazolinyl]ethoxy] phenyl]propanoic acid;
(±)-Ethyl 2-phenoxy-3-[4-[2-[2-methyl-4-oxo-3,4-dihydro-3-quinazolinyl] ethoxy]phenyl]propanoate;
(±)-2-Phenoxy-3-[4-[2-[2-methyl-4-oxo-3,4-dihydro-3-quinazolinyl]ethoxy] phenyl]propanoic acid;
(±)-Ethyl 2-ethoxy-3-[4-[2-[2-ethyl-4-methyl-6-oxo-1-pyrimidinyl] ethoxy] phenyl]propanoate;
(±)-2-Ethoxy-3-[4-[2-[2-ethyl-4-methyl-6-oxo-1-pyrimidinyl]ethoxy] phenyl] propanoic acid;

(±)-Ethyl 2-ethoxy-3-[4-[[3-phenyl-4-oxo-3,4-dihydro-2-quinazolinyl] methoxy]phenyl]propanoate;
(±)-2-Ethoxy-3-[4-[[3-phenyl-4-oxo-3,4-dihydro-2-quinazolinyl] methoxy] phenyl]propanoic acid;
(±)-Ethyl 2-ethoxy-3-[4-[[3-methyl-4-oxo-3,4-dihydro-6,7-dimethoxy-2-quinazolinyl]methoxy]phenyl]propanoate;
(±)-2-Ethoxy-3-[4-[[3-methyl-4-oxo-3,4-dihydro-6,7-dimethoxy-2-quinazolinyl]methoxy] phenyl]propanoic acid;
(±)-Ethyl 2-ethoxy-3-[4-[[3-(4-methylphenyl)-4-oxo-3,4-dihydro-2-quinazolinyl]methoxy] phenyl]propanoate;
(±)-2-Ethoxy-3-[4-[[3-(4-methylphenyl)-4-oxo-3,4-dihydro-2-quinazolinyl) methoxy]phenyl]propanoic acid;
(±)-Ethyl 2-ethoxy-3-[4-[[3-(4-methoxyphenyl)-4-oxo-3,4-dihydro-2-quinazolinyl]methoxy] phenyl]propanoate;
(±)-2-Ethoxy-3-[4-[[3-(4-methoxyphenyl)-4-oxo-3,4-dihydro-2-quinazolinyl] methoxy]phenyl]propanoic acid;
(±)-Ethyl 2-ethoxy-3-[4-[[3-benzyl-4-oxo-3,4-dihydro-2-quinazolinyl) methoxy]phenyl]propanoate;
(±)-2-Ethoxy-3-[4-[[3-benzyl-4-oxo-3,4-dihydro-2-quinazolinyl] methoxy] phenyl]propanoic acid;
(±)-Ethyl 2-ethoxy-3-[4-[[3-(3-chlorophenyl)-4-oxo-3,4-dihydro-2-quinazolinyl]methoxy]phenyl]propanoate;
(±)-2-Ethoxy-3-[4-[[3-(3-chlorophenyl)-4-oxo-3,4-dihydro-2-quinazolinyl] methoxy]phenyl]propanoic acid;
(±)-Ethyl 2-ethoxy-3-[4-[[3-(3-chloro-4-fluorophenyl)-4-oxo-3,4-dihydro-2-quinazolinyl]methoxy]phenyl]propanoate; or
(±)-2-Ethoxy-3-[4-[[3-(3-chloro-4-fluorophenyl)-4-oxo-3,4-dihydro-2-quinazolinyl] methoxy]phenyl]propanoic acid.

**14.** A composition which comprises a compound of formula (I) as defined in claim 1 or 13 and a pharmaceutically acceptable carrier, diluent, excipient or solvate.

**15.** A composition which comprises a compound of formula (IIIm) as defined in claim 11 and a pharmaceutically acceptable carrier, diluent, excipient or solvate.

**16.** A pharmaceutical composition as claimed in claim 14 or 15, in the form of a tablet, capsule, powder, syrup, solution or suspension.

**17.** Use of a compound of formula (I) as defined in any one of claims 1 or 13 or a compound of formula (IIIm) as defined in claim 11 for preparing a medicament for preventing or treating hyperlipemia, hypercholesteremia, hyperglycemia, osteoporosis, obesity, glycose intolerance, leptin resistance, insulin resistance, or diseases in which insulin resistance is the underlying pathophysiological mechanism.

**18.** Use of a compound of formula (I) as defined in any one of claims 1 or 13 or a compound of formula (IIIm) as defined in claim 11 for preparing a medicament for reducing plasma glucose, triglycerides, total cholesterol, LDL, VLDL or free fatty acids in the plasma.

**19.** Use of a compound of formula (I) as defined in any one of claims 1 or 13 or a compound of formula (IIIm) as defined in claim 11 for preparing a medicament in combination/concomittant with HMG CoA reductace inhibitors, fibrates, nicotinic acid, cholestyramine, colestipol or probucol for preventing or treating hypelipemia, hypercholesteremia, hyperglycemia, osteoporosis, obesity, glucose intolerance, leptin resistance, insulin resistance, or diseases in which insulin resistance is the underlying pathophysiological mechanism.

**20.** Use of a compound of formula (I) as defined in any one of claims 1 or 13 or a compound of formula (IIIm) as defined in claim 11 for preparing a medicament in combination/concomittant with HMG CoA reductace inhibitors, fibrates, nicotinic acid, cholestyramine, colestipol or probucol for reducing plasma glucose, triglycerides, total cholesterol, LDL, VLDL or free fatty acids in the plasma.

**21.** Use according to any one of claims 17 or 19 wherein the disease is type II diabetes, impaired glucose tolerance, dyslipidaemia, disorders related to Syndrome X such as hypertension, obesity, atherosclerosis, hyperlipidemia, coronary artery disease and other cardiovascular disorders, certain renal diseases including glomerulonephritis, glomerulosclerosis, nephrotic syndrome, hypertensive nephrosclerosis, retinopathy, nephropathy, disorders related to endothelial cell activation, psoriasis, polycystic ovarian syndrome (PCOS), useful as aldose reductase inhibitors, for improving cognitive functions in dementia and treating diabetic complications, osteoporosis, inflammatory bowel diseases, myotonic dystrophy, pancreatitis, arteriosclerosis, xanthoma or cancer.

**22.** Use of an agonist of PPARα and/or an agonist of PPARγ of Formula (I) as defined in claim 1 or 13 or of a formula (IIIm) as defined in claim 11 for preparation of a medicament for the treatment or prophylaxis of disorders related to Syndrome X.

**Patentansprüche**

1. Verbindung der Formel (I):

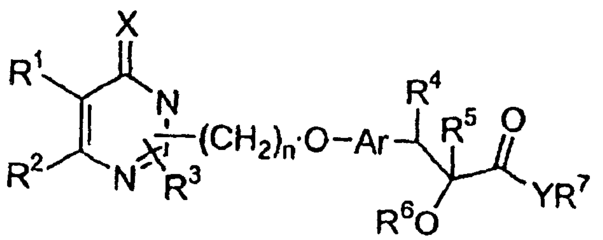

(I)

ihre tautomeren Formen, ihre Stereoisomere, ihre Polymorphe, ihre pharmazeutisch zulässigen Salze oder ihre pharmazeutisch zulässigen Solvate, worin X O oder S darstellt; die Gruppen $R^1$, $R^2$ und die Gruppe $R^3$, sofern am Kohlenstoffatom vorhanden, sind gleich oder verschieden und stellen Wasserstoff dar, Halogen, Hydroxy oder unsubstituierte oder substituierte Gruppen, ausgewählt aus $(C_1$-$C_6)$-Alkoxy, $(C_1$-$C_6)$-Alkyl oder Phenyl, worin in den Gruppen, sofern sie substituiert sind, der/die Substituent(en) ausgewählt ist/sind aus Halogen oder Hydroxy; oder $R^1$ und $R^2$ bilden gemeinsam mit den angrenzenden Atomen, an denen sie sich befinden, eine substituierte oder unsubstituierte Phenyl-Gruppe, worin, wenn die Phenyl-Gruppe durch $R^1$ und $R^2$ gebildet wird, diese substituiert ist und der/die Substituent(en) ausgewählt ist aus Halogen, Hydroxy, $(C_1$-$C_3)$-Alkyl oder $(C_1$-$C_3)$-Alkoxy; $R^3$, sofern es sich an einem Stickstoffatom befindet, stellt Wasserstoff dar oder unsubstituierte oder substituierte Gruppen, die ausgewählt sind aus $(C_1$-$C_6)$-Alkyl, Phenyl, Naphthyl, Benzyl oder Phenethyl, worin, wenn die am Stickstoff befindliche Gruppe $R^3$ substituiert ist, der Substituent ausgewählt ist aus Halogen, Hydroxy, Methyl, Ethyl, Isopropyl, n-Propyl, n-Butyl oder $(C_1$-$C_3)$-Alkoxy; die durch $-(CH_2)_n$-O- dargestellte verknüpfende Gruppe kann entweder über ein Stickstoffatom oder über ein Kohlenstoffatom angebracht sein, worin n eine ganze Zahl im Bereich von 1 bis 4 ist; Ar stellt Phenylen dar; $R^4$ stellt ein Wasserstoffatom dar, Methyl, Ethyl, Hydroxy, $(C_1$-$C_3)$-Alkoxy oder bildet eine Bindung mit $R^5$; $R^5$ stellt Wasserstoff dar, Methyl, Ethyl, Hydroxy, $(C_1$-$C_3)$-Alkoxy oder bildet eine Bindung mit $R^4$; $R^6$ stellt Wasserstoff dar, $(C_1$-$C_{12})$-Alkyl, $(C_1$-$C_6)$-Alkoxy$(C_1$-$C_6)$-alkyl, Phenyl oder Benzyl unter der Voraussetzung, dass $R^6$ nicht . Wasserstoff darstellt, wenn $R^7$ Wasserstoff darstellt oder eine $(C_1$-$C_{12})$-Alkyl-Gruppe; $R^7$ stellt Wasserstoff dar oder eine unsubstituierte oder substituierte Gruppe, die ausgewählt ist aus $(C_1$-$C_{12})$ -Alkyl oder Phenyl, worin, wenn $R^7$ substituiert ist, der/die Substituent(en) an $R^7$ ausgewählt ist/sind aus Halogen; Y stellt Sauerstoff oder $NR^8$ dar, worin $R^8$ darstellt oder $(C_1$-$C_{12})$-Alkyl; oder $R^7$ und $R^8$ bilden gemeinsam mit dem Stickstoffatom, an dem sie sich befinden, eine 5-oder 6-gliedrige cyclische Struktur, die Kohlenstoffatome enthält, ein Stickstoffatom und wahlweise ein Sauerstoffatom.

2. Verfahren für die Herstellung einer Verbindung der Formel (I):

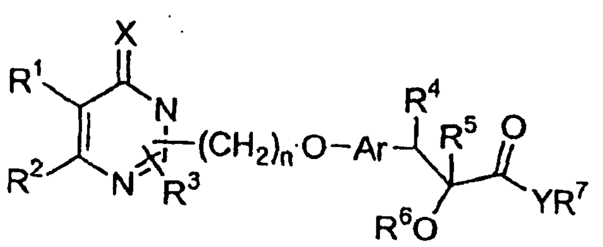

(I)

worin sind X, $R^1$, $R^2$, $R^3$, $R^4$, $R^5$, $R^6$, Ar, n und $R^7$ wie in Anspruch 1 festgelegt und Y stellt ein Sauerstoffatom dar, welches Verfahren umfasst:

a) Reduzieren einer Verbindung der Formel (IVa):

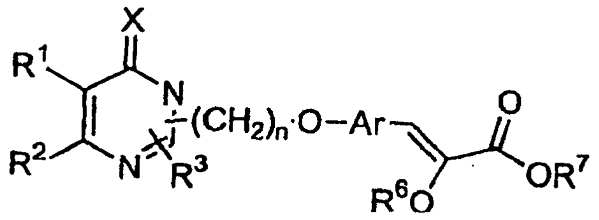

(IVa)

worin alle Symbole wie vorstehend festgelegt sind, um eine Verbindung der Formel (I) zu liefern, worin $R^4$ und $R^5$ jeweils ein Wasserstoffatom darstellen und alle anderen Symbole wie vorstehend festgelegt sind; oder

b) Umsetzen einer Verbindung der Formel (IVa):

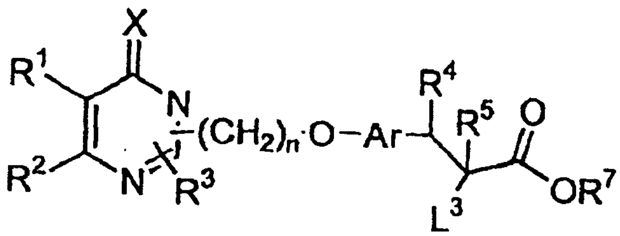

(IVb)

worin alle Symbole wie vorstehend festgelegt sind, $R^7$ ist wie vorstehend festgelegt mit Ausnahme von Wasserstoff und $L^3$ ist ein Halogenatom; und zwar mit einem Alkohol der Formel (IVc):

$R^6$-OH **(IVc)**

worin $R^6$ darstellt: ($C_1$-$C_{12}$)-Alkyl, ($C_1$-$C_6$)-Alkoxy($C_1$-$C_6$)-alkyl, Phenyl oder Benzyl,

um eine Verbindung der Formel (I) entsprechend der vorstehenden Festlegung zu erzeugen; oder

c) Umsetzen einer Verbindung der Formel (IIIh):

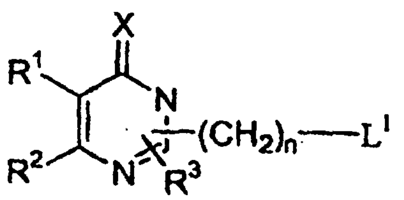

(IIIh)

worin $L^1$ ein Halogenatom ist, p-Toluolsulfonat, Methansulfonat oder Trifluormethansulfonat und alle anderen Symbole wie vorstehend festgelegt sind;

und zwar mit einer Verbindung der Formel (IIIi):

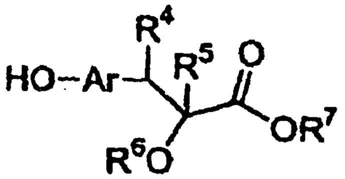

(IIIi)

worin alle Symbole wie vorstehend festgelegt sind; um eine Verbindung der Formel (I) entsprechend der vorstehenden Festlegung zu erzeugen; oder

d) Umsetzen einer Verbindung der Formel (IIIj)

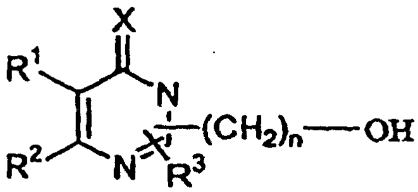

(IIIj)

worin alle Symbole wie vorstehend festgelegt sind;
und zwar mit einer Verbindung der Formel (IIIi):

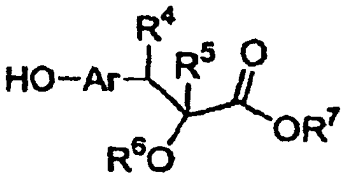

(IIIi)

worin alle Symbole wie vorstehend festgelegt sind; um eine Verbindung der Formel (I) entsprechend der vorstehenden Festlegung zu erzeugen; oder

e) Umsetzen einer Verbindung der Formel (IVd):

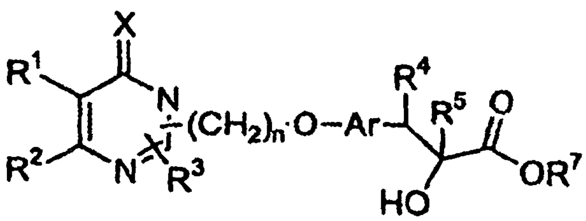

(IVd)

die eine Verbindung der Formel (I) darstellt, worin $R^6$ ein Wasserstoffatom darstellt und alle anderen Symbole wie vorstehend festgelegt sind;

und zwar mit einer Verbindung der Formel (IVe):

$R^6$-$L^3$ **(IVe)**

worin $R^6$ darstellt: ($C_1$-$C_{12}$)-Alkyl, ($C_1$-$C_6$)-Alkoxy($C_1$-$C_6$)-alkyl, Phenyl oder Benzyl und $L^3$ ein Halogenatom ist;

und zwar um eine Verbindung der Formel (I) entsprechend der vorstehenden Festlegung zu erzeugen; oder

f) Umsetzen einer Verbindung der Formel (IIIa):

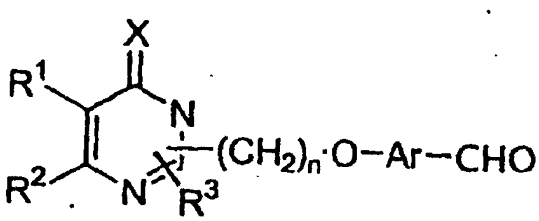

(IIIa)

worin alle Symbole wie vorstehend festgelegt sind; mit einer Verbindung der Formel IIIg:

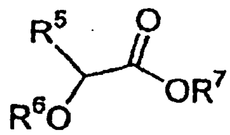

(IIIg)

worin $R^5$ Wasserstoff ist und alle anderen Symbole wie vorstehend festgelegt sind, um eine Verbindung der Formel (I) entsprechend der vorstehenden Festlegung nach Dehydroxylierung zu liefern; oder

g) Umsetzen einer Verbindung der Formel (IIIc):

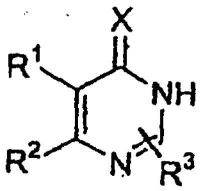

(IIIc)

worin alle Symbole wie vorstehend festgelegt sind; und zwar mit einer Verbindung der Formel (IIId):

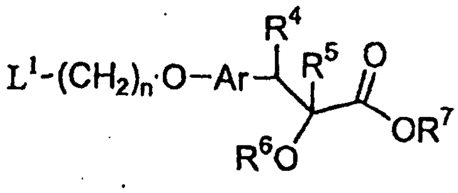

(IIId)

worin $L^1$ ein Halogenatom ist, p-Toluolsulfonat, Methansulfonat oder Trifluormethansulfonat-spaltende Gruppe und alle anderen Symbole wie vorstehend festgelegt sind;

und zwar um eine Verbindung der Formel (I) entsprechend der vorstehenden Festlegung zu erzeugen, worin die verknüpfende Gruppe $-(CH_2)_n-O-$ an einem Stickstoffatom angebracht ist; oder

h) Umsetzen einer Verbindung der Formel (IIIe):

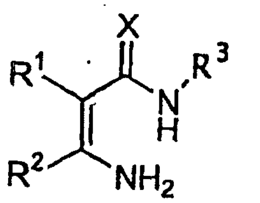

(IIIe)

worin alle Symbole wie vorstehend festgelegt sind; und zwar mit einer Verbindung der Formel (IIIf)

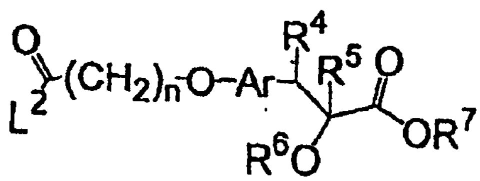

(IIIf)

worin alle Symbole wie vorstehend festgelegt sind und $L^2$ ein Halogenatom ist;

und zwar um eine Verbindung der Formel (I) entsprechend der vorstehenden Festlegung zu erzeugen, worin die verknüpfende Gruppe $-(CH_2)_n$-O- an ein Kohlenstoffatom angebracht ist; oder

i) Umwandeln einer Verbindung der Formel (IVf):

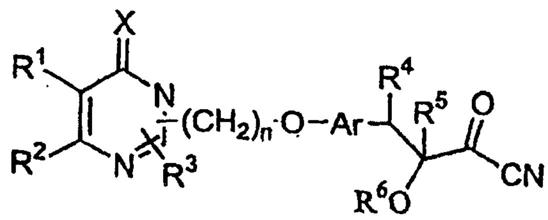

**(IVf)**

worin alle Symbole wie vorstehend festgelegt sind; in eine Verbindung der Formel (I) entsprechend der vorstehenden Festlegung; oder

j) Umsetzen einer Verbindung der Formel (IVg):

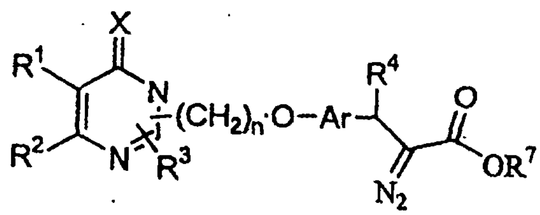

**(IVg)**

worin $R^7$ wie vorstehend festgelegt ist unter Ausnahme von Wasserstoff und alle anderen Symbole wie vorstehend festgelegt sind; und zwar mit einer Verbindung der Formel (IVc):

$R^6$-OH **(IVc)**

worin $R^6$ darstellt: $(C_1-C_{12})$-Alkyl, $(C_1-C_6)$-Alkoxy$(C_1-C_6)$-alkyl, Phenyl oder Benzyl;

und zwar um eine Verbindung der Formel (I) entsprechend der vorstehenden Festlegung zu erzeugen; oder

k) Cyclisieren einer Verbindung der Formel (IIIm):

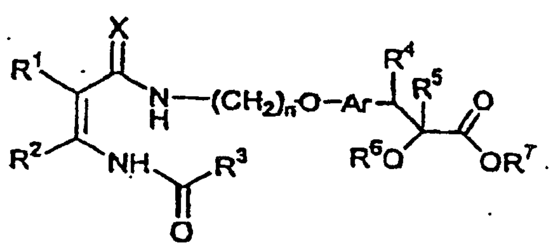

**(IIIm)**

worin $R^7$ wie vorstehend festgelegt ist unter Ausnahme von Wasserstoff und alle anderen Symbole wie vorstehend festgelegt sind; und zwar um eine Verbindung der Formel (I) entsprechend der vorstehenden Festlegung zu erzeugen, worin die verknüpfende Gruppe $-(CH_2)_n$-O- an einem Stickstoffatom angebracht ist;

und nach Erfordernis:

l) Umwandeln der Verbindungen der Formel (I), die nach einem beliebigen der vorstehend beschriebenen Verfahren erhalten wurden, in ihre pharmazeutisch zulässigen Salze oder ihre pharmazeutisch zulässigen Solvate.

**3.** Verfahren zur Herstellung der Verbindung der Formel (I):

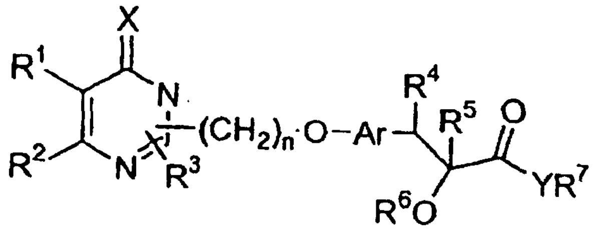

(I)

worin X, $R^1$, $R^2$, $R^3$, $R^4$, $R^5$, $R^6$ wie in Anspruch 1 festgelegt sind, $R^7$ stellt Wasserstoff dar und Y stellt ein Sauerstoffatom dar; welches Verfahren umfasst:

Hydrolysieren einer Verbindung der Formel (I) entsprechend der Festlegung in Anspruch 2, worin $R^7$ $(C_1-C_{12})$-Alkyl oder Phenyl darstellt, wahlweise substituiert mit Halogen.

**4.** Verfahren zur Herstellung einer Verbindung der Formel (I):

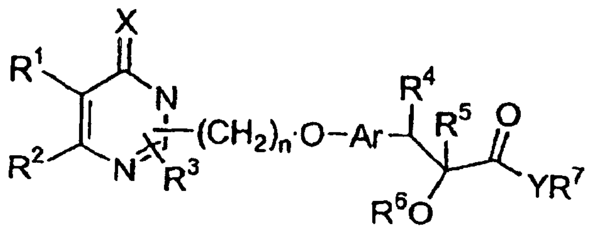

(I)

worin X, $R^1$, $R^2$, $R^3$, $R^4$, $R^5$, $R^6$, $R^7$, Ar und n wie in Anspruch 1 festgelegt sind, Y stellt $NR^8$ dar, worin $R^8$ Wasserstoff darstellt oder $(C_1-C_{12})$-Alkyl, oder $R^7$ und $R^8$ bilden gemeinsam mit dem Stickstoffatom, an dem sie sich befinden, eine 5- oder 6-gliedrige cyclische Struktur, die Kohlenstoffatome enthält, ein Stickstoffatom und wahlweise ein Sauerstoffatom;

welches Verfahren umfasst:

a) Umsetzen einer Verbindung der Formel (I):

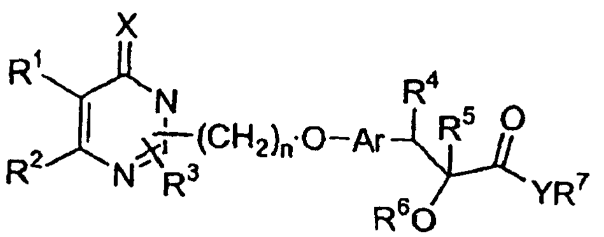

(I)

worin alle Symbole wie vorstehend festgelegt sind und Y Sauerstoff darstellt und $R^7$ Wasserstoff darstellt oder eine $(C_1-C_{12})$-Alkyl-Gruppe, oder $YR^7$ stellt ein Halogenatom dar oder $COYR^7$ stellt eine gemischte Anhydrid-Gruppe dar; und zwar mit geeigneten Aminen der Formel $NHR^7R^8$, worin $R^7$ und $R^8$ wie vorstehend festgelegt sind;

und nach Erfordernis:

b) Umwandeln der Verbindungen der vorstehend erhaltenen Verbindungen der Formel (I) in ihre pharmazeutisch zulässigen Salze oder ihre pharmazeutisch zulässigen Solvate.

**5.** Intermediat der Formel (IVf):

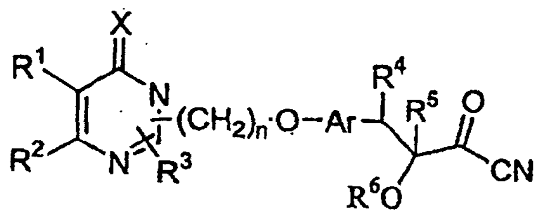

(IVf)

worin X, $R^1$, $R^2$, $R^3$, n, Ar, $R^4$, $R^5$ und $R^6$ wie in Anspruch 1 festgelegt sind.

**6.** Verfahren zur Herstellung des Intermediats der Formel (IVf) nach Anspruch 5, welches Verfahren umfasst:

a) Umsetzen einer Verbindung der Formel (IIIa):

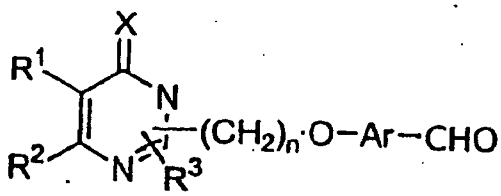

(IIIa)

worin alle Symbole wie in Anspruch 5 festgelegt sind;
und zwar mit einer Verbindung der Formel (IVh):

$$R^6OCH_2P^+Ph_3{}^-Hal \qquad (IVh)$$

worin $R^6$ darstellt: $(C_1\text{-}C_{12})$-Alkyl, $(C_1\text{-}C_6)$-Alkoxy$(C_1\text{-}C_6)$-alkyl, Phenyl oder Benzyl; und Hal ein Halogenatom darstellt; und zwar um eine Verbindung der Formel (IVi) zu liefern:

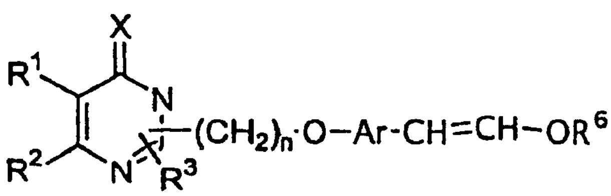

(IVi)

worin alle Symbole wie vorstehend festgelegt sind;
b) Umsetzen der Verbindung der Formel (IVi) mit einem Alkohol der Formel $R^6OH$, worin $R^6$ darstellt $(C_1\text{-}C_{12})$-Alkyl, $(C_1\text{-}C_6)$-Alkoxy$(C_1\text{-}C_6)$-alkyl, Phenyl oder Benzyl, um eine Verbindung der Formel (IVj) zu liefern:

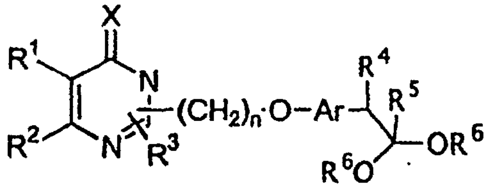

(IVj)

worin $R^6$ wie vorstehend festgelegt ist, $R^4$ und $R^5$ stellen Wasserstoff dar und alle anderen Symbole sind wie vorstehend festgelegt; und
c) Umsetzen der Verbindung der vorstehend erhaltenen Verbindung der Formel (IVj) mit Trialkylsilylcyanid, um eine Verbindung der Formel (IVf) zu erzeugen, worin alle Symbole wie vorstehend festgelegt wurden.

**7.** Intermediat der Formel (IVg):

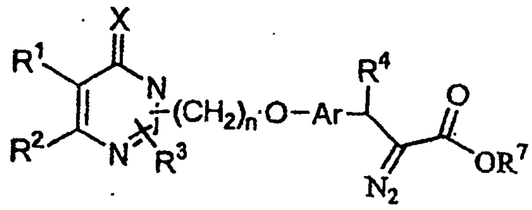

(IVg)

worin X, $R^1$, $R^2$, $R^3$, n, Ar, $R^4$ und $R^7$ wie in Anspruch 1 festgelegt wurde.

**8.** Verfahren zur Herstellung des Intermediats der Formel (Ivg) nach Anspruch 7, welches Verfahren umfasst:

a) Umsetzen einer Verbindung der Formel (IIIh):

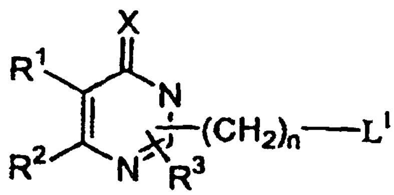

(IIIh)

worin $L^1$ ein Halogenatom ist, p-Toluolsulfonat, Methansulfonat oder Trifluormethansulfonat und alle anderen Symbole wie vorstehend festgelegt sind; und zwar mit einer Verbindung der Formel (IVI):

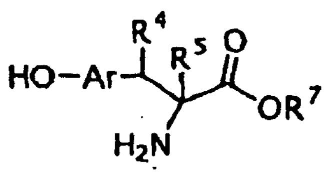

(IVI)

worin $R^5$ ein Wasserstoffatom ist und alle anderen Symbole wie vorstehend festgelegt, um eine Verbindung der Formel (IVk) zu liefern:

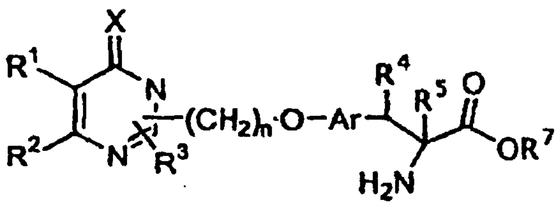

(IVk)

worin $R^5$ ein Wasserstoffatom ist und alle anderen Symbole wie vorstehend festgelegt sind; sowie
b) Umsetzen einer Verbindung der Formel (IVk), die vorstehend erhalten wurde, mit einem Mittel zum Diazotieren.

**9.** Intermediat der Formel (IIIn):

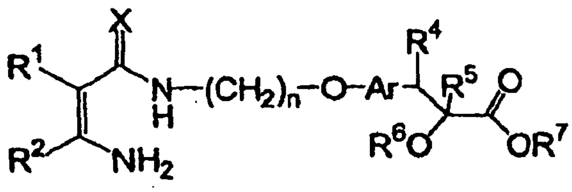 (IIIn)

worin X, $R^1$, $R^2$, n, Ar, $R^4$, $R^5$, $R^6$ und $R^7$ wie in Anspruch 1 festgelegt sind.

**10.** Verfahren zur Herstellung des Intermediats der Formel (IIIn) nach Anspruch 9:

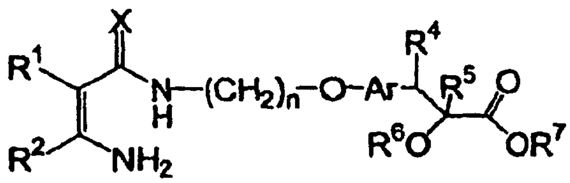 (IIIn)

welches Verfahren umfasst: Umsetzen einer Verbindung der Formel (IVm):

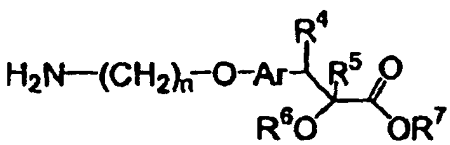 (IVm)

worin alle Symbole wie in Anspruch 9 festgelegt sind;
und zwar mit einer Verbindung der Formel (IVo):

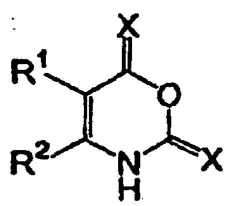 (IVo)

worin $R^1$, $R^2$ und X wie vorstehend festgelegt sind; um eine Verbindung der Formel (IIIn) entsprechend der vorstehenden Festlegung zu erzeugen.

**11.** Intermediat der Formel (IIIm):

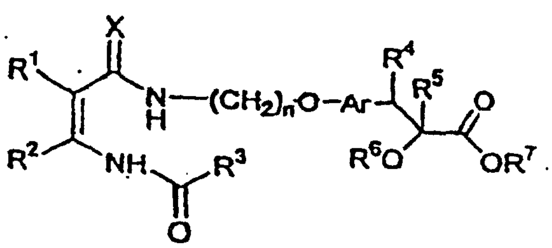 (IIIm)

worin X, $R^1$, $R^2$, $R^3$, n, Ar, $R^4$, $R^5$, $R^6$ und $R^7$ wie in Anspruch 1 festgelegt sind.

**12.** Verfahren zur Herstellung des Intermediats der Formel (IIIm) nach Anspruch 11:

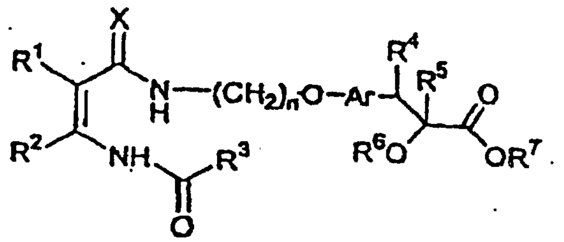

(IIIm)

welches Verfahren umfasst: Umsetzen einer Verbindung der Formel (IIIn):

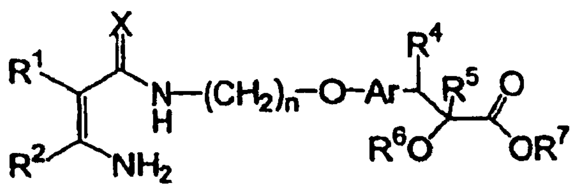

(IIIn)

worin alle Symbole wie vorstehend festgelegt sind; und zwar mit einer Verbindung der Formel (IIIo):

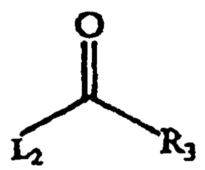

(IIIo)

worin $L^2$ ein Halogenatom ist und alle anderen Symbole wie vorstehend festgelegt sind;
und zwar um eine Verbindung der Formel (IIIm) zu erzeugen, worin alle Symbole wie vorstehend festgelegt sind.

**13.** Verbindung nach Anspruch 1, die ausgewählt ist aus:

(±)-Ethyl-2-ethoxy-3-[4-[[3-methyl-4-oxo-3,4-dihydro-2-chinazolinyl]methoxy]phenyl]propanoat;

(±)-2-Ethoxy-3-[4-[[3-methyl-4-oxo-3,4-dihydro-2-chinazolinyl]methoxy]phenyl]propansäure;

(±)-Natrium-2-ethoxy-3-[4-[[3-methyl-4-oxo-3,4-dihydro-2-chinazolinyl]methoxy]phenyl]propanoat;

(+)-2-Ethoxy-3-[4-[[3-methyl-4-oxo-3,4-dihydro-2-chinazolinyl]methoxy]phenyl]propansäure;

(-)-2-Ethoxy-3-[4-[[3-methyl-4-oxo-3,4-dihydro-2-chinazolinyl]methoxy]phenyl]propansäure;

(±)-Natrium-2-ethoxy-3-[4-[[3-methyl-4-oxo-3,4-dihydro-2-chinazolinyl]methoxy]phenyl]propanoat;

(±)-(Morpholin-4-yl)-2-ethoxy-3-[4-[[3-methyl-4-oxo-3,4-dihydro-2-chinazolinyl]methoxy]phenyl]propanamid;

(±)-2-Ethoxy-3-[4-[[3-methyl-4-oxo-3,4-dihydro-2-chinazolinyl]methoxy]phenyl]-N-(2-fluorophenyl)-propanamid;

(±)-Ethyl-2-methoxy-3-[4-[[3-methyl-4-oxo-3,4-dihydro-2-chinazolinyl]methoxy]phenyl]propanoat;

(±)-2-Methoxy-3-[4-[[3-methyl-4-oxo-3,4-dihydro-2-chinazolinyl]methoxy]phenyl]propansäure;

(±)-Ethyl-2-propoxy-3-[4-[[3-methyl-4-oxo-3,4-dihydro-2-chinazolinyl]methoxy]phenyl]propanoat;

(±)-2-Propoxy-3-[4-[[3-methyl-4-oxo-3,4-dihydro-2-chinazolinyl]methoxy]phenyl]propansäure;

(±)-Ethyl-2-(n-butoxy)-3-[4-[[3-methyl-4-oxo-3,4-dihydro-2-chinazolinyl]methoxy]phenyl]propanoat;

(±)-2-(n-Butoxy)-3-[4-[[3-methyl-4-oxo-3,4-dihydro-2-chinazolinyl]methoxy]phenyl]propansäure;

(±)-Ethyl-2-(n-octyloxy)-3-[4-[[3-methyl-4-oxo-3,4-dihydro-2-chinazolinyl]methoxy]phenyl]propanoat;

(±)-2-(n-Octyloxy)-3-[4-[[3-methyl-4-oxo-3,4-dihydro-2-chinazolinyl]methoxy]phenyl]propansäure;

(±)-Ethyl-2-benzyloxy-3-[4-[[3-methyl-4-oxo-3,4-dihydro-2-chinazolinyl]methoxy]phenyl]propanoat;

(±)-2-Benzyloxy-3-[4-[[3-methyl-4-oxo-3,4-dihydro-2-chinazolinyl]methoxy]phenyl]propansäure;

(±)-Ethyl-2-phenoxy-3-[4-[[3-methyl-4-oxo-3,4-dihydro-2-chinazolinyl]methoxy]phenyl]propanoat;

(±)-2-Phenoxy-3-[4-[[3-methyl-4-oxo-3,4-dihydro-2-chinazolinyl]methoxy]phenyl]propansäure;

(±)-Ethyl-2-(2-methoxyethoxy)-3-[4-[[3-methyl-4-oxo-3,4-dihydro-2-chinazolinyl]methoxy]phenyl]propanoat;

(±)-2-(2-Methoxyethoxy)-3-[4-[[3-methyl-4-oxo-3,4-dihydro-2-chinazolinyl]methoxy]phenyl]propansäure;

(±)-Ethyl-2-ethoxy-3-[4-[2-[2-ethyl-4-oxo-3,4-dihydro-3-chinazolinyl]ethoxy]phenyl]propanoat;

(±)-2-Ethoxy-3-[4-[2-[2-ethyl-4-oxo-3,4-dihydro-3-chinazolinyl]ethoxy]phenyl]propansäure;

(+)-2-Ethoxy-3-[4-[2-[2-ethyl-4-oxo-3,4-dihydro-3-chinazolinyl]ethoxy]phenyl]propansäure;

(-)-2-Ethoxy-3-[4-[2-[2-ethyl-4-oxo-3,4-dihydro-3-chinazolinyl]ethoxy]phenyl]propansäure;

(+)-Ethyl-2-ethoxy-3-[4-[2-[2-ethyl-4-oxo-3,4-dihydro-3-chinazolinyl]ethoxy]phenyl]propanoat;

(-)-Ethyl-2-ethoxy-3-[4-[2-[2-ethyl-4-oxo-3,4-dihydro-3-chinazolinyl]ethoxy]phenyl]propanoat;

(±)-Ethyl-2-ethoxy-3-[4-[2-[2-methyl-4-oxo-3,4-dihydro-3-chinazolinyl]ethoxy]phenyl]propanoat;

(±)-2-Ethoxy-3-[4-[2-[2-methyl-4-oxo-3,4-dihydro-3-chinazolinyl]ethoxy]phenyl]propansäure;

(+)-2-Ethoxy-3-[4-[2-[2-methyl-4-oxo-3,4-dihydro-3-chinazolinyl]ethoxy]phenyl]propansäure;

(-)-2-Ethoxy-3-[4-[2-[2-methyl-4-oxo-3,4-dihydro-3-chinazolinyl]ethoxy]phenyl]propansäure;

(+)-Ethyl-2-ethoxy-3-[4-[2-[2-methyl-4-oxo-3,4-dihydro-3-chinazolinyl]ethoxy]phenyl]propanoat;

(-)-Ethyl-2-ethoxy-3-[4-[2-[2-methyl-4-oxo-3,4-dihydro-3-chinazolinyl]ethoxy]phenyl]propanoat;

(±)-Ethyl-2-ethoxy-3-[4-[2-[4-oxo-3,4-dihydro-3-chinazolinyl]ethoxy]phenyl]propanoat;

(±)-2-Ethoxy-3-[4-[2-[4-oxo-3,4-dihydro-3-chinazolinyl]ethoxy]phenyl]propansäure;

(±)-Ethyl-2-phenoxy-3-[4-[2-[2-ethyl-4-oxo-3,4-dihydro-3-chinazolinyl]ethoxy]phenyl]propanoat;

(±)-2-Phenoxy-3-[4-[2-[2-ethyl-4-oxo-3,4-dihydro-3-chinazolinyl]ethoxy]phenyl]propansäure;

(±)-Ethyl-2-phenoxy-3-[4-[2-[2-methyl-4-oxo-3,4-dihydro-3-chinazolinyl]ethoxy]phenyl]propanoat;

(±)-2-Phenoxy-3-[4-[2-[2-methyl-4-oxo-3,4-dihydro-3-chinazolinyl]ethoxy]phenyl]propansäure;

(±)-Ethyl-2-ethoxy-3-[4-[2-[2-ethyl-4-methyl-6-oxo-1-pyrimidinyl]ethoxy]phenyl]propanoat;

(±)-2-Ethoxy-3-[4-[2-[2-ethyl-4-methyl-6-oxo-1-pyrimidinyl]ethoxy]phenyl]propansäure;

(±)-Ethyl-2-ethoxy-3-[4-[[3-phenyl-4-oxo-3,4-dihydro-2-chinazolinyl]methoxy]phenyl]propanoat;

(±)-2-Ethoxy-3-[4-[[3-phenyl-4-oxo-3,4-dihydro-2-chinazolinyl]methoxy]phenyl]propansäure;

(±)-Ethyl-2-ethoxy-3-[4-[[3-methyl-4-oxo-3,4-dihydro-6,7-dimethoxy-2-chinazolinyl]methoxy]phenyl]propanoat;

(±)-2-Ethoxy-3-[4-[[3-methyl-4-oxo-3,4-dihydro-6,7-dimethoxy-2-chinazolinyl]methoxy]phenyl]propansäure;

(±)-Ethyl-2-ethoxy-3-[4-[[3-(4-methylphenyl)-4-oxo-3,4-dihydro-2-chinazolinyl]methoxy]phenyl]propanoat;

(±)-2-Ethoxy-3-[4-[[3-(4-methylphenyl)-4-oxo-3,4-dihydro-2-chinazolinyl]methoxy]phenyl]propansäure;

(±)-Ethyl-2-ethoxy-3-[4-[[3-(4-methoxyphenyl)-4-oxo-3,4-dihydro-2-chinazolinyl]methoxy]phenyl]propanoat;

(±)-2-Ethoxy-3-[4-[[3-(4-methoxyphenyl)-4-oxo-3,4-dihydro-2-chinazolinyl]methoxy]phenyl]propansäure;

(±)-Ethyl-2-ethoxy-3-[4-[[3-benzyl-4-oxo-3,4-dihydro-2-chinazolinyl]methoxy]phenyl]propanoat;

(±)-2-Ethoxy-3-[4-[[3-benzyl-4-oxo-3,4-dihydro-2-chinazolinyl]methoxy]phenyl]propansäure;

(±)-Ethyl-2-ethoxy-3-[4-[[3-(3-chlorphenyl)-4-oxo-3,4-dihydro-2-chinazolinyl]methoxy]phenyl]propanoat;

(±)-2-Ethoxy-3-[4-[[3-(3-chlorphenyl)-4-oxo-3,4-dihydro-2-chinazolinyl]methoxy]phenyl]propansäure;

(±)-Ethyl-2-ethoxy-3-[4-[[3-(3-chlor-4-fluorphenyl)-4-oxo-3,4-dihydro-2-chinazolinyl]methoxy]phenyl]propanoat; oder

(±)-2-Ethoxy-3-[4-[[3-(3-chlor-4-fluorphenyl)-4-oxo-3,4-dihydro-2-chinazolinyl]methoxy]phenyl]propansäure.

**14.** Zusammensetzung, aufweisend eine Verbindung der Formel (I) nach Anspruch 1 oder 13 sowie einen pharmazeutisch zulässigen Trägersubstanz, Streckmittel, Arzneimittelträger oder Solvat.

**15.** Zusammensetzung, aufweisend eine Verbindung der Formel (IIIm) nach Anspruch 11 und einen pharmazeutisch zulässigen Trägersubstanz, Streckmittel, Arzneimittelträger oder Solvat.

**16.** Pharmazeutische Zusammensetzung nach Anspruch 14 oder 15 in Form einer Tablette, Kapsel, eines Pulvers, Sirups, einer Lösung oder Suspension.

**17.** Verwendung einer Verbindung der Formel (I) nach einem der Ansprüche 1 oder 13 oder eine Verbindung der Formel (IIIm) nach Anspruch 11 zur Herstellung eines Medikaments für die Verhütung oder Behandlung von Hyperlipämie, Hypercholesterinämie, Hyperglykämie, Osteoporosis, Obesitas, Glykoseintoleranz, Leptinresistenz, Insulinresistenz oder Erkrankungen, bei denen Insulinresistenz der zugrunde liegende pathophysiologische Mechanismus ist.

**18.** Verwendung einer Verbindung der Formel (I) nach einem der Ansprüche 1 oder 13 oder einer Verbindung der Formel (IIIm) nach Anspruch 11 zur Herstellung eines Medikaments für die Verringerung von Plasmaglucose, Triglyceriden, Gesamtcholesterin, LDL, VLDL oder freie Fettsäuren im Plasma.

**19.** Verwendung einer Verbindung der Formel (I) nach einem der Ansprüche 1 oder 13 oder eine Verbindung der

Formel (IIIm) nach Anspruch 11 zur Herstellung eines Medikaments in Kombination/konkumittierend mit HMG CoA-Reduktase-Inhibitoren, Fibraten, Nicotinsäure, Cholesterylamin, Colestipol oder Probucol zur Verhütung oder Behandlung von Hyperlipämie, Hypercholesterinämie, Hyperglykämie, Osteoporosis, Obesitas, Glykoseintoleranz, Leptinresistenz, Insulinresistenz oder Erkrankungen, bei denen Insulinresistenz der zugrunde liegende pathophysiologische Mechanismus ist.

**20.** Verwendung einer Verbindung der Formel (I) nach einem der Ansprüche 1 oder 13 oder einer Verbindung der Formel (IIIm) nach Anspruch 11 zur Herstellung eines Medikamentes in Kombination/konkumittierend mit HMG CoA-Reduktase-Inhibitoren, Fibraten, Nicotinsäure, Cholesterylamin, Colestipol oder Probucol zur Verringerung von Plasmaglucose, Triglyceriden, Gesamtcholesterin, LDL, VLDL oder freien Fettsäuren in Plasma.

**21.** Verwendung nach einem der Ansprüche 17 oder 19, worin die Erkrankung Diabetes Typ 11 ist, beeinträchtigte Glucosetoleranz, Dyslipidämie, Erkrankungen im Zusammenhang mit dem Syndrom X, wie beispielsweise Bluthochdruck, Obesitas, Atherosklerose, Hyperlipidämie, koronare Herzerkrankung und andere kardiovaskuläre Erkrankungen, bestimmte Nierenerkrankungen, einschließlich Glomerulonephritis, Glomerulosklerose, nephrotisches Syndrom, hypertensive Nephrosklerose, Retinopathie, Nephropathie, Erkrankungen im Zusammenhang mit endothelialer Zellaktivierung, Psoriasis, polycystisches Ovarsyndrom (PCOS), verwendbar als Aldosereduktase-Inhibitoren, zur Verbesserung kognitiver Funktionen bei Demens und Behandlung diabetischer Komplikationen, Osteoporose, entzündliche Darmerkrankungen, myotonische Dystrophie, Pankreatitis, Arteriosklerose, Xanthom oder Krebs.

**22.** Verwendung eines Agonisten von PPARα und/oder eines Agonisten von PPARγ der Formel (I) nach Anspruch 1 oder 13 oder einer Formel (IIIm) nach Anspruch 11 zur Herstellung eines Medikaments für die Behandlung oder Prophylaxe von Erkrankungen im Zusammenhang mit Syndrom X.

## Revendications

**1.** Composé de formule (I)

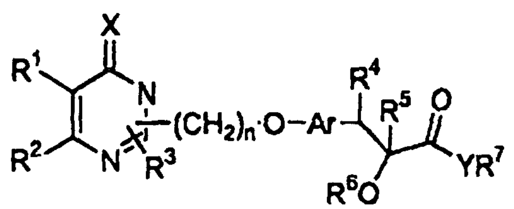

(I)

ses formes tautomères, ses stéréo-isomères, ses polymorphes, ses sels pharmaceutiquement acceptables, ou ses produits de solvatation pharmaceutiquement acceptables, dans laquelle X représente O ou S ; les groupements $R^1$, $R^2$ et le groupement $R^3$ lorsque présents sur un atome de carbone, sont identiques ou différents et représentent un hydrogène, un halogène, un hydroxy, ou des groupements substitués ou non-substitués choisis parmi un alcoxy en $C_1$-$C_6$, un alkyle en $C_1$-$C_6$, ou un phényle, dans lesquels lorsque lesdits groupements sont substitués, le(s) substituant(s) sont choisis parmi un halogène ou un hydroxy ; ou $R^1$, $R^2$ ainsi que les atomes adjacents auxquels ils sont liés forment un groupement phényle substitué ou non-substitué, dans lequel lorsque le groupement phényle formé par $R^1$ et $R^2$ est substitué, le(s) substituant(s) sont choisis parmi un halogène, un hydroxy, un alkyle en $C_1$-$C_3$ ou un alcoxy en $C_1$-$C_3$ ; $R^3$ lorsque lié à un atome d'azote représente un hydrogène, ou des groupements non-substitués ou substitués choisis parmi un alkyle en $C_1$-$C_6$, un phényle, un naphtyle, un benzyle ou un phénéthyle, dans lequel lorsque le groupement $R^3$ lié à l'azote est substitué, le substituant est choisi parmi un halogène, un hydroxy, un méthyle, un éthyle, un isopropyle, un n-propyle, un n-butyle ou un alcoxy en $C_1$-$C_3$ ; le groupement liant représenté par $-(CH_2)_n-O-$ peut être lié soit par un atome d'azote, soit par un atome de carbone où n est un entier compris dans la plage allant de 1 à 4 ; Ar représente un phénylène ; $R^4$ représente un atome d'hydrogène, un méthyle, un éthyle, un hydroxy, un alcoxy en $C_1$-$C_3$ ou forme une liaison avec $R^5$ ; $R^5$ représente un hydrogène méthyle, un éthyle, un hydroxy, un alcoxy en $C_1$-$C_3$ ou forme une liaison avec $R^4$ ; $R^6$ représente un hydrogène, un alkyle en $C_1$-$C_{12}$, un ($C_1$-$C_6$)alcoxy($C_1$-$C_6$)alkyle, un phényle ou un benzyle, à la condition que $R^6$ ne représente par un hydrogène lorsque $R^7$ représente un hydrogène ou un groupement alkyle

en $C_1$-$C_{12}$ ; $R^7$ représente un hydrogène ou un groupement substitué ou non-substitué choisi parmi un alkyl en $C_1$-$C_{12}$ ou un phényle, dans lequel lorsque $R^7$ est substitué, le(s) substituant(s) sur $R^7$ sont choisis parmi un halogène : Y représente un oxygène ou $NR^8$ ; où $R^8$ représente un hydrogène ou un alkyle en $C_1$-$C_{12}$ ; ou $R^7$ et $R^8$ conjointement avec l'atome d'azote auquel ils sont liés forment une structure cyclique penta- ou hexagonale contenant des atomes de carbone, un atome d'azote et facultativement un atome d'oxygène.

**2.** Procédé pour la préparation d'un composé de formule (I)

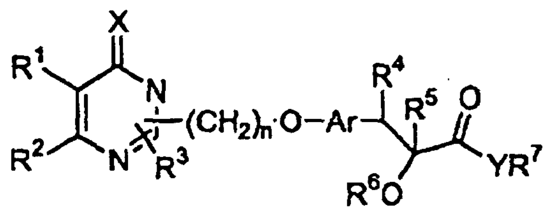

**(I)**

où X, $R^1$, $R^2$, $R^3$, $R^4$, $R^5$, $R^6$, Ar, n et $R^7$ sont tels que définis dans la revendication 1 et Y représente un atome d'oxygène, qui comprend :

a)la réduction d'un composé de formule (IVa)

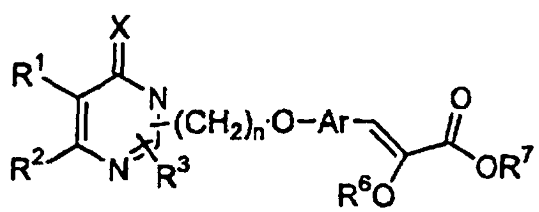

**(IVa)**

où tous les symboles sont tels que définis plus tôt, pour produire un composé de la formule (I) où $R^4$ et $R^5$ représentent chacun un atome d'hydrogène, et tous les autres symboles sont tels que définis ci-dessus ;
ou
b) la réaction d'un composé de formule (IVb)

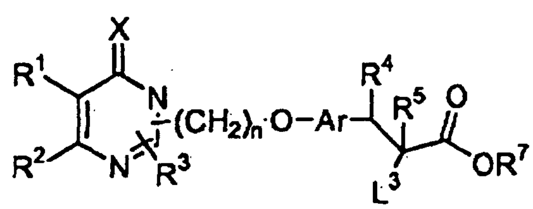

**(IVb)**

où tous les symboles sont tels que définis ci-dessus, $R^7$ est tel que défini ci-dessus à l'exclusion de l'hydrogène, et $L^3$ est un atome d'halogène, avec un alcool de formule (IVc),

$$R^6\text{-OH} \qquad \textbf{(IVc)}$$

où $R^6$ représente un alkyle en $C_1$-$C_{12}$, un ($C_1$-$C_6$)alcoxy($C_1$-$C_6$)alkyle, un phényle ou un benzyle, pour produire un composé de la formule (I) définie ci-dessus ; ou
c) la réaction d'un composé de formule (IIIh)

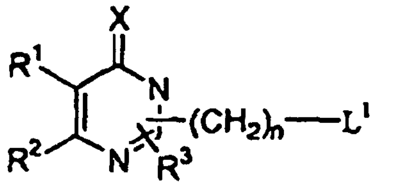

(IIIh)

où $L^1$ est un atome d'halogène, un p-toluènesulfonate, un méthanesulfonate ou un trifluorométhanesulfonate, et tous les autres symboles sont tels que définis ci-dessus, avec un composé de formule (IIIi)

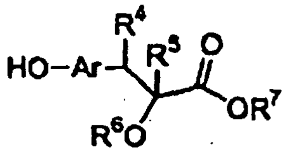

(IIIi)

où tous les symboles sont tels que définis plus tôt, pour produire un composé de la formule (I) définie ci-dessus ; ou

d) la réaction d'un composé de formule (IIIj)

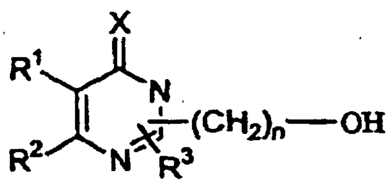

(IIIj)

où tous les symboles sont tels que définis ci-dessus, avec un composé de formule (IIIi)

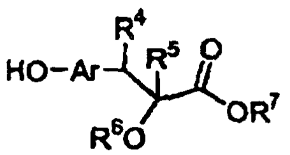

(IIIi)

où tous les symboles sont tels que définis ci-dessus, pour produire un composé de la formule (I) définie ci-dessus ; ou

e) la réaction d'un composé de formule (IVd)

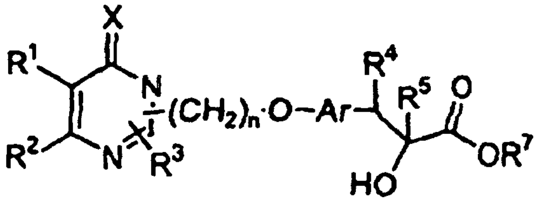

(IVd)

qui représente un composé de formule (I) où $R^6$ représente un atome d'hydrogène et tous les autres symboles sont tels que définis ci-dessus, avec un composé de formule (IVe)

$$R^6\text{-}L^3 \qquad \textbf{(IVe)}$$

où $R^6$ représente un alkyle en $C_1$-$C_{12}$,un ($C_1$-$C_6$)alcoxy($C_1$-$C_6$)alkyle, un phényle ou un benzyle, et $L^3$ est un atome d'halogène, pour produire un composé de formule (I) définie ci-dessus ; ou
f) la réaction d'un composé de la formule (IIIa)

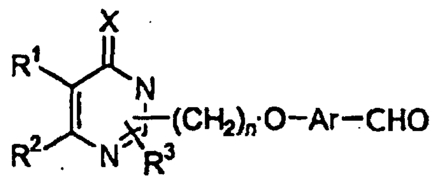

**(IIIa)**

où tous les symboles sont tels que définis ci-dessus, avec un composé de formule (IIIg)

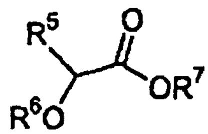

**(IIIg)**

où $R^5$ est un hydrogène, et tous les autres symboles sont tels que définis ci-dessus, pour produire un composé de formule (I) telle que définie ci-dessus après déshydroxylation ; ou
g) la réaction d'un composé de formule (IIIc)

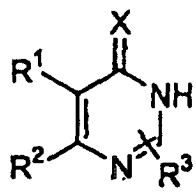

**(IIIc)**

où tous les symboles sont tels que définis ci-dessus, avec un composé de formule (IIId)

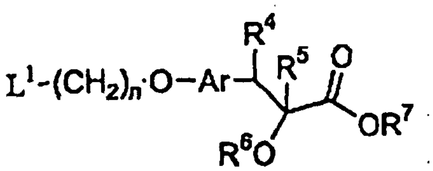

**(IIId)**,

où $L^1$ est un atome d'halogène, un p-toluènesulfonate, un méthanesulfonate ou un groupe partant trifluorométhanesulfonate, et tous les autres symboles sont tels que définis ci-dessus pour produire un composé de formule (I) défini ci-dessus, où le groupement liant $-(CH_2)_n$-O- est lié à un atome d'azote ; ou
h) la réaction d'un composé de formule (IIIe)

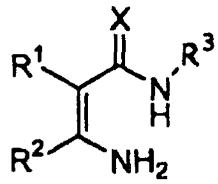

(IIIe)

où tous les symboles sont tels que définis ci-dessus, avec un composé de formule (IIIf)

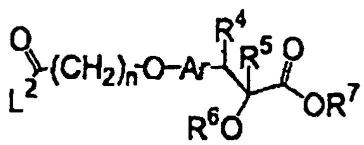

(IIIf)

où tous les symboles sont tels que définis ci-dessus, et $L^2$ est un atome d'halogène, pour produire un composé de formule (I) défini ci-dessus, où le groupement liant $-(CH_2)_n-O-$ est lié à un atome de carbone ; ou

i) la conversion d'un composé de formule (IVf)

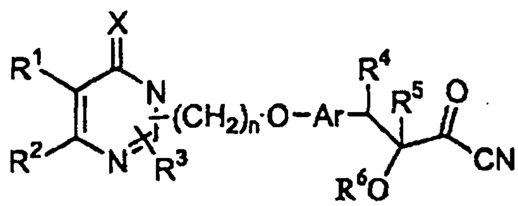

(IVf)

où tous les symboles sont tels que définis ci-dessus, pour donner un composé de formule (I) défini ci-dessus ; ou

j) la réaction d'un composé de formule (IVg)

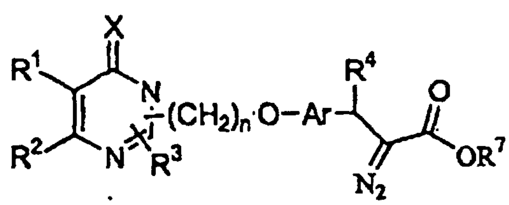

(IVg)

où $R^7$ est tel que défini ci-dessus à l'exclusion de l'hydrogène, et tous les autres symboles sont tels que définis ci-dessus, avec un composé de formule (IVc)

**$R^6$-OH (IVc)**

où $R^6$ représente un alkyle en $C_1$-$C_{12}$, un ($C_1$-$C_6$)alcoxy($C_1$-$C_6$)alkyle, un phényle ou un benzyle, pour produire un composé de la formule (I) défini ci-dessus ; ou

k) la cyclisation d'un composé de formule (IIIm)

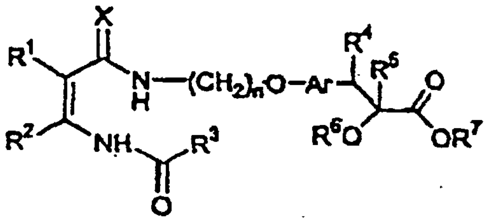

(IIIm)

où $R^7$ est tel que défini ci-dessus à l'exclusion de l'hydrogène, et tous les autres symboles sont tels que définis ci-dessus, pour produire un composé de formule (I) défini ci-dessus où le groupement liant $-(CH_2)_n-O-$ est lié à l'atome d'hydrogène ;

et, si désiré,

l) la conversion de composés de formule (I) obtenus dans l'un quelconque des procédés décrits ci-dessus en ses sels pharmaceutiquement acceptables ou ses produits de solvatation pharmaceutiquement acceptables.

**3.** Procédé pour la préparation d'un composé de formule (I)

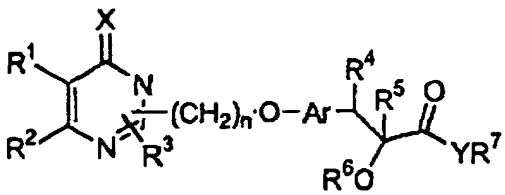

(I)

dans laquelle X, $R^1$, $R^2$, $R^3$, $R^4$, $R^5$, $R^6$, Ar et n sont tels que définis dans la revendication 1, $R^7$ représente un hydrogène et Y représente un atome d'oxygène, qui comprend :

l'hydrolyse d'un composé de formule (I) tel que défini dans la revendication 2, où $R^7$ représente un alkyle en $C_1$-$C_{12}$ ou un phényle, facultativement substitué par un halogène.

**4.** Procédé pour la préparation d'un composé de formule (I)

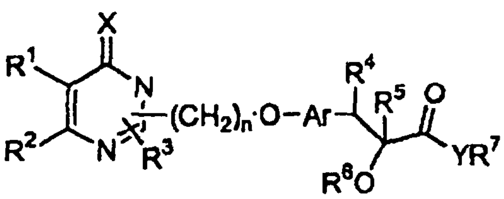

(I)

dans laquelle X, $R^1$, $R^2$, $R^3$, $R^4$, $R^5$, $R^6$,$R^7$, Ar et n sont tels que définis dans la revendication 1, Y représente un $NR^8$, où $R^8$ représente un hydrogène ou un alkyle en $C_1$-$C_{12}$, ou $R^7$ et $R^8$ conjointement avec l'atome d'hydrogène auquel ils sont liés forment une structure cyclique penta- ou hexagonale contenant des atomes de carbone, un atome d'azote et facultativement un atome d'oxygène, qui comprend :

a) la réaction d'un composé de formule (I)

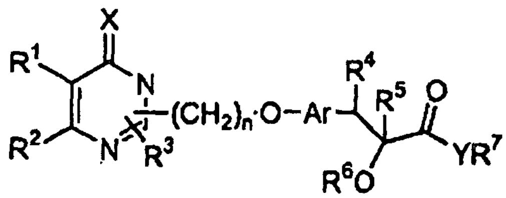

(I)

où tous les symboles sont tels que définis ci-dessus et Y représente un oxygène et $R^7$ représente un hydrogène ou un groupement alkyle en $C_1$-$C_{12}$ ou $YR^7$ représente un atome d'halogène, ou $COYR^7$ représente un groupement anhydride mixte, avec des amines appropriées de la formule $NHR^7R^8$, où $R^7$ et $R^8$ sont tels que définis plus tôt, et, si désiré,

b) la conversion des composés de formule (I) obtenus ci-dessus en ses sels pharmaceutiquement acceptables ou ses produits de solvatation pharmaceutiquement acceptables.

**5.** Un intermédiaire de formule (IVf)

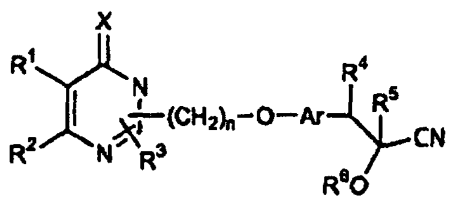

(IVf)

dans laquelle X, $R^1$, $R^2$, $R^3$, n, Ar, $R^4$, $R^5$ et $R^6$ sont tels que définis dans la revendication 1.

**6.** Procédé pour la préparation de l'intermédiaire de formule (IVf) défini dans la revendication 5 qui comprend :

a) la réaction d'un composé de formule (IIIa)

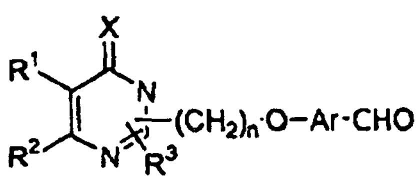

(IIIa)

où tous les symboles sont tels que définis dans la revendication 5, avec un composé de formule (IVh)

$$R^6OCH_2P^+Ph_3{}^-Hal \qquad \textbf{(IVh)}$$

où $R^6$ représente un alkyle en $C_1$-$C_{12}$, un ($C_1$-$C_6$)alcoxy($C_1$-$C_6$)alkyle, un phényle ou un benzyle, et Hal représente un atome d'halogène, pour produire un composé de formule (IVi)

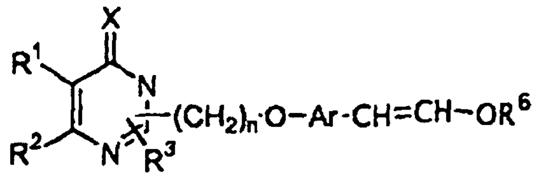

(IVi)

où tous les symboles sont tels que définis ci-dessus,

b) la réaction d'un composé de formule (Ivi) avec un alcool de formule $R^6OH$ où $R^6$ représente un alkyle en $C_1$-$C_{12}$, un ($C_1$-$C_6$)alcoxy($C_1$-$C_6$), un phényle ou un benzyle, pour produire un composé de formule (IVj)

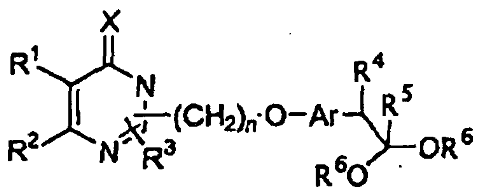

(IVj)

où $R^6$ est tel que défini ci-dessus, $R^4$ et $R^5$ représentent un hydrogène, et tous les autres symboles sont tels que définis plus tôt, et

c) la réaction d'un composé de formule (IVj) obtenu ci-dessus avec du trialkylsilyle cyanure pour produire un composé de formule (IVf) où tous les symboles sont tels que définis ci-dessus.

**7.** Intermédiaire de formule (IVg)

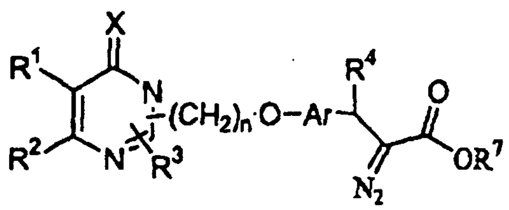

(IVg)

dans laquelle X, $R^1$, $R^2$, $R^3$, n, Ar, $R^4$, et $R^7$ sont tels que définis dans la revendication 1.

**8.** Procédé pour la préparation de l'intermédiaire de formule (IVg) tel que défini dans la revendication 7, qui comprend :

la réaction d'un composé de formule (IIIh)

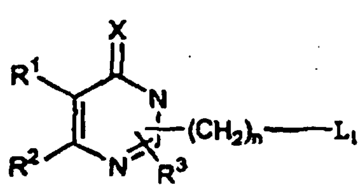

(IIIh)

où $L^1$ est un atome d'halogène, un p-toluènesulfonate, un méthanesulfonate ou un trifluorométhanesulfonate, et tous les autres symboles sont tels que définis ci-dessus, avec un composé de formule (IV1)

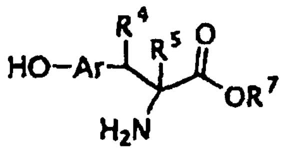

(IVl)

où $R^5$ est un atome d'hydrogène et tous les autres symboles sont tels que définis ci-dessus, pour produire un composé de formule (IVk)

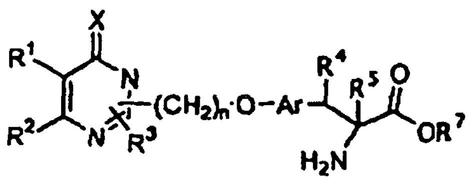

(IVk)

où $R^5$ est un atome d'hydrogène et tous les autres symboles sont tels que définis ci-dessus, et

b) la réaction d'un composé de formule (IVk) obtenu ci-dessus avec un agent de diazotation.

**9.** Intermédiaire de formule (IIIN)

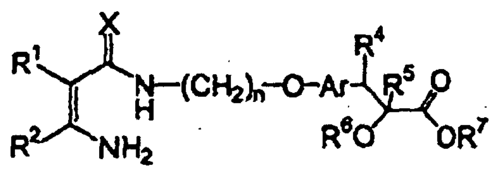

(IIIn)

dans laquelle X, $R^1$, $R^2$, n, Ar, $R^4$, $R^5$, $R^6$, et $R^7$ sont tels que définis dans la revendication 1.

**10.** Procédé pour la préparation d'un intermédiaire de formule (IIIn) défini en revendication 9,

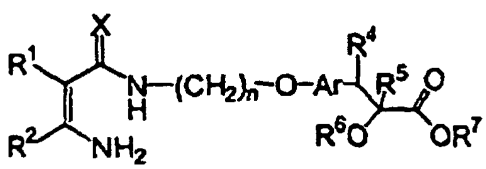

(IIIn)

qui comprend la réaction d'un composé de formule (IVm)

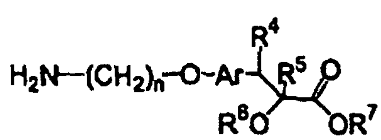

(IVm)

où tous les symboles sont tels que définis dans la revendication 9, avec un composé de formule (IVo)

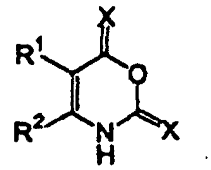

(IVo)

où $R^1$, $R^2$ et X sont tels que définis plus tôt, pour produire un composé de formule (IIIn) défini ci-dessus.

**11.** Intermédiaire de formule (IIIm)

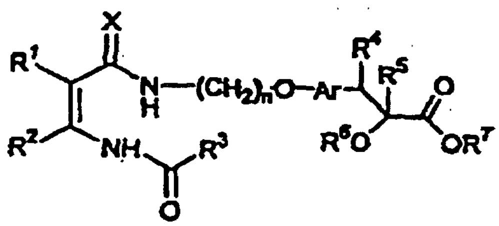

(IIIm)

dans laquelle X, $R^1$, $R^2$, $R^3$, n, Ar, $R^4$, $R^5$, $R^6$ et $R^7$ sont tels que définis dans la revendication 1.

**12.** Procédé pour la préparation de l'intermédiaire de formule (IIIm) défini dans la revendication 11

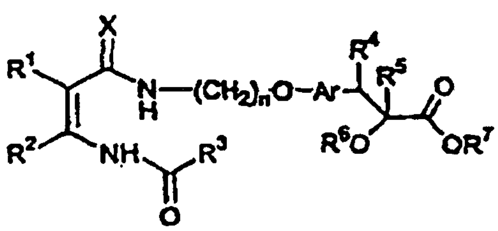

(IIIm)

qui comprend la réaction d'un composé de formule (IIIn)

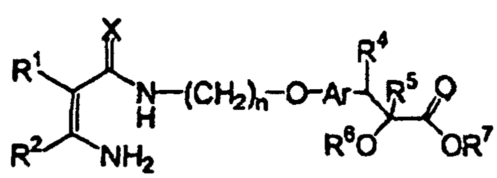

(IIIn)

où tous les symboles sont tels que définis ci-dessus, avec un composé de formule (IIIo)

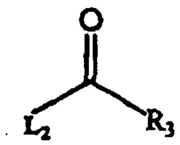

(IIIo)

où $L^2$ est un atome d'halogène et tous les autres symboles sont tels que définis ci-dessus, pour produire un composé de formule (IIIm) où tous les symboles sont tels que définis ci-dessus.

**13.** Composé selon la revendication 1 qui est choisi parmi :

(±)-Ethyl 2-éthoxy-3[4-[[3-méthyl-4-oxo-3,4-dihydro-2-quinazolinyl]méthoxy]phényl]propanoate ;
(±)-2-Ethoxy-3-[4-[[3-méthyl-4-oxo-3,4-dihydro-2-quinazolinyl]méthoxy]phényl]acide propanoïque ;
(±)-Sodium2-éthoxy-3-[4-[[3-méthyl-4-oxo-3,4-dihydro-2-quinazolinyl]méthoxy]phényl]propanoate ;
(+)-2-Ethoxy-3-[4-[[3-méthyl-4-oxo-3,4-dihydro-2-quinazolinyl]méthoxy]phényl]acide propanoïque ;
(-)-2-Ethoxy-3-[4-[[3-méthyl-4-oxo-3,4-dihydro-2-quinazolinyl]méthoxy]phényl]acide propanoïque ;
(-)-Sodium 2-éthoxy-3-[4-[[3-méthyl-4-oxo-3,4-dihydro-2-quinazolinyl]méthoxy]phényl]propanoate ;
(±)-(Morpholine-4-yl)2-éthoxy-3-[4[[3-méthyl-4-oxo-3,4-dihydro-2-quinazolinyl]méthoxy]phényl] propanamide ;
(±)-2-Ethoxy-3-4[[3-méthyl-4-oxo-3,4-dihydro-2-quinazolinyl]méthoxy]phényl]-N-(2-fluorophényl) propanamide ;
(±)-Ethyl 2-méthoxy-3-[4-[[3-méthyl-4-oxo-3,4-dihydro-2-quinazolinyl]méthoxy]phényl]propanoate ;
(±)-2-Méthoxy-3-[4-[[3-méthyl-4-oxo-3,4-dihydro-2-quinazolinyl]méthoxy]phényl]acide propanoïque ;
(±)-Ethyl 2-propoxy-3-[4-[[3-méthyl-4-oxo-3,4-dihydro-2-quinazolinyl]méthoxy]phényl]propanoate ;
(±)-2-Propoxy-3-[4-[[3-méthyl-4-oxo-3,4-dihydro-2-quinazolinyl]méthoxy]phényl]acide propanoïque ;
(±)-Ethyl 2-(n-butoxy)-3-[4-[[3-méthyl-4-oxo-3,4-dihydro-2-quinazolinyl]méthoxy]phényl]propanoate ;
(±)-2-(n-Butoxy)-3-[4-[[3-méthyl]4-oxo-3,4-dihydro-2-quinazolinyl]méthoxy]phényl]acide propanoïque ;
(±)Ethyl2-(n-octyloxy)-3-[4-[[3-méthyl-4-oxo-3,4-dihydro-2-quinazolinyl]méthoxy]phényl]propanoate ;
(±)-2-(n-Octyloxy)-3-[4-[[3-méthyl-4-oxo-3,4-dihydro-2-quinazolinyl]méthoxy]phényl]acide propanoïque ;
(±)-Ethyl 2-benzyloxy-3-[4-[[3-méthyl-4-oxo-3,4-dihydro-2-quinazolinyl)méthoxy]phényl]propanoate ;
(±)-2-Benzyloxy-3-[4-[[3-méthyl-4-oxo-3,4-dihydro-2-quinazolinyl]méthoxy]phényl]acide propanoïque ;
(±)-Ethyl 2-phénoxy 3-[4-[[3-méthyl-4-oxo-3,4-dihydro-2-quinazolinyl]méthoxy]phényl]propanoate ;
(±)-2-Phénoxy-3-[4-[[3-méthyl-4-oxo-3,4-dihydro-2-quinazolinyl]méthoxy]phényl]acide propanoïque ;
(±)-Ethyl 2-(2-méthoxyéthoxy)-3-[4-[[3-méthyl-4-oxo-3,4-dihydro-2-quinazolinyl]méthoxy]phényl]propanoate ;
(±)-2-(2-Méthoxyéthoxy)-3-[4-[[3-méthyl-4-oxo-3,4-dihydro-2-quinazolinyl]méthoxy]phényl]acide propanoïque ;
(±)-Ethyl 2-éthoxy-3-[4-[2-éthyl-4-oxo-3,4-dihydro-3-quinazolinyl]éthoxy]phényl]propanoate ;
(±)-2-Ethoxy-3-[4-[2-[2-éthyl-4-oxo-3,4-dihydro-3-quinazolinyl]éthoxy]phényl]acide propanoïque ;
(+)-2-Ethoxy-3-[4-[2-[2-éthyl-4-oxo-3,4-dihydro-3-quinazolinyl]éthoxy]phényl]acide propanoïque ;
(-)-2-Ethoxy-3-[4-[2-[2-éthyl-4-oxo-3,4-dihydro-3-quinazolinyl]éthoxy]phényl]acide propanoïque ;
(+)-Ethyl2-éthoxy-3-[4-[2-[2-éthyl-4-oxo-3,4-dihydro-3-quinazolinyl]éthoxy]phényl]propanoate ;
(-)-Ethyl2-éthoxy-3-[4-[2-[2-éthyl-4-oxo-3,4-dihydro-3-quinazolinyl]éthoxy]phényl]propanoate ;
(±)-Ethyl2-éthoxy-3-[4-[2-[2-méthyl-4-oxo-3,4-dihydro-3-quinazolinyl]éthoxy]phényl]propanoate ;
(±)-2-Ethoxy-3-[4-[2-[2-méthyl-4-oxo-3,4-dihydro-3-quinazolinyl]éthoxy]phényl]acide propanoïque ;
(+)-2-Ethoxy-3-[4-[2-[2-méthyl-4-oxo-3,4-dihydro-3-quinazolinyl]éthoxy]phényl]acide propanoïque ;
(-)-2-Ethoxy-3-[4-[2-[2-méthyl-4-oxo-3,4-dihydro-3-quinazolinyl]éthoxy]phényl]acide propanoïque ; (+)-Ethyl2-éthoxy-3-[4-[2-[2-méthyl-4-oxo-3,4-dihydro-3-quinazolinyl]éthoxy]phényl]propanoate ;
(-)-Ethyl-2-éthoxy-3-[4-[2-[2-méthyl-4-oxo-3,4-dihydro-3-quinazolinyl]éthoxy]phényl]propanoate ;
(±)-Ethyl 2-éthoxy-3-[4-[2-[4-oxo-3,4-dihydro-3-quinazolinyl]éthoxy]phényl]propanoate ;
(±)-2-Ethoxy-3-[4-[2-[4-oxo-3,4-dihydro-3-quinazolinyl]éthoxy]phényl]acide propanoïque ;
(t)-Ethyl 2-phénoxy-3-[4-[2-[2-éthyl-4-oxo-3,4-dihydro-3-quinazolinyl]éthoxy]phényl]propanoate ;
(±)-2-Phénoxy-3-[4-[2-[2-éthyl-4-oxo-3,4-dihydro-3-quinazolinyl]éthoxy]phényl]acide propanoïque ;
(±)-Ethyl 2-phénoxy-3-[4-[2-[2-méthyl-4-oxo-3,4-dihydro-3-quinazolinyl]éthoxy]phényl]propanoate ;
(±)-2-Phénoxy-3-[4-[2-[2-méthyl-4-oxo-3,4-dihydro-3-quinazolinyl]éthoxy]phényl]acide propanoïque ;
(±)-Ethyl 2-éthoxy-3-[4-[2-[2-éthyl-4-méthyl-6- oxo-1-pyrimidyl]éthoxy]phényl]propanoate ;
(±)-2-Ethoxy-3-[4-[2-[2-éthyl-4-méthyl-6-oxo-1-pyrimidyl]éthoxy]phényl]acide propanoïque ;

(±)-Ethyl2-éthoxy-3-[4-[[3-phényl-4-oxo-3,4-dihydro-2-quinazolinyl]méthoxy]phényl]propanoate ;
(±)-2-Ethoxy-3-[4-[[3-phényl-4-oxo-3,4-dihydro-2-quinazolinyl]méthoxy]phényl]acide propanoïque ;
(±)-Ethyl 2-éthoxy-3-[4-[[3-méthyl-4-oxo-3,4-dihydro-6,7-diméthoxy-2- quinazolinyl]méthoxy]phényl]propanoate ;
(±)-2-Ethoxy-3-[4-[[3-méthyl-4-oxo-3,4-dihydro-6,7-diméthoxy-2-quinazolinyl]méthoxy]phényl]acide propanoïque ;
(±)-Ethyl 2-éthoxy-3-[4-[[3-(4-méthylphényl)-4-oxo-3,4-dihydro-2-quinazolinyl]méthoxy]phényl] propanoate ;
(±)-2-Ethoxy-3-[4-[[3-(4-méthylphényl)-4-oxo-3,4-dihydro-2-quinazolinyl]méthoxy]phényl]acide propanoïque ;
(±)-Ethyl 2-éthoxy-3-[4-[[3-(4-méthoxyphényl)-4-oxo-3,4-dihydro-2-quinazolinyl]méthoxy]phényl]propanoate ;
(±)-2-Ethoxy-3-[4-[[3-(4-méthoxyphényl)-4-oxo-3,4-dihydro-2-quinazolinyl]méthoxy]phényl]acide propanoïque ;
(±)-Ethyl2-éthoxy-3-[4-[[3-benzyl-4-oxo-3,4-dihydro-2-quinazolinyl]méthoxy]phényl]propanoate ;
(±)-2-Ethoxy-3-[4-[(3-benzyl-4-oxo-3,4-dihydro-2-quinazolinyl]méthoxy]phényl]acide propanoïque ;
(±)-Ethyl 2-éthoxy-3-[4-[[3-(3-chlorophényl)4-oxo-3,4-dihydro-2-quinazolinyl]méthoxy]phényl]propanoate ;
(±)-2-Ethoxy-3-[4-[[3-(3-chlorophényl)-4-oxo-3,4-dihydro-2-quinazolinyl]méthoxy]phényl]acide propanoïque ;
(t)-Ethyl 2-éthoxy-3-[4-[[3-(3-chloro-4-fluorophényl)-4-oxo-3,4-dihydro-2-quinazolinyl]méthoxy]phényl]propanoate ; ou
(±)-2-Ethoxy-3-[4-[[3-(3-chloro-4-fluorophényl)-4-oxo-3,4-dihydro-2-quinazolinyl]méthoxy]phényl]acide propanoïque ;

**14.** Composition qui comprend un composé de formule (I) tel que défini dans la revendication 1 ou 13 et un vecteur, un diluant, un excipient ou un produit de solvatation pharmaceutiquement acceptables.

**15.** Composition qui comprend un composé de formule (IIIm) tel que défini dans la revendication 11 et un vecteur, un diluant, un excipient ou un produit de solvatation pharmaceutiquement acceptables.

**16.** Composition pharmaceutique telle que revendiquée dans la revendication 14 ou 15, sous la forme d'un comprimé, d'une gélule, d'une poudre, d'un sirop, d'une solution ou d'une suspension.

**17.** Utilisation d'un composé de formule (I) tel que défini dans l'une quelconque des revendications 1 ou 13 ou un composé de formule (IIIm) tel que défini dans la revendication 11 pour la préparation d'un médicament pour la prophylaxie ou le traitement de l'hyperlipémie, l'hypercholestérolémie, l'hyperglycémie, l'ostéoporose, l'obésité, l'intolérance au glucose, une résistance à la leptine, une résistance à l'insuline, ou des affections dans lesquelles la résistance à l'insuline est le mécanisme pathophysiologique sous-jacent.

**18.** Utilisation d'un composé de formule (I) tel que défini dans l'une quelconque des revendications 1 ou 13 ou un composé de formule (IIIm) tel que défini dans la revendication 11 pour la préparation d'un médicament pour réduire le glucose plasmatique, les triglycérides, le cholestérol total, les LDL, VLDL ou les acides gras libres dans le plasma.

**19.** Utilisation d'un composé de formule (I) tel que défini dans l'une quelconque des revendications 1 ou 13 ou un composé de formule (IIIm) tel que défini dans la revendication 11 pour la préparation d'un médicament en combinaison/de manière concomitante avec des inhibiteurs de la HMG CoA réductase, des fibrates, l'acide nicotinique, la cholestyramine, le colestipol ou le probucol pour la prophylaxie ou le traitement de l'hyperlipémie, de l'hypercholestérolémie, de l'hyperglycémie, de l'ostéoporose, de l'obésité, de l'intolérance au glucose, de la résistance à la leptine, de la résistance à l'insuline, ou d'affections dans lesquelles la résistance à l'insuline est le mécanisme pathophysiologique sous-jacent.

**20.** Utilisation d'un composé de formule (I) tel que défini dans l'une quelconque des revendications 1 ou 13 ou un composé de formule (IIIm) tel que défini dans la revendication 11 pour la préparation d'un médicament en combinaison/de manière concomitante avec des inhibiteurs de la HMG CoA réductase, des fibrates, l'acide nicotinique, la cholestyramine, le colestipol ou le probucol pour la réduction du glucose plasmatique, des triglycérides, du cholestérol total, des LDL, des VLDL ou des acides gras libres dans le plasma.

**21.** Utilisation selon l'une quelconque des revendications 17 ou 19 dans laquelle la maladie est un diabète de type II, un trouble de la tolérance au glucose, une dyslipidémie, des troubles liés au Syndrome X, tels que l'hypertension, l'obésité, l'athérosclérose, l'hyperlipidémie, l'insuffisance coronarienne et d'autres troubles cardiovasculaires, certaines affections rénales, notamment la glomérulonéphrite, la glomérulosclérose, le syndrome néphrotique, la néphrosclérose hypertensive, la rétinopathie, la néphropathie, les troubles liés à l'activation cellulaire endothéliale, le psoriasis, le syndrome ovarien polykystique (SOPK), utile en tant qu'inhibiteurs de l'aldose réductase, pour l'amélioration des fonctions cognitives dans la démence et le traitement de complications diabétiques, l'ostéoporose, les affections intestinales inflammatoires, la dystrophie myotonique, la pancréatite, l'artériosclérose, le xanthome ou le cancer.

**22.** Utilisation d'un agoniste du PPAR$\alpha$ et/ou un agoniste du PPAR$\gamma$ de Formule (I) telle que défini dans la revendication 1 ou 13 ou d'une formule (IIIm) telle que définie dans la revendication 11 pour la préparation d'un médicament pour le traitement ou la prophylaxie de troubles liés au Syndrome X.